(19) Europäisches Patentamt
European Patent Office
Office européen des brevets

(11) EP 4 375 284 A1

(12) **EUROPEAN PATENT APPLICATION**
published in accordance with Art. 153(4) EPC

(43) Date of publication:
**29.05.2024 Bulletin 2024/22**

(21) Application number: **22852252.0**

(22) Date of filing: **03.08.2022**

(51) International Patent Classification (IPC):
***C07D 471/04*** (2006.01) ***C07D 453/02*** (2006.01)
***C07D 519/00*** (2006.01) ***A61P 35/00*** (2006.01)
***A61K 31/519*** (2006.01)

(52) Cooperative Patent Classification (CPC):
**A61K 31/519; A61P 35/00; C07D 453/02; C07D 471/04; C07D 519/00**

(86) International application number:
**PCT/CN2022/110019**

(87) International publication number:
**WO 2023/011540 (09.02.2023 Gazette 2023/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
**03.08.2021 CN 202110884595**
**10.09.2021 CN 202111063419**
**04.11.2021 CN 202111300099**
**25.11.2021 CN 202111415010**
**20.01.2022 CN 202210068494**

(71) Applicant: **Evopoint Biosciences Co., Ltd.**
**Suzhou, Jiangsu 215000 (CN)**

(72) Inventors:
- **WU, Dongdong**
  **Suzhou, Jiangsu 215000 (CN)**
- **LI, Xin**
  **Suzhou, Jiangsu 215000 (CN)**
- **XU, Jiajing**
  **Suzhou, Jiangsu 215000 (CN)**
- **XU, Linfeng**
  **Suzhou, Jiangsu 215000 (CN)**
- **WU, Yuchuan**
  **Suzhou, Jiangsu 215000 (CN)**
- **HU, Yonghan**
  **Suzhou, Jiangsu 215000 (CN)**

(74) Representative: **Henderson, Helen Lee**
**Withers & Rogers LLP**
**2 London Bridge**
**London SE1 9RA (GB)**

(54) **FUSED RING COMPOUND, PHARMACEUTICAL COMPOSITION, AND APPLICATION THEREOF**

(57) A fused ring compound, a pharmaceutical composition, and an application thereof. Provided is a fused ring compound shown in formula I or a pharmaceutically acceptable salt thereof. The compound has a novel structure and good SOS1 inhibitory activity.

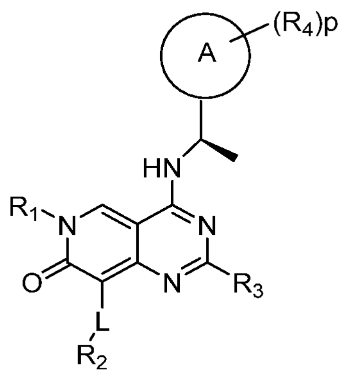

(I)

EP 4 375 284 A1

## Description

The present application claims priority to Chinese Patent Application No. 202110884595.1 filed on August 3, 2021, Chinese Patent Application No. 202111063419.8 filed on September 10, 2021, Chinese Patent Application No. 202111300099.3 filed on November 4, 2021, Chinese Patent Application No. 202111415010.8 filed on November 25, 2021, and Chinese Patent Application No. 2022100684941 filed on January 20, 2022, which are incorporated herein by reference in their entireties.

### TECHNICAL FIELD

The present invention relates to a fused ring compound, a pharmaceutical composition, and use thereof.

### BACKGROUND

SOS (son of sevenless) protein was first discovered in 1992 by Bonfini et al. in their study of Drosophila eyes. SOS proteins are products of SOS genes encoding guanyl-releasing proteins and play an important role in the growth and development of Drosophila, nematodes, mice, and humans. SOS1(son of sevenless homolog 1) is a guanine nucleotide exchange factor (GEF) of Ras and Rac, which can play an important role in Ras and Rac signaling pathways by regulating the guanosine diphosphate (GDP)/guanosine triphosphate (GTP) exchange of G proteins. SOS 1 was identified to be involved in the Ras signaling pathway and in the tumorigenic process of many tumors.

There are two SOS homologues in humans, namely hSOS1 and hSOS2. The hSOS1 protein has a size of 150 kDa and consists of 1300 amino acid residues. The C-terminus of hSOS1 contains a proline-rich (PxxP) domain that can interact with the SH3 (Src homology 3) domain of proteins such as growth factor receptor-bound protein 2 (Grb2) in the Ras pathway, E3B1 in the Rac pathway, and the like. Ras-GTP is a more effective SOS allosteric activator, and single-molecule sequencing results show that Ras-GTP can bind to an allosteric binding site of SOS1 to cause a change in the structure of SOS1, which prevents the PxxP domain of SOS1 from binding to Grb2 to generate activity, thus exerting an inhibitory effect.

SOS1 can cause Ras-GTP to be converted to activate Ras. The activation of Ras plays an important role in cell growth and differentiation, and can further cause the activation of the Raf-mitogen-activated protein kinase (MAPK)-extracellular signal-regulated protein kinase (ERK) signaling pathway, thereby having a decisive role in cell proliferation, transformation and migration. Excessive activation of this signaling pathway can cause the development of cancer.

### SUMMARY

The present invention aims to solve the technical problem that existing SOS1 inhibitors have fewer structural varieties, and therefore, the present invention provides a fused ring compound, a pharmaceutical composition, and use thereof. The compound has a novel structure and good SOS1 inhibitory activity.

The present invention solves the above technical problem by means of the following technical solutions.

The present invention provides a fused ring compound represented by formula I or a pharmaceutically acceptable salt thereof:

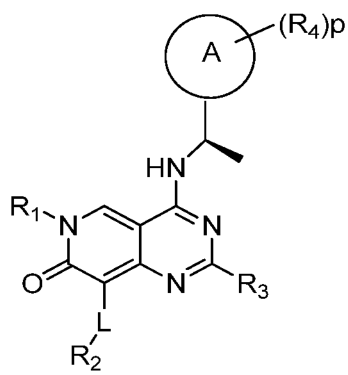

I ,

wherein,

$R_1$ is $C_{1-6}$ alkyl, $C_{1-6}$ alkyl substituted with one or more $R_{1-1a}$, $C_{3-10}$ cycloalkyl, $C_{3-10}$ cycloalkyl substituted with one or more $R_{1-1b}$, "3- to 10-membered heterocyclyl having 1, 2 or 3 heteroatoms selected from 1, 2 or 3 of N, O and S", "3- to 10-membered heterocyclyl having 1, 2 or 3 heteroatoms selected from 1, 2 or 3 of N, O and S" substituted with one or more $R_{1-1c}$, $C_{6-10}$ aryl, $C_{6-10}$ aryl substituted with one or more $R_{1-1d}$, "5- to 10-membered heteroaryl having 1, 2 or 3 heteroatoms selected from 1, 2 or 3 of N, O and S", or "5- to 10-membered heteroaryl having 1, 2 or 3 heteroatoms selected from 1, 2 or 3 of N, O and S" substituted with one or more $R_{1-1e}$;

$R_{1-1a}$, $R_{1-1b}$, $R_{1-1c}$, $R_{1-1d}$, and $R_{1-1e}$ are each independently -$OR_{1-2a}$, -$NR_{1-2b}R_{1-2c}$, halogen, - CN, -C(O)$R_{1-2d}$, -C(O)O$R_{1-2e}$, -C(O)N$R_{1-2f}R_{1-2g}$, $C_{1-6}$ alkyl, $C_{1-6}$ alkyl substituted with one or more $R_{1-3a}$, $C_{3-10}$ cycloalkyl, or "$C_{3-10}$ cycloalkyl substituted with one or more $R_{1-3b}$";

$R_{1-2a}$, $R_{1-2b}$, $R_{1-2c}$, $R_{1-2d}$, $R_{1-2e}$, $R_{1-2f}$, and $R_{1-2g}$ are each independently H, $C_{1-6}$ alkyl, $C_{1-6}$ alkyl substituted with one or more $R_{1-3c}$, $C_{3-10}$ cycloalkyl, $C_{3-10}$ cycloalkyl substituted with one or more $R_{1-3d}$, "3- to 10-membered heterocyclyl having 1, 2 or 3 heteroatoms selected from 1, 2 or 3 of N, O and S", "3- to 10-membered heterocyclyl having 1, 2 or 3 heteroatoms selected from 1, 2 or 3 of N, O and S" substituted with one or more $R_{1-3e}$, $C_{6-10}$ aryl, $C_{6-10}$ aryl substituted with one or more $R_{1-3f}$, "5- to 10-membered heteroaryl having 1, 2 or 3 heteroatoms selected from 1, 2 or 3 of N, O and S", or "5- to 10-membered heteroaryl having 1, 2 or 3 heteroatoms selected from 1, 2 or 3 of N, O and S" substituted with one or more $R_{1-3g}$;

$R_{1-3a}$, $R_{1-3b}$, $R_{1-3c}$, $R_{1-3d}$, $R_{1-3e}$, $R_{1-3f}$, and $R_{1-3g}$ are each independently -$OR_{1-4a}$, -$NR_{1-4b}R_{1-4c}$, halogen, $C_{1-6}$ alkyl substituted with one or more halogens, $C_{3-10}$ cycloalkyl, "3- to 10-membered heterocyclyl having 1, 2 or 3 heteroatoms selected from 1, 2 or 3 of N, O and S", $C_{6-10}$ aryl, or "5- to 10-membered heteroaryl having 1, 2 or 3 heteroatoms selected from 1, 2 or 3 of N, O and S";

$R_{1-4a}$, $R_{1-4b}$, and $R_{1-4c}$ are each independently hydrogen, $C_{1-6}$ alkyl, $C_{1-6}$ alkyl substituted with one or more halogens, $C_{3-10}$ cycloalkyl, "3- to 10-membered heterocyclyl having 1, 2 or 3 heteroatoms selected from 1, 2 or 3 of N, O and S", $C_{6-10}$ aryl, or "5- to 10-membered heteroaryl having 1, 2 or 3 heteroatoms selected from 1, 2 or 3 of N, O and S";

L is -O-, -S-, -$SO_2$-, or -$NR_{L-1}$; $R_{L-1}$ is hydrogen or $C_{1-6}$ alkyl;

$R_2$ is selected from hydrogen, $C_{1-6}$ alkyl, $C_{1-6}$ alkyl substituted with one or more $R_{2-1}$, $C_{3-10}$ cycloalkyl, or "3- to 10-membered heterocyclyl having 1, 2 or 3 heteroatoms selected from 1, 2 or 3 of N, O and S";

each $R_{2-1}$ is independently deuterium, halogen, $C_{3-10}$ cycloalkyl, or hydroxy;

$R_3$ is hydrogen, $C_{1-6}$ alkyl, or $C_{1-6}$ alkyl substituted with one or more halogens;

each $R_4$ is independently halogen, -$NH_2$, $C_{1-6}$ alkyl, $C_{1-6}$ alkyl substituted with one or more $R_{4-1}$, "3- to 10-membered heterocyclyl having 1, 2 or 3 heteroatoms selected from 1, 2 or 3 of N, O and S", $C_{3-6}$ cycloalkyl, $C_{6-10}$ aryl, or $C_{6-10}$ aryl substituted with one or more -$(CH_2)_mNR_{4-2}R_{4-3}$, wherein m is 0, 1, 2 or 3;

each $R_{4-1}$ is independently halogen or hydroxy;

$R_{4-2}$ and $R_{4-3}$ are each independently $C_{1-6}$ alkyl;

ring A is $C_{6-10}$ aryl, "5- to 10-membered heterocyclyl having 1, 2 or 3 heteroatoms selected from 1, 2 or 3 of N, O and S", or "5- to 10-membered heteroaryl having 1, 2 or 3 heteroatoms selected from 1, 2 or 3 of N, O and S"; and

p is 1, 2 or 3.

In certain preferred embodiments of the present invention, certain groups in the fused ring compound represented by formula I or the pharmaceutically acceptable salt thereof are defined below, and groups not mentioned are as described in any of the embodiments of the present invention (referred to as " in some embodiments").

In some embodiments, each $R_4$ may also be independently -CN.

In some embodiments, $R_1$ is $C_{3-10}$ cycloalkyl, $C_{3-10}$ cycloalkyl substituted with one or more $R_{1-1b}$, "3- to 10-membered heterocyclyl having 1, 2 or 3 heteroatoms selected from 1, 2 or 3 of N, O and S", or "3- to 10-membered heterocyclyl having 1, 2 or 3 heteroatoms selected from 1, 2 or 3 of N, O and S" substituted with one or more $R_{1-1c}$, preferably $C_{3-10}$ cycloalkyl substituted with one or more $R_{1-1b}$, "3- to 10-membered heterocyclyl having 1, 2 or 3 heteroatoms selected from 1, 2 or 3 of N, O and S", or "3- to 10-membered heterocyclyl having 1, 2 or 3 heteroatoms selected from 1, 2 or 3 of N, O and S" substituted with one or more $R_{1-1c}$.

In some embodiments, $R_{1-1a}$, $R_{1-1b}$, $R_{1-1c}$, $R_{1-1d}$, and $R_{1-1e}$ are each independently halogen, - CN, -C(O)$R_{1-2d}$, $C_{1-6}$ alkyl, or $C_{1-6}$ alkyl substituted with one or more $R_{1-3a}$, preferably $C_{1-6}$ alkyl or $C_{1-6}$ alkyl substituted with one or more $R_{1-3a}$.

In some embodiments, $R_{1-2a}$, $R_{1-2b}$, $R_{1-2c}$, $R_{1-2d}$, $R_{1-2e}$, $R_{1-2f}$, and $R_{1-2g}$ are each independently $C_{3-10}$ cycloalkyl.

In some embodiments, $R_{1-3a}$, $R_{1-3b}$, $R_{1-3c}$, $R_{1-3d}$, $R_{1-3e}$, $R_{1-3f}$, and $R_{1-3g}$ are each independently -$OR_{1-4a}$ or halogen, preferably halogen.

In some embodiments, $R_{1-4a}$, $R_{1-4b}$, and $R_{1-4c}$ are each independently hydrogen or $C_{1-6}$ alkyl.

In some embodiments, $R_3$ is $C_{1-6}$ alkyl.

In some embodiments, each $R_4$ is independently halogen, -$NH_2$, $C_{1-6}$ alkyl, $C_{1-6}$ alkyl substituted with one or more $R_{4-1}$, or $C_{6-10}$ aryl substituted with one or more -$(CH_2)_mNR_{4-2}R_{4-3}$.

In some embodiments, L is -O- or -S-.

In some embodiments, $R_2$ is $C_{1-6}$ alkyl, $C_{1-6}$ alkyl substituted with one or more $R_{2-1}$, $C_{3-10}$ cycloalkyl, or "3- to 10-membered heterocyclyl having 1, 2 or 3 heteroatoms selected from 1, 2 or 3 of N, O and S", preferably $C_{1-6}$ alkyl or $C_{1-6}$ alkyl substituted with one or more $R_{2-1}$. In some embodiments, each $R_4$ is independently halogen, $-NH_2$, $C_{1-6}$ alkyl, or $C_{1-6}$ alkyl substituted with one or more $R_{4-1}$.

In some embodiments, each $R_4$ is independently -CN.

In some embodiments, $R_1$ is $C_{3-10}$ cycloalkyl substituted with one or more $R_{1-1b}$, "3- to 10-membered heterocyclyl having 1, 2 or 3 heteroatoms selected from 1, 2 or 3 of N, O and S", or "3- to 10-membered heterocyclyl having 1, 2 or 3 heteroatoms selected from 1, 2 or 3 of N, O and S" substituted with one or more $R_{1-1c}$;

each $R_{1-1b}$ is independently $C_{1-6}$ alkyl substituted with one or more $R_{1-3a}$;
each $R_{1-1c}$ is independently $C_{1-6}$ alkyl;
each $R_{1-3a}$ is independently halogen;
L is -O-;
$R_2$ is $C_{1-6}$ alkyl or $C_{1-6}$ alkyl substituted with one or more $R_{2-1}$;
$R_{2-1}$ is deuterium;
$R_3$ is $C_{1-6}$ alkyl;
ring A is $C_{6-10}$ aryl, "5- to 10-membered heterocyclyl having 1, 2 or 3 heteroatoms selected from 1, 2 or 3 of N, O and S", or "5- to 10-membered heteroaryl having 1, 2 or 3 heteroatoms selected from 1, 2 or 3 of N, O and S";
each $R_4$ is independently halogen, $-NH_2$, $C_{1-6}$ alkyl, $C_{1-6}$ alkyl substituted with one or more $R_{4-1}$, or $C_{6-10}$ aryl substituted with one or more $-(CH_2)_mNR_{4-2}R_{4-3}$; and
each $R_{4-1}$ is independently halogen or hydroxy.

In some embodiments, $R_1$ is $C_{3-10}$ cycloalkyl substituted with one or more $R_{1-1b}$, "3- to 10-membered heterocyclyl having 1, 2 or 3 heteroatoms selected from 1, 2 or 3 of N, O and S", or "3- to 10-membered heterocyclyl having 1, 2 or 3 heteroatoms selected from 1, 2 or 3 of N, O and S" substituted with one or more $R_{1-1c}$;

each $R_{1-1b}$ is independently $C_{1-6}$ alkyl substituted with one or more $R_{1-3a}$;
each $R_{1-1c}$ is independently $C_{1-6}$ alkyl;
each $R_{1-3a}$ is independently halogen;
L is -O-;
$R_2$ is $C_{1-6}$ alkyl or $C_{1-6}$ alkyl substituted with one or more $R_{2-1}$;
$R_{2-1}$ is deuterium;
$R_3$ is $C_{1-6}$ alkyl;
ring A is $C_{6-10}$ aryl, "5- to 10-membered heterocyclyl having 1, 2 or 3 heteroatoms selected from 1, 2 or 3 of N, O and S", or "5- to 10-membered heteroaryl having 1, 2 or 3 heteroatoms selected from 1, 2 or 3 of N, O and S";
each $R_4$ is independently halogen, $-NH_2$, -CN, $C_{1-6}$ alkyl, $C_{1-6}$ alkyl substituted with one or more $R_{4-1}$, or $C_{6-10}$ aryl substituted with one or more $-(CH_2)_mNR_{4-2}R_{4-3}$; and
each $R_{4-1}$ is independently halogen or hydroxy.

In some embodiments, in $R_1$, $R_{1-1a}$, $R_{1-1b}$, $R_{1-1c}$, $R_{1-1d}$, $R_{1-1e}$, $R_{1-2a}$, $R_{1-2b}$, $R_{1-2c}$, $R_{1-2d}$, $R_{1-2e}$, $R_{1-2f}$, $R_{1-2g}$, $R_{1-3a}$, $R_{1-3b}$, $R_{1-3c}$, $R_{1-3d}$, $R_{1-3e}$, $R_{1-3f}$, $R_{1-3g}$, $R_{1-4a}$, $R_{1-4b}$, $R_{1-4c}$, $R_{L-1}$, $R_2$, $R_3$, $R_4$, $R_{4-2}$, and $R_{4-3}$, the "$C_{1-6}$ alkyl" in each of the "$C_{1-6}$ alkyl substituted with one or more $R_{1-1a}$", the "$C_{1-6}$ alkyl substituted with one or more $R_{1-3a}$", the "$C_{1-6}$ alkyl substituted with one or more $R_{1-3c}$", the "$C_{1-6}$ alkyl substituted with one or more halogens", the "$C_{1-6}$ alkyl", the "$C_{1-6}$ alkyl substituted with one or more $R_{2-1}$", and the "$C_{1-6}$ alkyl substituted with one or more $R_{4-1}$" is independently methyl, ethyl, n-propyl, isopropyl, n-butyl, isobutyl, sec-butyl, or tert-butyl, preferably methyl, ethyl, isobutyl, or isopropyl.

In some embodiments, in $R_1$, $R_{1-1a}$, $R_{1-1b}$, $R_{1-1c}$, $R_{1-1d}$, $R_{1-1e}$, $R_{1-2a}$, $R_{1-2b}$, $R_{1-2c}$, $R_{1-2d}$, $R_{1-2e}$, $R_{1-2f}$, $R_{1-2g}$, $R_{1-3a}$, $R_{1-3b}$, $R_{1-3c}$, $R_{1-3d}$, $R_{1-3e}$, $R_{1-3f}$, $R_{1-3g}$, $R_{1-4a}$, $R_{1-4b}$, $R_{1-4c}$, $R_2$, and $R_{2-1}$, the "$C_{3-10}$ cycloalkyl" in each of the "$C_{3-10}$ cycloalkyl", the "$C_{3-10}$ cycloalkyl substituted with one or more $R_{1-1b}$", the "$C_{3-10}$ cycloalkyl substituted with one or more $R_{1-3b}$", and the "$C_{3-10}$ cycloalkyl substituted with one or more $R_{1-3d}$" is independently $C_{3-6}$ cycloalkyl, such as cyclopropyl, cyclobutyl, or cyclopentyl (e.g.,

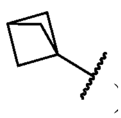

), preferably

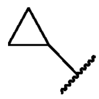.

In some embodiments, in $R_1$, $R_{1-2a}$, $R_{1-2b}$, $R_{1-2c}$, $R_{1-2d}$, $R_{1-2e}$, $R_{1-2f}$, $R_{1-2g}$, $R_{1-3a}$, $R_{1-3b}$, $R_{1-3c}$, $R_{1-3d}$, $R_{1-3e}$, $R_{1-3f}$, $R_{1-3g}$, $R_{1-4a}$, $R_{1-4b}$, $R_{1-4c}$, $R_2$, and $R_4$, the "3- to 10-membered heterocyclyl" in each of the "3- to 10-membered heterocyclyl having 1, 2 or 3 heteroatoms selected from 1, 2 or 3 of N, O and S", the "'3- to 10-membered heterocyclyl having 1, 2 or 3 heteroatoms selected from 1, 2 or 3 of N, O and S' substituted with one or more $R_{1-1c}$", and the "'3- to 10-membered heterocyclyl having 1, 2 or 3 heteroatoms selected from 1, 2 or 3 of N, O and S' substituted with one or more $R_{1-3e}$" is independently 5- to 6-membered heterocyclyl, preferably saturated 5- to 6-membered heterocyclyl, and further preferably

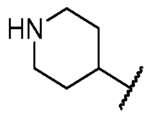, 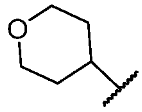, or 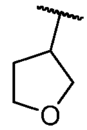.

In some embodiments, in $R_1$, $R_{1-2a}$, $R_{1-2b}$, $R_{1-2c}$, $R_{1-2d}$, $R_{1-2e}$, $R_{1-2f}$, $R_{1-2g}$, $R_{1-3a}$, $R_{1-3b}$, $R_{1-3c}$, $R_{1-3d}$, $R_{1-3e}$, $R_{1-3f}$, $R_{1-3g}$, $R_{1-4a}$, $R_{1-4b}$, $R_{1-4c}$, $R_4$, and ring A, the "$C_{6-10}$ aryl" in each of the "$C_{6-10}$ aryl", the "$C_{6-10}$ aryl substituted with one or more $R_{1-1d}$", the "$C_{6-10}$ aryl substituted with one or more $R_{1-31}$", and the "$C_{6-10}$ aryl substituted with one or more $-(CH_2)_mNR_{4-2}R_{4-3}$" is independently phenyl.

In some embodiments, in $R_1$, $R_{1-2a}$, $R_{1-2b}$, $R_{1-2c}$, $R_{1-2d}$, $R_{1-2e}$, $R_{1-2f}$, $R_{1-2g}$, $R_{1-3a}$, $R_{1-3b}$, $R_{1-3c}$, $R_{1-3d}$, $R_{1-3e}$, $R_{1-3f}$, $R_{1-3g}$, $R_{1-4a}$, $R_{1-4b}$, $R_{1-4c}$, and ring A, the "5- to 10-membered heteroaryl" in each of the "5- to 10-membered heteroaryl having 1, 2 or 3 heteroatoms selected from 1, 2 or 3 of N, O and S", the "'5- to 10-membered heteroaryl having 1, 2 or 3 heteroatoms selected from 1, 2 or 3 of N, O and S' substituted with one or more $R_{1-1e}$", and the "'5- to 10-membered heteroaryl having 1, 2 or 3 heteroatoms selected from 1, 2 or 3 of N, O and S' substituted with one or more $R_{1-3g}$" is independently "9- to 10-membered heteroaryl", such as benzofuranyl, preferably

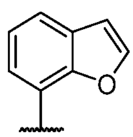.

In some embodiments, in ring A, the "5- to 10-membered heterocyclyl" in the "5- to 10-membered heterocyclyl having 1, 2 or 3 heteroatoms selected from 1, 2 or 3 of N, O and S" is "9- to 10-membered heterocyclyl", such as 2,3-dihydrobenzofuranyl, preferably

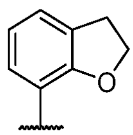.

In some embodiments, in $R_{1-1a}$, $R_{1-1b}$, $R_{1-1c}$, $R_{1-1d}$, $R_{1-1e}$, $R_{1-3a}$, $R_{1-3b}$, $R_{1-3c}$, $R_{1-3d}$, $R_{1-3e}$, $R_{1-3f}$, $R_{1-3g}$, $R_{2-1}$, $R_3$, $R_4$, and $R_{4-1}$, the "halogen" in each of the "halogen" and the "$C_{1-6}$ alkyl substituted with one or more halogens" is independently fluorine, chlorine, bromine, or iodine, preferably fluorine.

In some embodiments, $R_1$ is

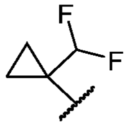, 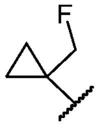, 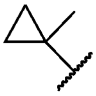, 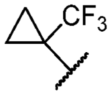, 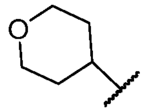,

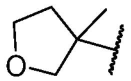, 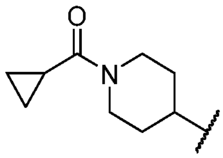, 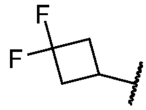, 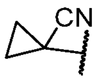, 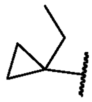, 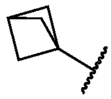, 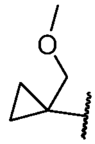, or 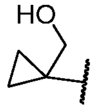,

preferably

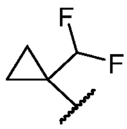, 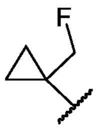, 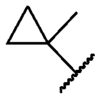, 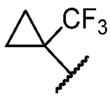, 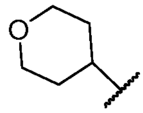, or 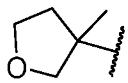.

In some embodiments, $R_2$ is hydrogen, methyl, ethyl, $-CD_3$, isopropyl, $-CH_2CF_3$,

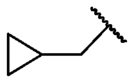

, cyclopropyl,

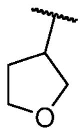, 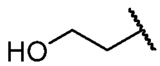, or 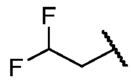,

preferably methyl, ethyl, or $-CD_3$.

In some embodiments, -L-$R_2$ is $-OCH_3$, $-OCH_2CH_3$, $-OCD_3$, $-SO_2CH_3$, $-OCH_2CF_3$,

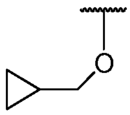,

$-SCH_3$, $-N(CH_3)(CH_3)$, $-NH_2$,

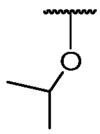

, -OH,

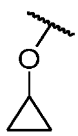, 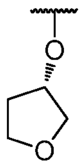, 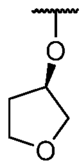,

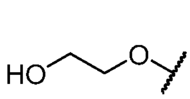, 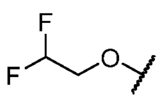,

or $-SO_2CH_2CH_3$, preferably $-OCH_3$, $-OCH_2CH_3$, $-OCD_3$, or - $SCH_3$.

In some embodiments, $R_3$ is methyl.

In some embodiments, $R_4$ is

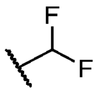, 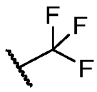, 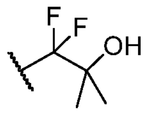, 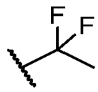, 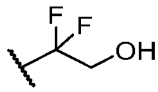,

-F, - $CH_3$, $-NH_2$,

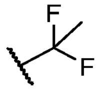, or 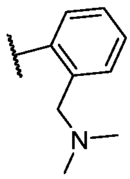,

preferably

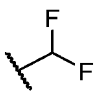,

-F, $-CH_3$,

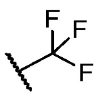, 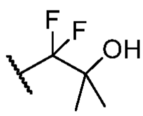,

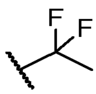,

$-NH_2$,

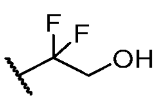, or 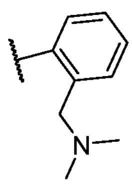.

In some embodiments, ring A is phenyl, benzofuranyl, 2,3-dihydrobenzofuranyl, or thienyl, preferably phenyl,

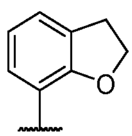, 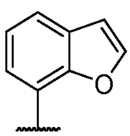, or 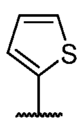.

In some embodiments,

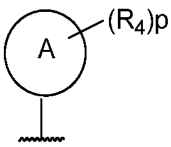 is 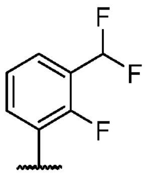, 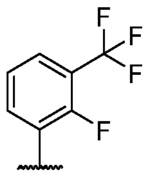, 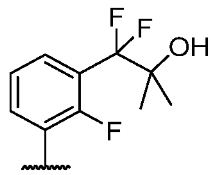,

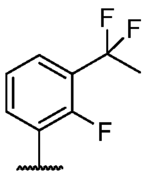, 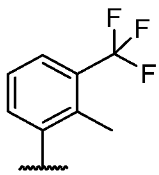, 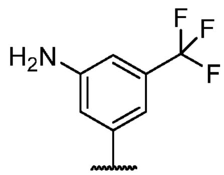, 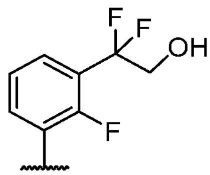, 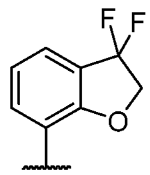, 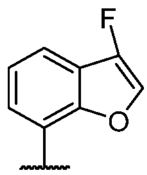,

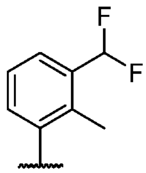, 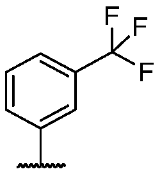, 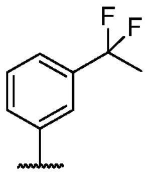, or 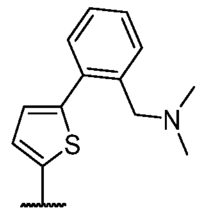,

preferably

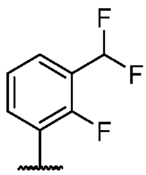,

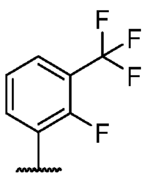, 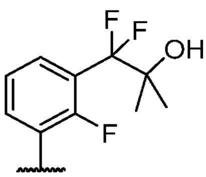, 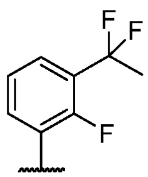, 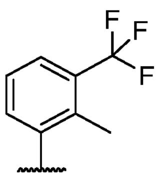, 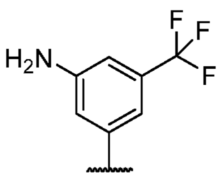, 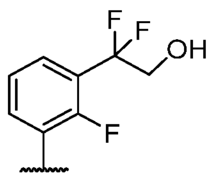,

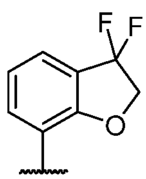, 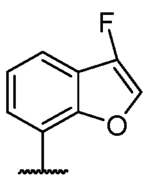, 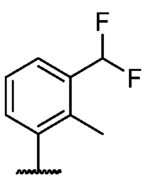, or 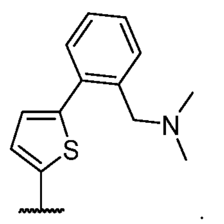.

In some embodiments,

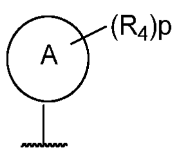

is

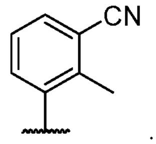

.

In some embodiments, the fused ring compound represented by formula I is any one of the following compounds:

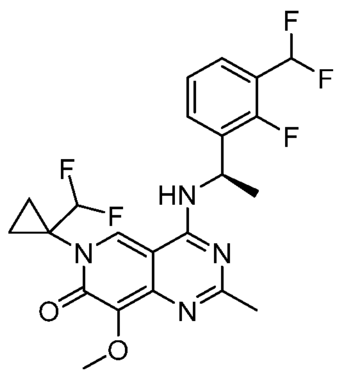

**SZ-022244** ,

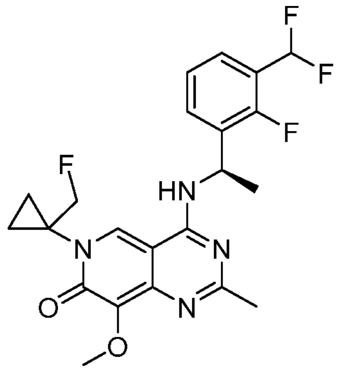

**SZ-022301** ,

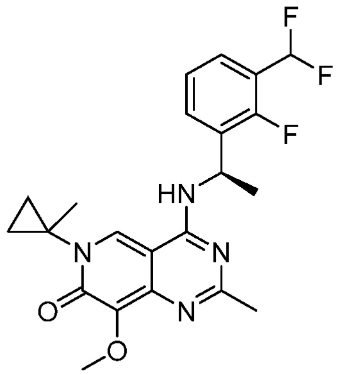

**SZ-022300** ,

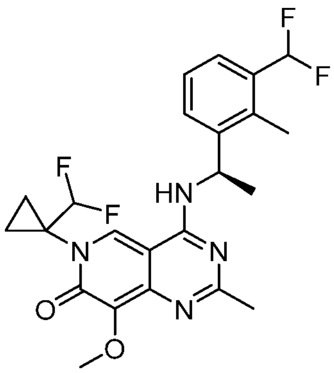

**SZ-022302** ,

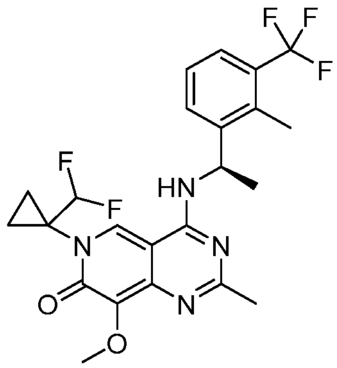

**SZ-022304** ,

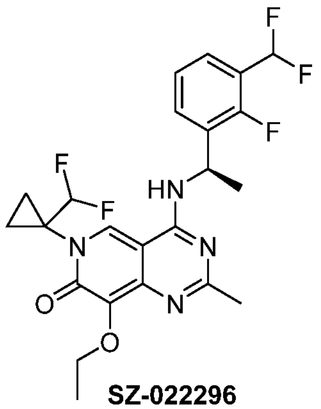

**SZ-022296** ,

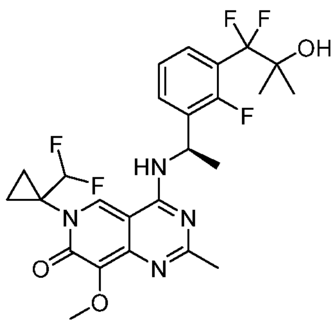

**SZ-022317** ,

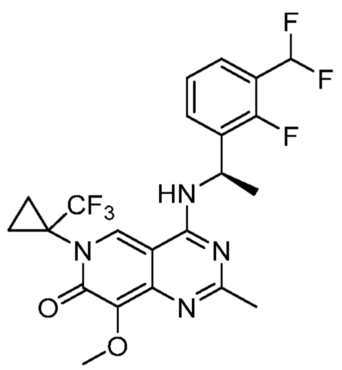

**SZ-022325** ,

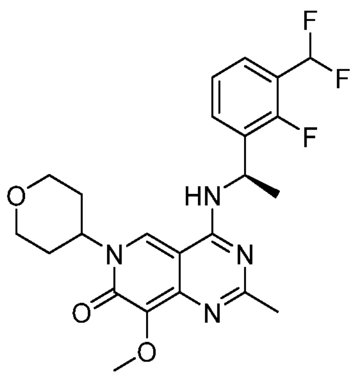

**SZ-022326** ,

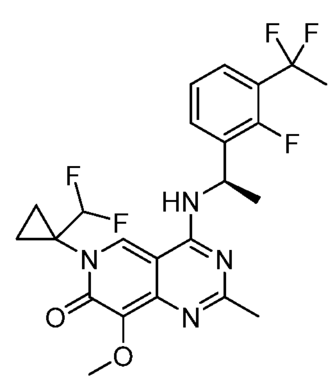

SZ-022303 ,

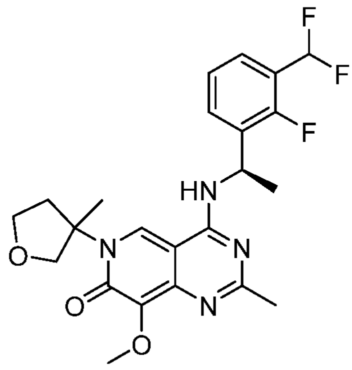

SZ-022328 ,

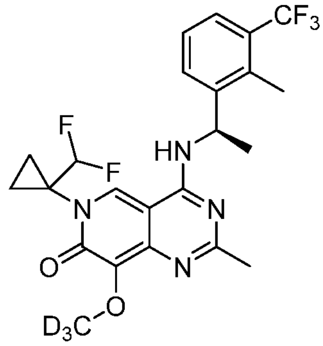

SZ-022334 ,

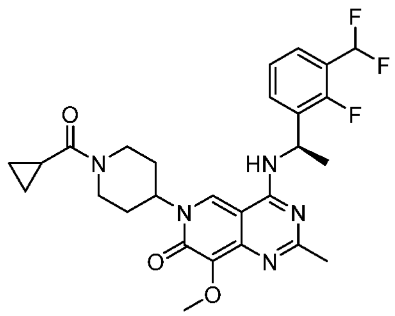

SZ-022335 ,

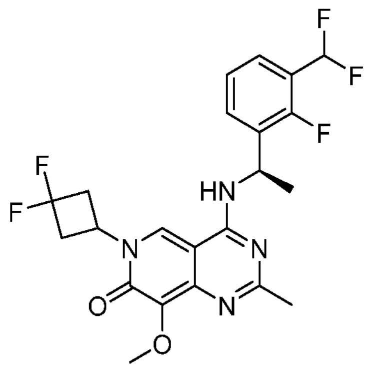

SZ-022336 ,

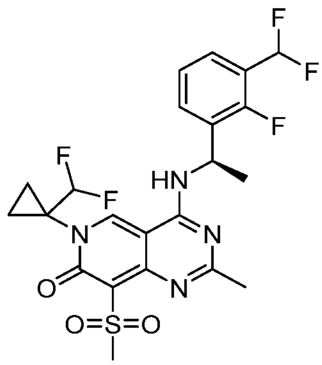

SZ-022307 ,

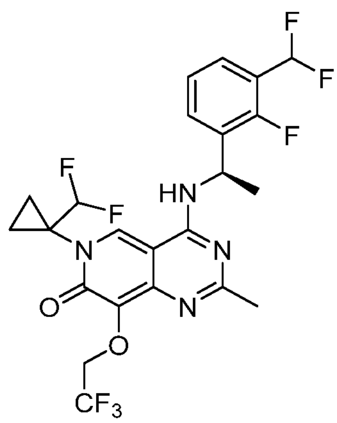

SZ-022312 ,

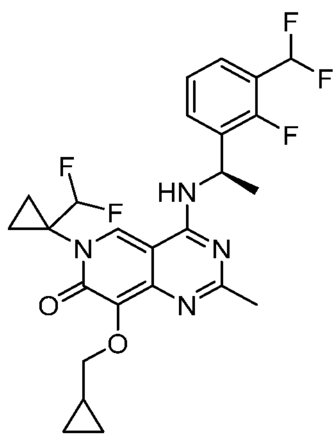

SZ-022314 ,

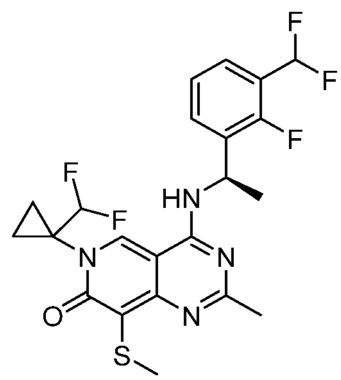

SZ-022306 ,

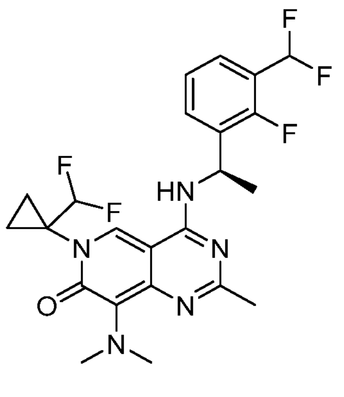

SZ-022251 ,

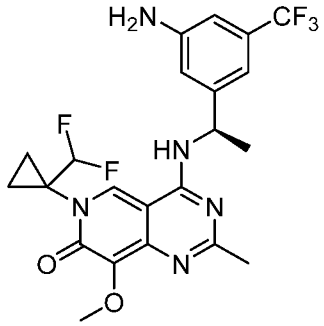

SZ-022327 ,

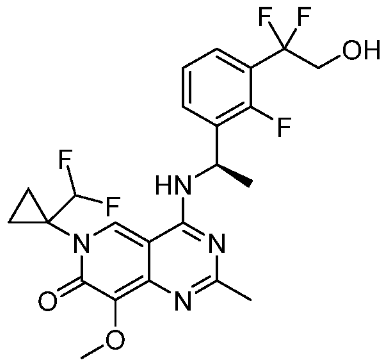

SZ-022329 ,

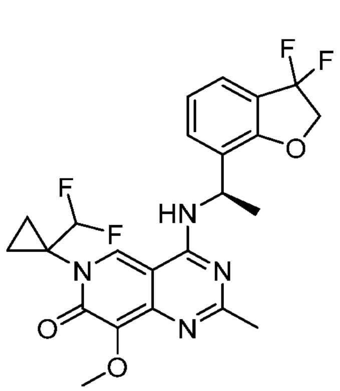

SZ-022330 ,

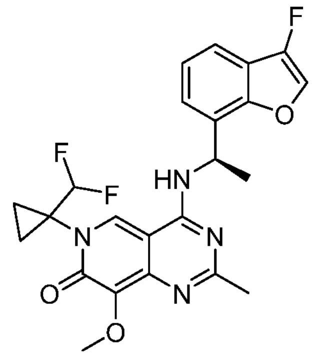

SZ-022330B ,

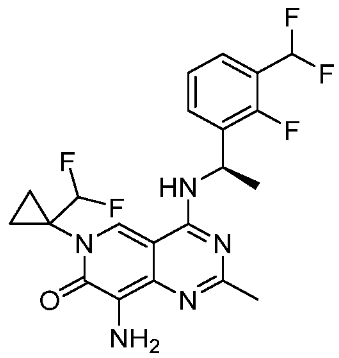

SZ-022224 ,

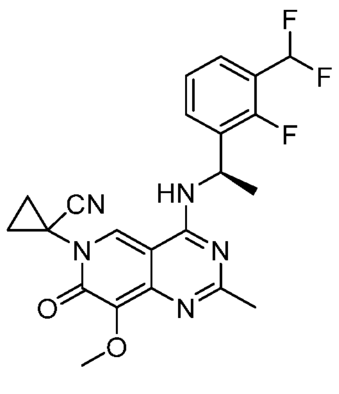

SZ-022344 ,

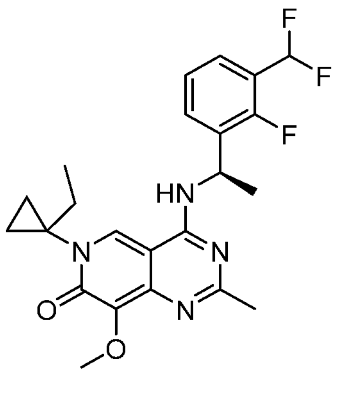

SZ-022343 ,

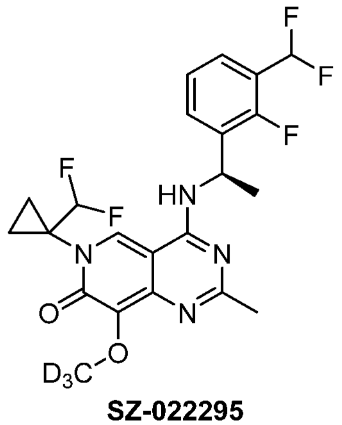

SZ-022295 ,

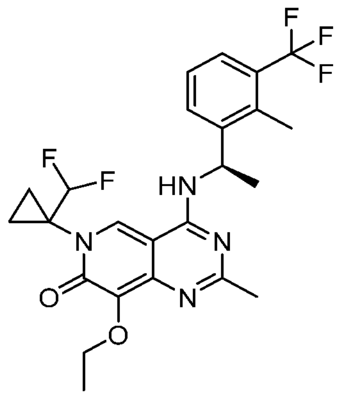

**SZ-022339** ,

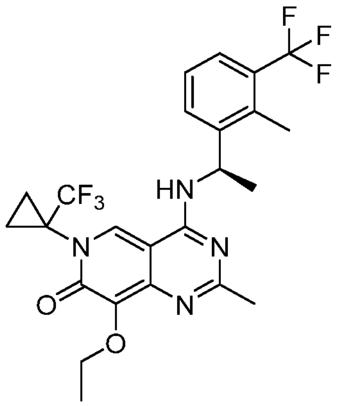

**SZ-022340** ,

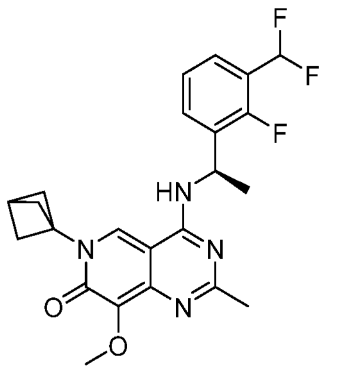

**SZ-022345** ,

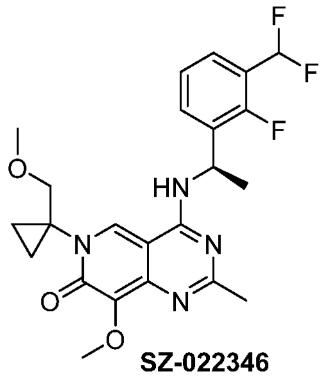

**SZ-022346** ,

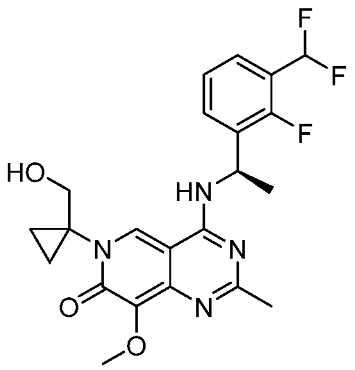

**SZ-022347** ,

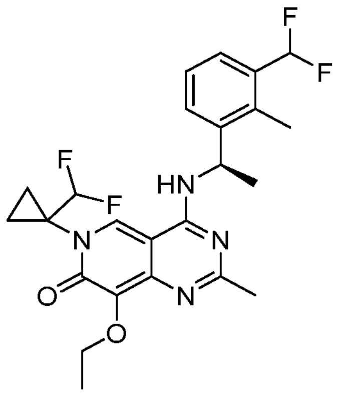

**SZ-022337** ,

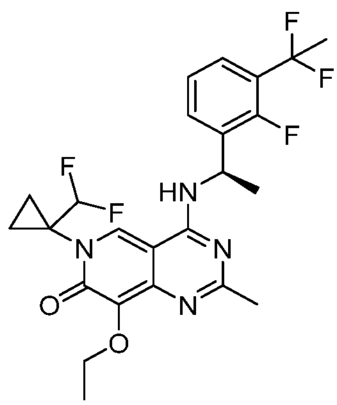

**SZ-022338** ,

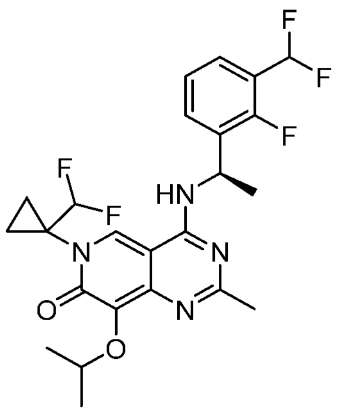

**SZ-022313** ,

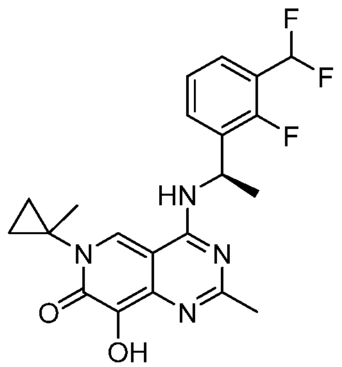

**SZ-022323** ,

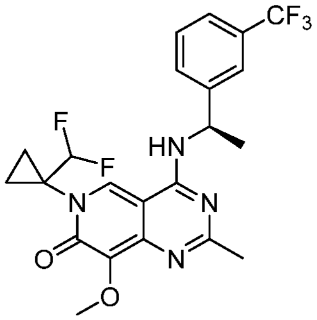
SZ-022348,

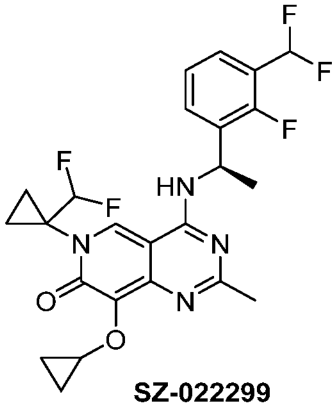
SZ-022299,

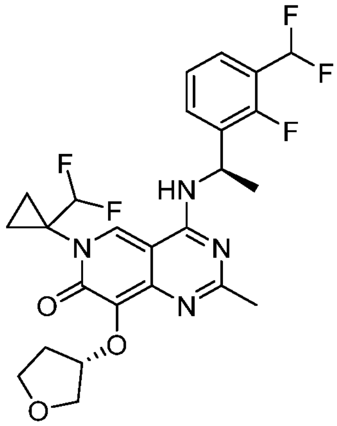
SZ-022351

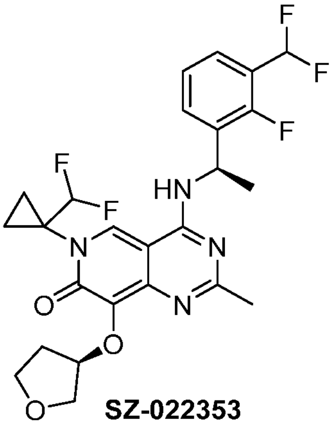
SZ-022353,

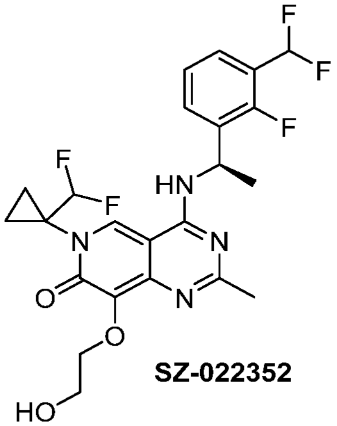
SZ-022352,

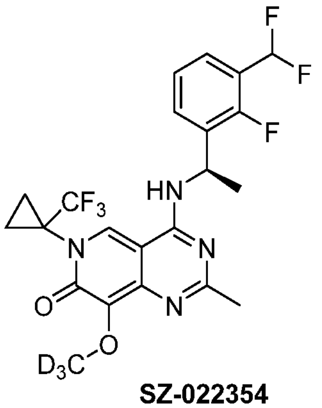
SZ-022354,

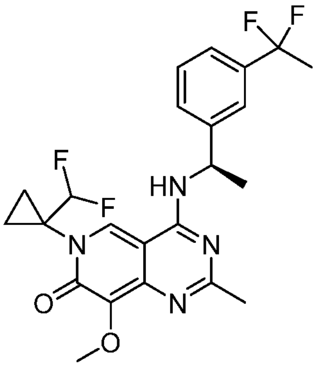
SZ-022350,

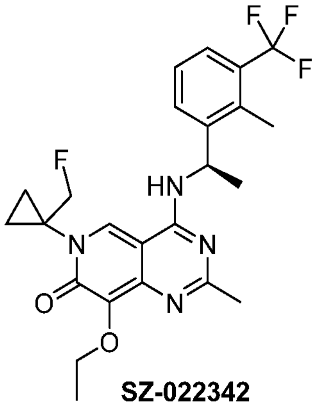
SZ-022342,

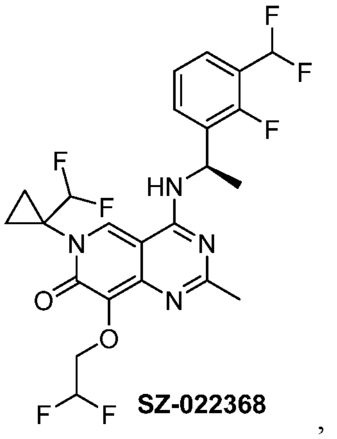
SZ-022368,

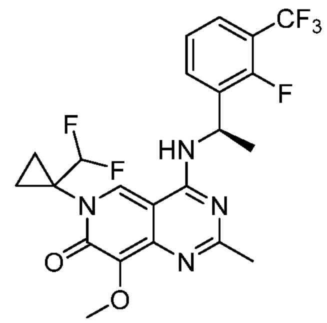
SZ-022349 ,

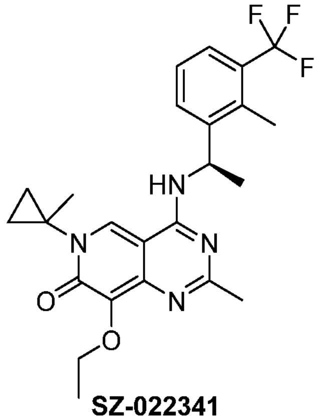
SZ-022341 ,

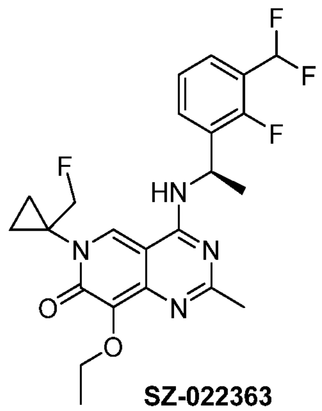
SZ-022363 ,

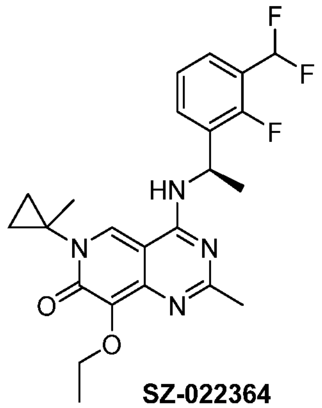
SZ-022364 ,

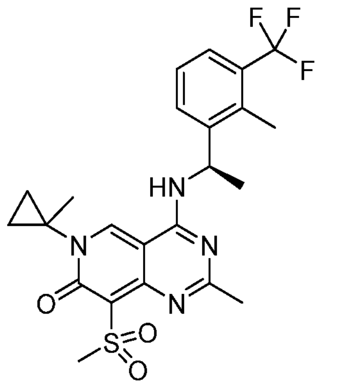
SZ-022371 ,

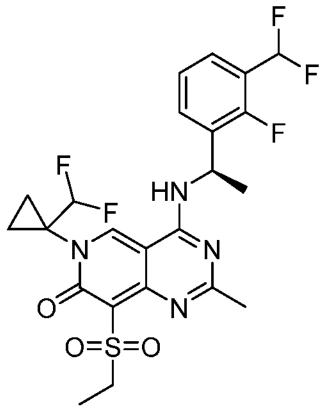
SZ-022372 ,

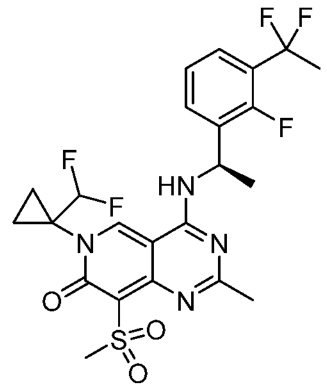
SZ-022370 ,

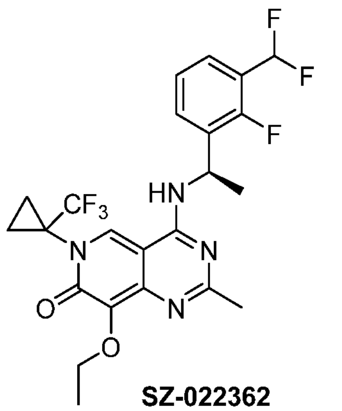
SZ-022362 ,

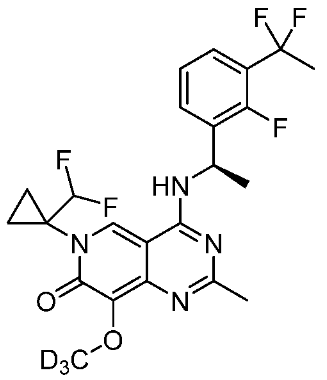
SZ-022355 ,

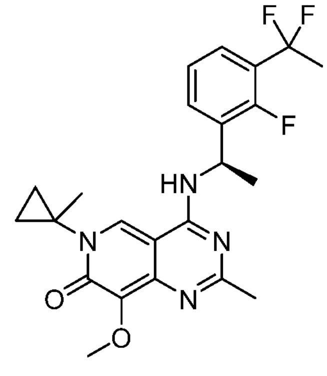

**SZ-022356**,

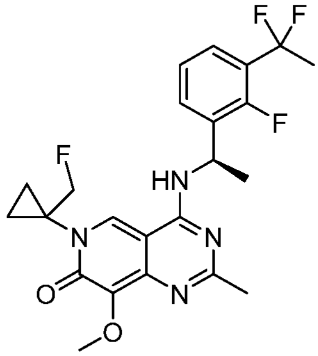

**SZ-022357**,

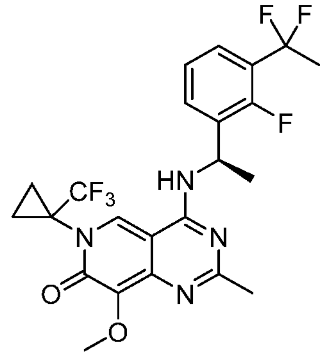

**SZ-022358**,

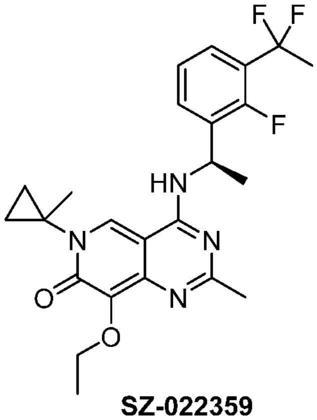

**SZ-022359**,

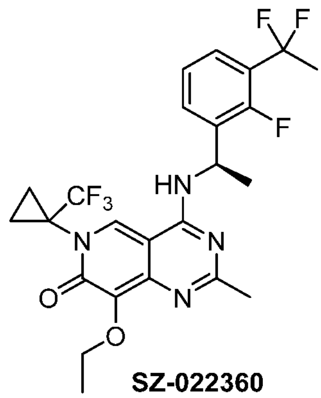

**SZ-022360**

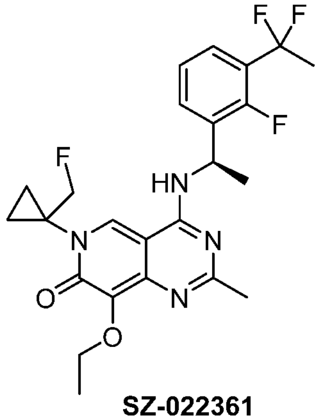

**SZ-022361**,

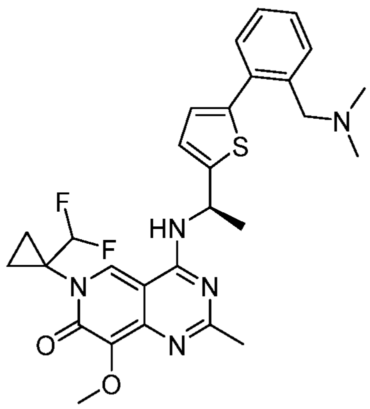

**SZ-022373**.

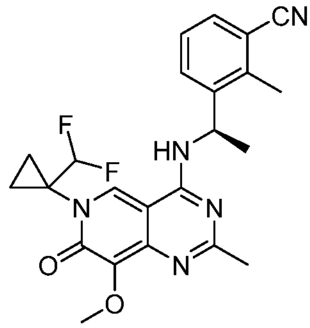

**SZ-022381**.

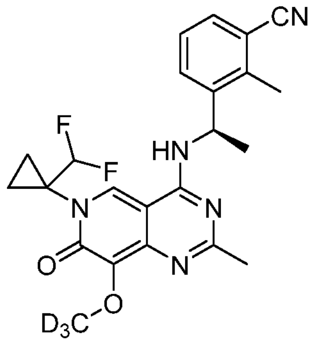

**SZ-022382**.

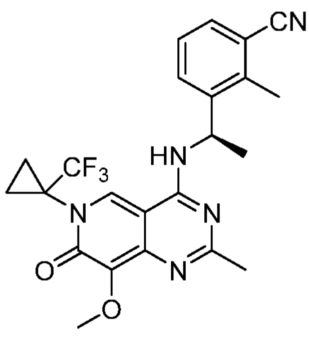

SZ-022383 , and

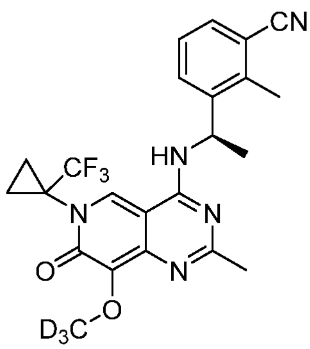

SZ-022384 .

The present invention provides a pharmaceutical composition, comprising:

(1) the fused ring compound represented by formula I or the pharmaceutically acceptable salt thereof as described above, and
(2) a pharmaceutically acceptable auxiliary material.

The present invention further provides use of the fused ring compound represented by formula I or the pharmaceutically acceptable salt as described above or the pharmaceutical composition as described above in the preparation of an SOS1 inhibitor.
In the use, a disease mediated by the SOS1 is lung cancer, pancreatic cancer, pancreatic ductal carcinoma, colon cancer, rectal cancer, appendiceal cancer, esophageal squamous carcinoma, head and neck squamous carcinoma, breast cancer, or other solid tumors.
In the use, the SOS1 inhibitor can be used in a mammalian organism; the SOS1 inhibitor can also be used *in vitro,* mainly for experimental purposes, for example, used as a standard sample or a control sample to provide comparison, or prepared into a kit according to a conventional method in the art to provide rapid detection for the SOS1 inhibitory effect.
The present invention further provides use of the fused ring compound represented by formula I or the pharmaceutically acceptable salt as described above or the pharmaceutical composition as described above in the preparation of a medicament, wherein the medicament is used for preventing and/or treating one or more of the following diseases: lung cancer, pancreatic cancer, pancreatic ductal carcinoma, colon cancer, rectal cancer, appendiceal cancer, esophageal squamous carcinoma, head and neck squamous carcinoma, breast cancer, and other solid tumors.

Definitions and Description

Unless otherwise stated, the following terms and phrases used herein are intended to have the following meanings. A particular term or phrase, unless otherwise specifically defined, should not be considered as indefinite or unclear, but construed according to its common meaning. When referring to a trade name, it is intended to refer to its corresponding commercial product or its active ingredient.
As will be understood by those skilled in the art, '

“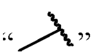”

used in the structural formulas describing groups herein means that the corresponding groups are connected to other fragments and groups in the compound through this site, according to conventions used in the art.
As used herein, a substituent may be preceded by a single dash "-" indicating that the named substituent is connected to a parent moiety by a single bond.
In the present invention, the term "pharmaceutically acceptable salt" refers to a salt prepared with the compound of the present invention and a relatively non-toxic, pharmaceutically acceptable acid or base.
In the present invention, the term "pharmaceutical auxiliary material" refers to excipients and additives used in the manufacture of a pharmaceutical product and in the formulation of pharmaceutical formulas, and refers to all substances, other than the active ingredient, contained in a pharmaceutical preparation. See Volume 4 of Pharmacopoeia of The People’s Republic of China (2015 Edition), or Handbook of Pharmaceutical Excipients (Raymond C Rowe, 2009

Sixth Edition).

In the present invention, the term "substituted" or "substituent" refers to the replacement of a hydrogen atom in a group by the designated group. When the substitution position is not specified, the substitution can be at any position, but is permissible only if it results in the formation of a stable or chemically feasible chemical. An example is given as follows: structure

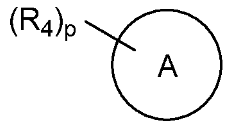

indicates that the hydrogen atom on ring A is replaced by p $R_4$.

When any variable (e.g., R) occurs more than once in the constitution or structure of a compound, the variable is independently defined in each case. Thus, for example, if a group is substituted with one or more R, the group can optionally be substituted with at least one R, and the definition of R in each case is independent. Furthermore, a combination of the substituent and/or the variant thereof is permissible only if the combination can result in a stable compound.

The term "more" refers to 2, 3, 4, or 5, preferably 2 or 3.

In the present invention, the term "alkyl" refers to a saturated linear or branched chain monovalent hydrocarbon group. $C_1$-$C_6$ alkyl refers to an alkyl group having 1-6 carbon atoms, preferably an alkyl group having 1-4 carbon atoms, specifically methyl, ethyl, n-propyl, isopropyl, n-butyl, isobutyl, sec-butyl, or tert-butyl.

In the present invention, the term "cycloalkyl" refers to a saturated monocyclic, bridged or spiro cyclic group having a specified number of ring carbon atoms (e.g., $C_{3-10}$), wherein the ring atoms are composed only of carbon atoms. The $C_{3-10}$ cycloalkyl can specifically be 3-, 4-, 5-, 6-, 7-, 8-, 9-, or 10-membered cycloalkyl, including cyclopropyl, cyclobutyl, cyclopentyl, cyclohexyl, and the like. Specific examples of the cycloalkyl include, but are not limited to, cyclopropyl, cyclobutyl, cyclopentyl, cyclohexyl, and

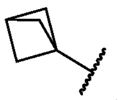.

In the present invention, the term "aryl" refers to an aromatic group in which at least one ring is aromatic, such as a benzene ring.

In the present invention, the term "heteroaryl" refers to an aromatic group containing a heteroatom, preferably an aromatic 5- to 6-membered monocyclic or 9- to 10-membered bicyclic ring containing 1, 2 or 3 heteroatoms independently selected from nitrogen, oxygen and sulfur (when the heteroaryl is bicyclic, each ring is aromatic), such as thienyl, furanyl, pyridinyl, benzofuranyl and the like.

In the present invention, the term "heterocyclyl" refers to a saturated or unsaturated, nonaromatic, monocyclic or polycyclic (e.g., fused, spiro, or bridged) cyclic group formed from carbon atoms and at least one heteroatom independently selected from N, O and S. Examples of the heterocyclyl include, but are not limited to

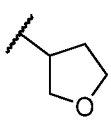, 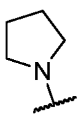, 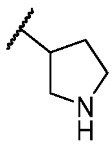, 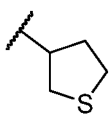, 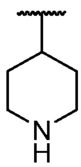,

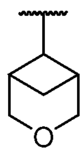, 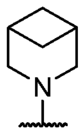, 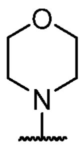, 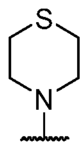, 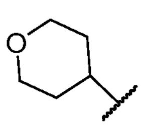,

or 2,3-dihydrobenzofuranyl. 5- to 10-membered heterocyclyl may specifically be 5-, 6-, 7-, 8-, 9- or 10-membered heterocyclyl. 3- to 10-membered heterocyclyl may specifically be 3-, 4-, 5-, 6-, 7-, 8-, 9- or 10-membered heterocyclyl.

In the present invention, the term "halogen" refers to fluorine, chlorine, bromine, or iodine.

In the present invention, the term "$C_{1-6}$ alkyl substituted with one or more halogens" refers to a group formed by substituting one or more (e.g., 2, 3, 4, 5, or 6) hydrogen atoms in an alkyl group with a halogen, wherein each halogen is independently F, Cl, Br, or I.

The above preferred conditions may be combined arbitrarily to obtain preferred embodiments of the present invention without departing from the general knowledge in the art.

The reagents and starting materials used in the present invention are commercially available.

The positive and progressive effects of the present invention are as follows: the compound has a novel structure and good SOS1 inhibitory activity.

## DETAILED DESCRIPTION

The present invention is further illustrated by the following examples, which are not intended to limit the present invention. Experimental procedures without specified conditions in the following examples were performed in accordance with conventional procedures and conditions, or in accordance with instructions.

Example 1: SZ-022244

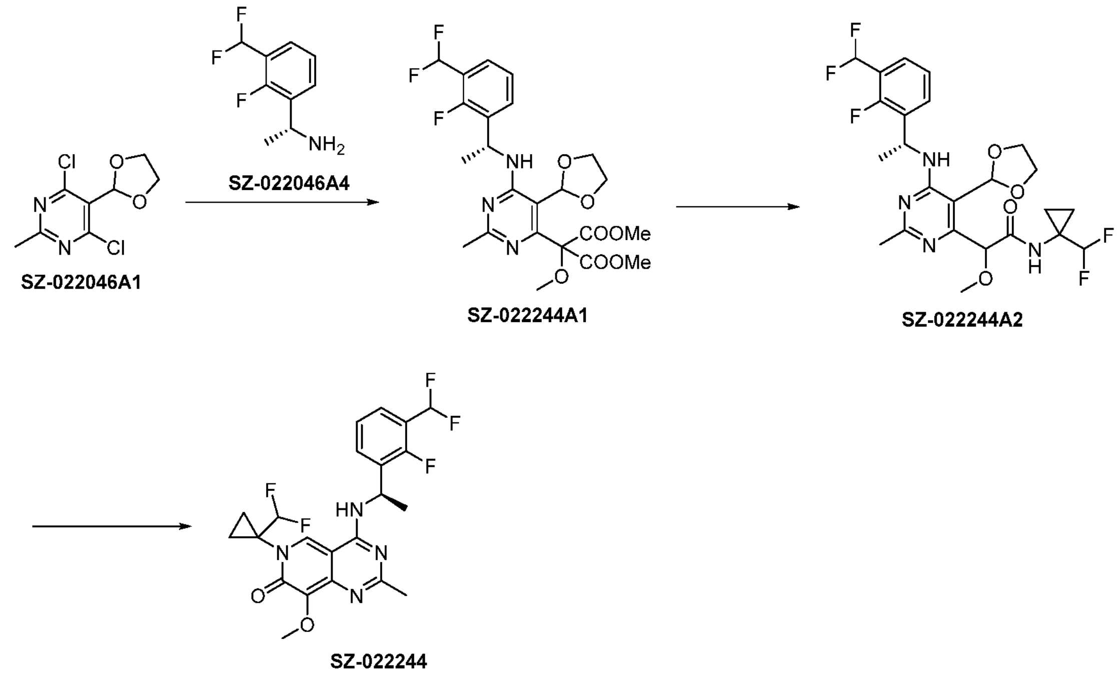

SZ-022046A1, SZ-022046A4, SZ-022244A1, SZ-022244A2, SZ-022244

Step 1:

Compound **SZ-022046A1** (800 mg, 3.48 mmol, synthesized according to WO2019122129), dimethyl methoxymalonate (560 mg, 3.48 mmol), and potassium carbonate (960 mg, 6.96 mmol) were added to dimethyl sulfoxide (10 mL), and the mixture was heated to 100 °C for reaction for 1 h. After the starting materials were consumed as monitored by LC-MS, the reaction mixture was cooled to 60 °C. **SZ-022046A4** (1.18 g, 5.22 mmol, synthesized according to WO2019122129) and triethylamine (528 mg, 5.22 mmol) were added, and the resulting mixture was allowed to react at 60 °C for 3 h. After the starting materials were consumed as monitored by LC-MS, the reaction mixture was cooled to room temperature. Water (50 mL) and ethyl acetate (50 mL) were added to the reaction mixture for extraction, and the organic layer was separated, dried, and concentrated by rotary evaporation to give a crude product. The crude product was purified by flash silica gel column chromatography (PE:EA = 3:1; Rf = 0.4) to give product **SZ-022244A1** as a yellow oil (170 mg). LC-MS: $[M+H]^+$ 514.15.

Step 2:

**SZ-022244A1** (170 mg, 0.33 mmol) was added to dimethyl sulfoxide (2 mL), and then a 20% sodium hydroxide

solution (150 μL) was added. The mixture was allowed to react at 40 °C for 2 h. 1-(Difluoromethyl)cyclopropan-1-amine hydrochloride (62 mg, 0.43 mmol), HATU (189 mg, 0.50 mmol), and triethylamine (67 mg, 0.66 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022244A2** as a yellow viscous substance (170 mg). LC-MS: $[M+H]^+$ 531.19.

Step 3:

**SZ-022244A2** (170 mg, 0.32 mmol) was added to isopropanol (2 mL), and then a hydrochloric acid solution (2 M, 0.8 mL) was added. The mixture was allowed to react at 50 °C for 4 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to reversed-phase column chromatography (acetonitrile/water with 0.1% formic acid) and then lyophilized to give **SZ-022244** (49.1 mg). LC-MS: $[M+H]^+$ 469.13.

**SZ-022244:** $^1$H NMR (400 MHz, DMSO-$d_6$) δ 8.92 (s, 1H), 8.79 (d, *J* = 6.8 Hz, 1H), 7.64 (t, *J* = 7.6 Hz, 1H), 7.52 (t, *J* = 7.2 Hz, 1H), 7.32 (t, *J* = 8 Hz, 1H), 7.23 (t, *J* = 56 Hz, 1H), 6.34 (t, *J* = 56 Hz, 1H), 5.80-5.70 (m, 1H), 3.78 (s, 3H), 2.21 (s, 3H), 1.58 (d, *J* = 7.2 Hz, 3H), 1.53-1.46 (m, 2H), 1.45-1.33 (m, 2H).

**Example 2: SZ-022300**

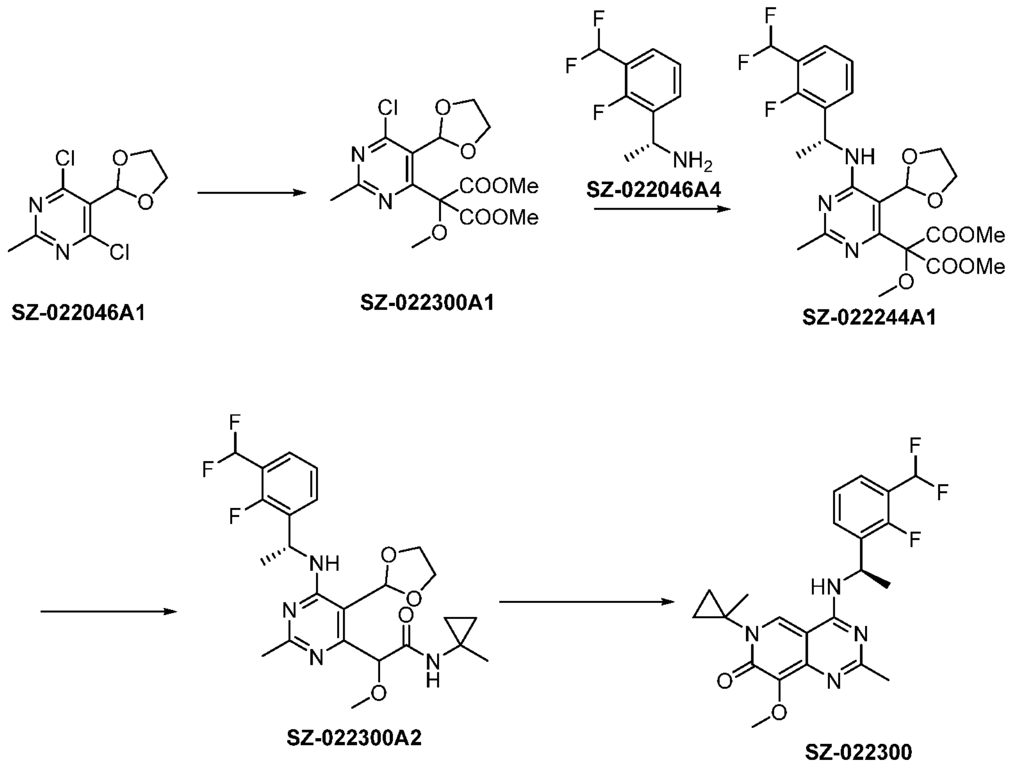

**SZ-022046A1** **SZ-022300A1** **SZ-022046A4** **SZ-022244A1**

**SZ-022300A2** **SZ-022300**

Step 1:

SZ-022046A1 (3.5 g, 15 mmol, synthesized according to WO2019122129) was added to dimethyl sulfoxide (30 mL), and the mixture was heated to 100 °C. Potassium carbonate (4.14 g, 30 mmol) and dimethyl methoxymalonate (3 g, 18.5 mmol) were then added, and the mixture was allowed to react for 2 h with the temperature maintained at 100 °C. After the starting materials were consumed as monitored by LC-MS, water (100 mL) and ethyl acetate (100 mL) were added to the reaction mixture for extraction, and the organic layer was separated, washed with a saturated sodium

chloride solution, dried, and concentrated by rotary evaporation to give a crude product. The crude product was purified by flash silica gel column chromatography (PE:EA = 3:1; Rf = 0.4) to give product **SZ-022300A1** as a yellow oil (3 g). LC-MS: $[M+H]^+$ 361.11, 363.11.

Step 2:

**SZ-022300A1** (670 mg, 1.86 mmol), **SZ-022046A4** (462 mg, 2 mmol, synthesized according to WO2019122129), and triethylamine (566 mg, 5.56 mmol) were added to dimethyl sulfoxide (5 mL), and the mixture was heated to 60 °C for reaction for 8 h. After the starting materials were consumed as monitored by LC-MS, water (100 mL) and ethyl acetate (100 mL) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA = 3:1; Rf = 0.35) to give product **SZ-022244A1** as a yellow oil (540 mg). LC-MS: $[M+H]^+$ 514.19.

Step 3:

**SZ-022244A1** (150 mg, 0.29 mmol) was added to dimethyl sulfoxide (2 mL), and then a 20% sodium hydroxide solution (150 μL) was added. The mixture was allowed to react at 40 °C for 1 h. 1-Methylcyclopropanamine hydrochloride (41 mg, 0.38 mmol), HATU (167 mg, 0.44 mmol), and triethylamine (59 mg, 0.58 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022300A2** as a yellow viscous substance (150 mg). LC-MS: $[M+H]^+$ 494.89.

Step 4:

**SZ-022300A2** (150 mg, 0.30 mmol) was added to isopropanol (2 mL), and then a hydrochloric acid solution (2 M, 0.75 mL) was added. The mixture was allowed to react at 50 °C for 4 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022300** (66 mg). LC-MS: $[M+H]^+$ 432.94.

**SZ-022300:** $^1$H NMR (400 MHz, DMSO-$d_6$) δ 8.99 (s, 1H), 8.69 (d, *J* = 7.2 Hz, 1H), 7.66 (t, *J* = 7.6 Hz, 1H), 7.52 (t, *J* = 7.2 Hz, 1H), 7.31 (t, *J* = 7.6 Hz, 1H), 7.23 (t, *J* = 54.4 Hz, 1H), 5.80-5.70 (m, 1H), 3.75 (s, 3H), 2.20 (s, 3H), 1.58 (d, *J* = 7.2 Hz, 3H), 1.52 (s, 3H), 1.17-1.09 (m, 2H), 1.09-1.00 (m, 2H).

**Example 3:** SZ-022296

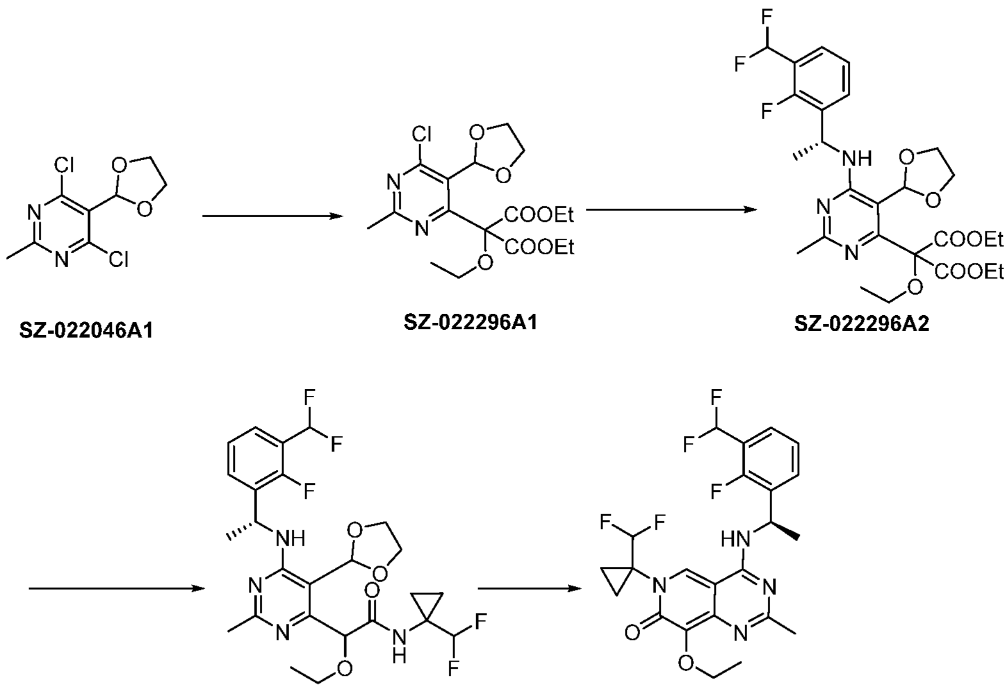

SZ-022046A1 SZ-022296A1 SZ-022296A2

SZ-022296A3 SZ-022296

Step 1:

Compound **SZ-022046A1** (500 mg, 2.12 mmol, synthesized according to WO2019122129), diethyl ethoxymalonate (502 mg, 2.55 mmol), and potassium carbonate (560 mg, 4.25 mmol) were added to dimethyl sulfoxide (5 mL), and the mixture was heated to 100 °C for reaction for 1 h. After the starting materials were consumed as monitored by LC-MS, the reaction mixture was cooled to room temperature. Water (50 mL) and ethyl acetate (50 mL) were added to the reaction mixture for extraction, and the organic layer was separated, dried, and concentrated by rotary evaporation to give a crude product. The crude product was purified by flash silica gel column chromatography (PE:EA = 5:1; Rf = 0.4) to give product **SZ-022296A1** as a colorless oil (310 mg). LC-MS: $[M+H]^+$ 402.8.

Step 2:

Compound **SZ-022296A1** (310 mg, 0.77 mmol), (1*R*)-1-(3-(difluoromethyl)-2-fluorophenyl)ethylamine hydrochloride (208 mg, 0.92 mmol, synthesized according to WO2019122129), and triethylamine (234 mg, 2.31 mmol) were allowed to react at 60 °C for 3 h. After the starting materials were consumed as monitored by LC-MS, the reaction mixture was cooled to room temperature. Water (50 mL) and ethyl acetate (50 mL) were added to the reaction mixture for extraction, and the organic layer was separated, dried, and concentrated by rotary evaporation to give a crude product. The crude product was purified by flash silica gel column chromatography (PE:EA= 3:1; Rf = 0.3) to give product **SZ-022296A2** as a light yellow oil (110 mg). LC-MS: $[M+H]^+$ 556.09.

Step 3:

**SZ-022296A2** (110 mg, 0.20 mmol) was added to dimethyl sulfoxide (2 mL), and then a 20% sodium hydroxide solution (100 μL) was added. The mixture was allowed to react at 35 °C for 2 h. 1-(Difluoromethyl)cyclopropan-1-amine hydrochloride (62 mg, 0.43 mmol), HATU (189 mg, 0.50 mmol), and triethylamine (37 mg, 0.26 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022296A3** as a yellow viscous substance (120 mg). LC-MS: $[M+H]^+$ 545.04.

Step 4:

**SZ-022296A3** (120 mg, 0.20 mmol) was added to isopropanol (2 mL), and then a hydrochloric acid solution (2

M, 0.5 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022296** (43 mg). LC-MS: $[M+H]^+$ 482.91.

**SZ-022296:** $^1$H NMR (400 MHz, DMSO-$d_6$) δ 8.91 (s, 1H), 8.80 (d, *J* = 7.6 Hz, 1H), 7.64 (t, *J* = 7.2 Hz, 1H), 7.52 (t, *J* = 6.8Hz, 1H), 7.32 (t, *J* = 7.6 Hz, 1H), 7.23 (t, *J* = 54.4 Hz, 1H), 6.34 (t, *J* = 56.8 Hz, 1H), 5.81-5.71 (m, 1H), 4.08 (q, *J* = 7.2 Hz, 2H), 2.21 (s, 3H), 1.58 (d, *J* = 7.2 Hz, 3H), 1.52-1.45 (m, 2H), 1.44-1.32 (m, 2H), 1.22 (t, *J* = 7.20 Hz, 3H).

**Example 4: SZ-022301**

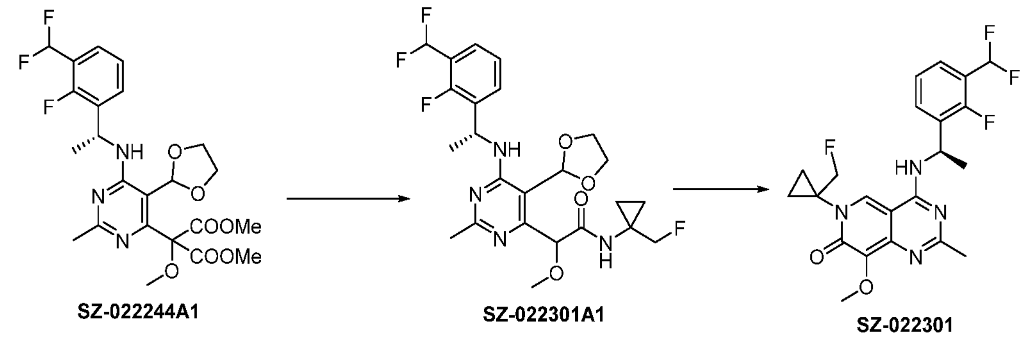

SZ-022244A1 SZ-022301A1 SZ-022301

Step 1:

**SZ-022244A1** (190 mg, 0.37 mmol) was added to dimethyl sulfoxide (2 mL), and then a 20% sodium hydroxide solution (150 μL) was added. The mixture was allowed to react at 40 °C for 2 h. 1-(Monofluoromethyl)cyclopropanamine hydrochloride (61 mg, 0.48 mmol), HATU (212 mg, 0.56 mmol), and triethylamine (75 mg, 0.74 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022301A1** as a yellow viscous substance (190 mg). LC-MS: $[M+H]^+$ 513.22.

Step 2:

**SZ-022301A1** (190 mg, 0.37 mmol) was added to isopropanol (2 mL), and then a hydrochloric acid solution (2 M, 0.93 mL) was added. The mixture was allowed to react at 50 °C for 4 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022301** (53 mg). LC-MS: $[M+H]^+$ 450.92.

**SZ-022301:** $^1$H NMR (400 MHz, DMSO-$d_6$) δ 8.96 (s, 1H), 8.90-8.805(br, 1H), 7.64 (t, *J* = 7.6 Hz, 1H), 7.52 (t, *J* = 7.2 Hz, 1H), 7.32 (t, *J* = 7.6 Hz, 1H), 7.23 (t, *J* = 54.4 Hz, 1H), 5.81-5.71 (m, 1H), 4.80-4.47 (m, 2H), 3.77 (s, 3H), 2.22 (s, 3H), 1.58 (d, *J* = 7.2 Hz, 3H), 1.38-1.22 (m, 4H).

**Example 5: SZ-022302**

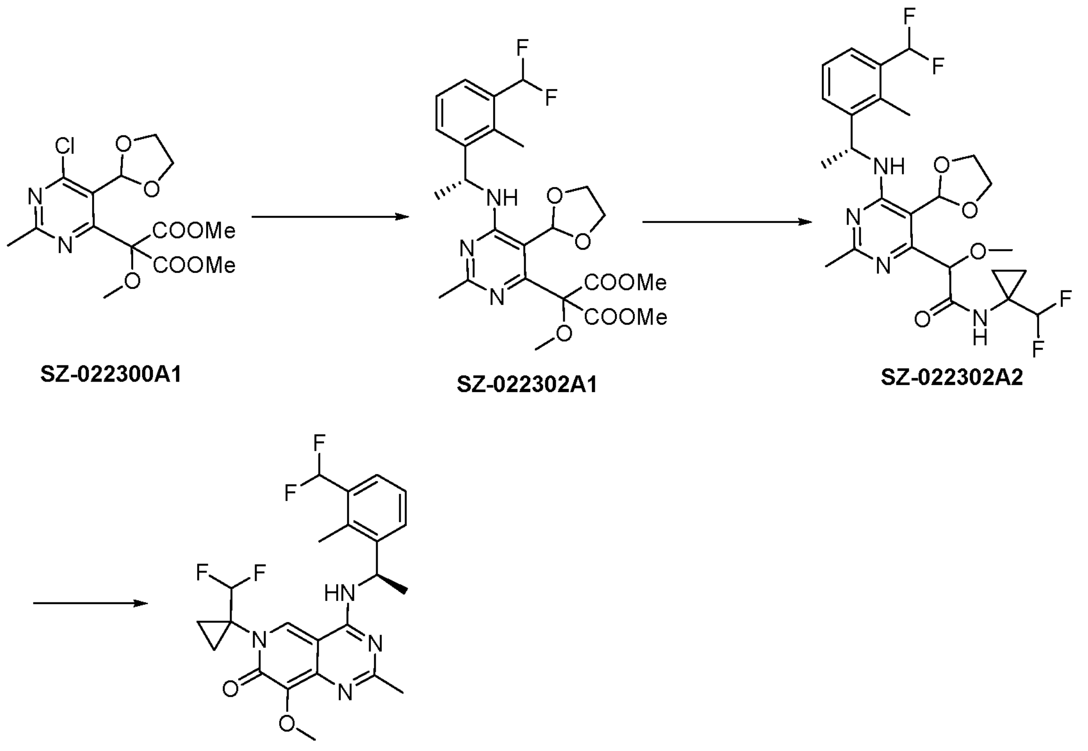

SZ-022300A1

SZ-022302A1

SZ-022302A2

SZ-022302

Step 1:

Compound **SZ-022300A1** (200 mg, 0.55 mmol), (1*R*)-1-(3-(difluoromethyl)-2-methylphenyl)ethylamine hydrochloride (147 mg, 0.67 mmol, synthesized according to WO2019122129), and triethylamine (112 mg, 1.11 mmol) were allowed to react at 60 °C for 3 h. After the starting materials were consumed as monitored by LC-MS, the reaction mixture was cooled to room temperature. Water (50 mL) and ethyl acetate (50 mL) were added to the reaction mixture for extraction, and the organic layer was separated, dried, and concentrated by rotary evaporation to give a crude product. The crude product was purified by flash silica gel column chromatography (PE:EA = 3:1; Rf = 0.2) to give product **SZ-022302A1** as a colorless oil (100 mg). LC-MS: $[M+H]^+$ 510.92.

Step 2:

**SZ-022302A1** (100 mg, 0.19 mmol) was added to dimethyl sulfoxide (2 mL), and then a 20% sodium hydroxide solution (100 μL) was added. The mixture was allowed to react at 35 °C for 2 h. 1-(Difluoromethyl)cyclopropan-1-amine hydrochloride (37 mg, 0.26 mmol), HATU (111 mg, 0.29 mmol), and triethylamine (40 mg, 0.39 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022302A2** as a yellow viscous substance (180 mg). LC-MS: $[M+H]^+$ 527.11.

Step 3:

**SZ-022302A2** (180 mg, 0.19 mmol) was added to isopropanol (2 mL), and then a hydrochloric acid solution (2 M, 0.5 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022302** (45 mg). LC-MS: $[M+H]^+$ +465.17.

**SZ-022302:** $^1$H NMR (400 MHz, DMSO-$d_6$) δ 8.91 (s, 1H), 8.83 (d, *J* = 7.2 Hz, 1H), 7.60 (d, *J* = 8.0 Hz, 1H), 7.42 (d, *J*= 8.0 Hz, 1H), 7.34 (d, *J* = 8.0 Hz, 1H), 7.21 (t, *J* = 44.8 Hz, 1H), 6.34 (t, *J* = 57.2 Hz, 1H), 5.78-5.68 (m, 1H),

3.77 (s, 3H), 2.51 (s, 3H), 2.24 (s, 3H), 1.53 (d, *J* = 7.2 Hz, 3H), 1.51-1.45 (m, 2H), 1.44-1.32 (m, 2H).

**Example 6: SZ-022304**

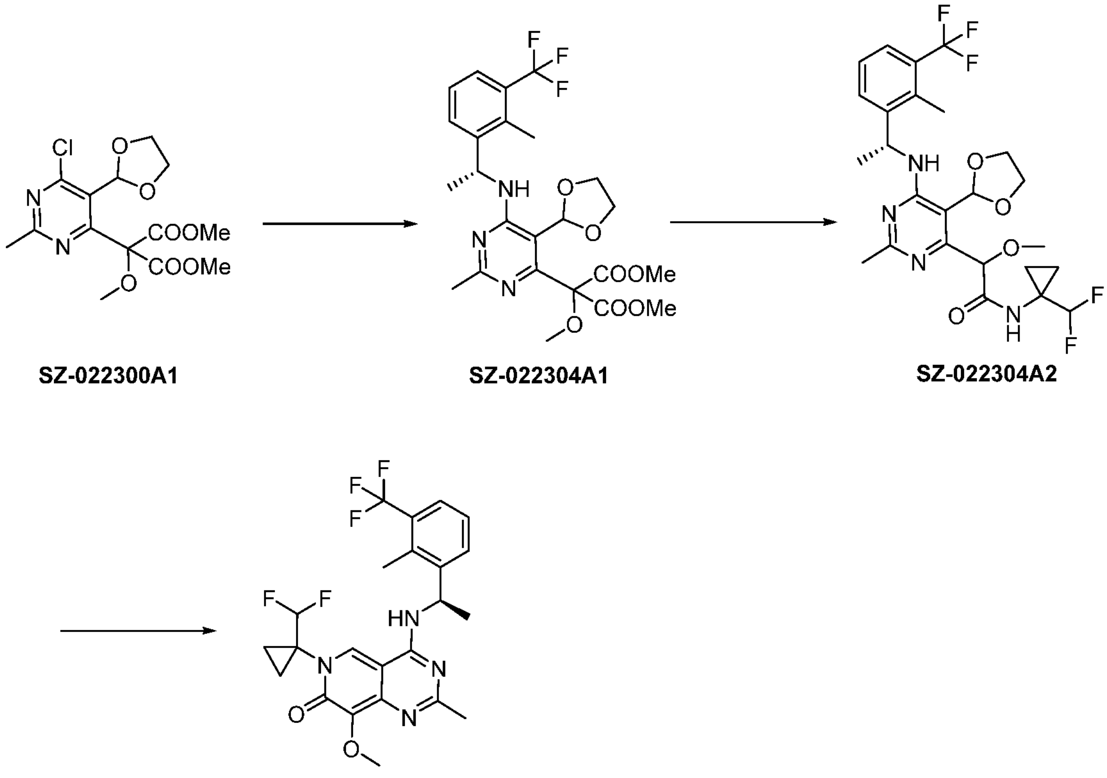

**SZ-022300A1** **SZ-022304A1** **SZ-022304A2**

**SZ-022304**

Step 1:

Compound **SZ-022300A1** (200 mg, 0.55 mmol), (1*R*)-1-(3-(trifluoromethyl)-2-methylphenyl)ethylamine hydrochloride (170 mg, 0.71 mmol, synthesized according to WO2019122129), and triethylamine (112 mg, 1.11 mmol) were allowed to react at 60 °C for 3 h. After the starting materials were consumed as monitored by LC-MS, the reaction mixture was cooled to room temperature. Water (50 mL) and ethyl acetate (50 mL) were added to the reaction mixture for extraction, and the organic layer was separated, dried, and concentrated by rotary evaporation to give a crude product. The crude product was purified by flash silica gel column chromatography (PE:EA = 3:1; Rf = 0.3) to give product **SZ-022304A1** as a colorless oil (150 mg). LC-MS: $[M+H]^+$ 528.22.

Step 2:

**SZ-022304A1** (150 mg, 0.28 mmol) was added to dimethyl sulfoxide (2 mL), and then a 20% sodium hydroxide solution (100 μL) was added. The mixture was allowed to react at 35 °C for 2 h. 1-(Difluoromethyl)cyclopropan-1-amine hydrochloride (53 mg, 0.37 mmol), HATU (162 mg, 0.43 mmol), and triethylamine (58 mg, 0.57 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022304A2** as a light yellow viscous substance (150 mg). LC-MS: $[M+H]^+$ 545.08.

Step 3:

**SZ-022304A2** (150 mg, 0.28 mmol) was added to isopropanol (2 mL), and then a hydrochloric acid solution (2 M, 0.5 mL) was added. The mixture was allowed to react at 50 °C for 3 h. After the starting materials were consumed

as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022304** (65 mg). LC-MS: $[M+H]^+$ 483.13.

**SZ-022304:** $^1$H NMR (400 MHz, DMSO-$d_6$) δ 9.56 (s, 1H), 9.03 (s, 1H), 7.72 (d, *J* = 7.6 Hz, 1H), 7.60 (d, *J* = 7.6 Hz, 1H), 7.42 (t, *J* = 8.0 Hz, 1H), 6.32 (t, *J* = 56.4 Hz, 1H), 5.83-5.73 (m, 1H), 3.81 (s, 3H), 2.56 (s, 3H), 2.33 (s, 3H), 1.58 (d, *J* = 6.8 Hz, 3H), 1.55-1.49 (m, 2H), 1.47-1.35 (m, 2H).

**Example 7: SZ-022317**

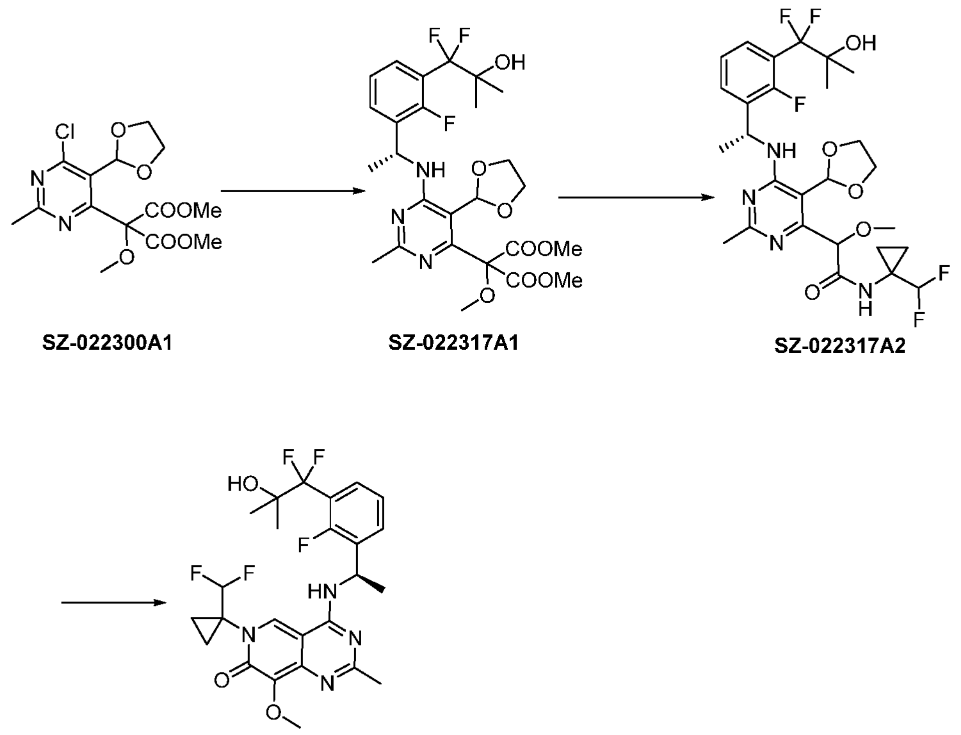

SZ-022300A1 SZ-022317A1 SZ-022317A2

SZ-022317

Step 1:

Compound **SZ-022300A1** (200 mg, 0.55 mmol), (1*R*)-1-(3-(1,1-difluoro-2-hydroxy-isobutyl)-2-fluorophenyl)ethylamine (135 mg, 0.55 mmol, synthesized according to WO2019122129), and triethylamine (112 mg, 1.11 mmol) were allowed to react at 60 °C for 3 h. After the starting materials were consumed as monitored by LC-MS, the reaction mixture was cooled to room temperature. Water (50 mL) and ethyl acetate (50 mL) were added to the reaction mixture for extraction, and the organic layer was separated, dried, and concentrated by rotary evaporation to give a crude product. The crude product was purified by flash silica gel column chromatography (PE:EA = 3:1; Rf = 0.3) to give product **SZ-022317A1** as a colorless oil (100 mg). LC-MS: $[M+H]^+$ 572.06.

Step 2:

**SZ-022317A1** (100 mg, 0.17 mmol) was added to dimethyl sulfoxide (2 mL), and then a 20% sodium hydroxide solution (100 μL) was added. The mixture was allowed to react at 35 °C for 2 h. 1-(Difluoromethyl)cyclopropan-1-amine hydrochloride (33 mg, 0.23 mmol), *N,N,N',N'*-tetramethyl-*O*-(7-azabenzotriazol-1-yl)uronium hexafluorophosphate (135 mg, 0.36 mmol), and triethylamine (42 mg, 0.40 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product

**SZ-022317A2** as a white viscous substance (95 mg). LC-MS: $[M+H]^+$ 588.98.

Step 3:

**SZ-022317A2** (95 mg, 0.16 mmol) was added to isopropanol (2 mL), and then a hydrochloric acid solution (2 M, 0.5 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022317** (48 mg). LC-MS: $[M+H]^+$ 527.18.

**SZ-022317:** $^1$H NMR (400 MHz, DMSO-$d_6$) δ 8.93 (s, 1H), 8.79 (d, *J* = 7.2 Hz, 1H), 7.55 (t, *J* = 6.4 Hz, 1H), 7.34 (t, *J* = 6.4 Hz, 1H), 7.24 (t, *J* = 7.6 Hz, 1H), 6.34 (t, *J* = 56.8 Hz, 1H), 5.78 - 5.71 (m, 1H), 5.32 (s, 1H), 3.77 (s, 3H), 2.21 (s, 3H), 1.56 (d, *J* = 7.2 Hz, 3H), 1.52 - 1.48 (m, 2H), 1.43 - 1.35 (m, 2H), 1.22 (s, 3H), 1.20 (s, 3H).

**Example 8: SZ-022325**

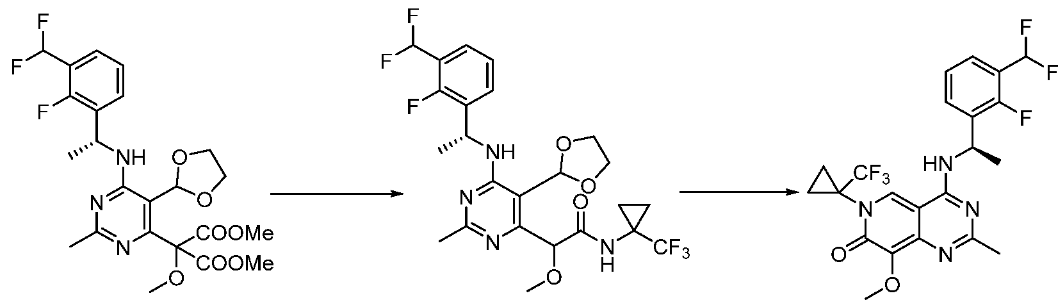

SZ-022244A1 SZ-022325A1 SZ-022325

Step 1:

**SZ-022244A1** (120 mg, 0.23 mmol) was added to dimethyl sulfoxide (2 mL), and then a 20% sodium hydroxide solution (100 μL) was added. The mixture was allowed to react at 40 °C for 2 h. 1-Trifluoromethylcyclopropanamine hydrochloride (57 mg, 0.35 mmol), *N,N,N',N'*-tetramethyl-O-(7-azabenzotriazol-1-yl)uronium hexafluorophosphate (267 mg, 0.70 mmol), and triethylamine (71 mg, 0.70 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022325A1** as a yellow viscous substance (120 mg). LC-MS: $[M+H]^+$ 549.02.

Step 2:

**SZ-022325A1** (120 mg, 0.22 mmol) was added to isopropanol (2 mL), and then a hydrochloric acid solution (2 M, 0.50 mL) was added. The mixture was allowed to react at 50 °C for 4 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022325** (53 mg). LC-MS: $[M+H]^+$ 487.10.

**SZ-022325**:$^1$H NMR (400 MHz, DMSO-$d_6$) δ 8.99 (s, 1H), 8.81 (d, *J* = 7.2 Hz, 1H), 7.63 (t, *J* = 7.2 Hz, 1H), 7.52 (t, *J* = 7.2 Hz, 1H), 7.33 (t, *J* = 6.8 Hz, 1H), 7.23 (t, *J* = 54 Hz, 1H), 5.80 - 5.70 (m, 1H), 3.76 (s, 3H), 2.21 (s, 3H), 1.88 - 1.48 (m, 4H), 1.68 (d, *J* = 6.8 Hz, 3H).

**Example 9: SZ-022326**

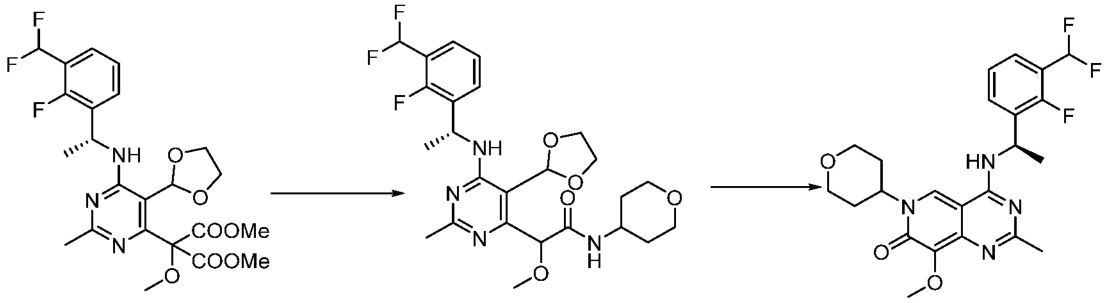

SZ-022244A1 SZ-022326A1 SZ-022326

Step 1:

**SZ-022244A1** (120 mg, 0.23 mmol) was added to dimethyl sulfoxide (2 mL), and then a 20% sodium hydroxide solution (100 μL) was added. The mixture was allowed to react at 40 °C for 2 h. 4-Aminotetrahydropyran hydrochloride (48 mg, 0.35 mmol), *N*,*N*,*N'*,*N'*-tetramethyl-O-(7-azabenzotriazol-1-yl)uronium hexafluorophosphate (267 mg, 0.70 mmol), and triethylamine (71 mg, 0.70 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022326A1** as a yellow viscous substance (120 mg). LC-MS: $[M+H]^+$ 525.12.

Step 2:

**SZ-022326A1** (120 mg, 0.23 mmol) was added to isopropanol (2 mL), and then a hydrochloric acid solution (2 M, 0.60 mL) was added. The mixture was allowed to react at 50 °C for 4 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022326** (53 mg). LC-MS: $[M+H]^+$ 463.17.

**SZ-022326:** $^1$H NMR (400 MHz, DMSO-$d_6$) δ 8.90 (s, 1H), 8.71 (d, *J* = 7.2 Hz, 1H), 7.64 (t, *J* = 7.6 Hz, 1H), 7.52 (t, *J* = 7.2 Hz, 1H), 7.32 (t, *J* = 7.6 Hz, 1H), 7.23 (t, *J* = 54 Hz, 1H), 5.78 - 5.70 (m, 1H), 5.27 - 5.18 (m, 1H), 4.09 - 4.03 (m, 2H), 3.76 (s, 3H), 3.58 - 3.50 (m, 2H), 2.22 (s, 3H), 2.11 - 2.02 (m, 2H), 1.83 - 1.77 (m, 2H), 1.59 (d, *J* = 7.2 Hz, 3H).

**Example 10: SZ-022303**

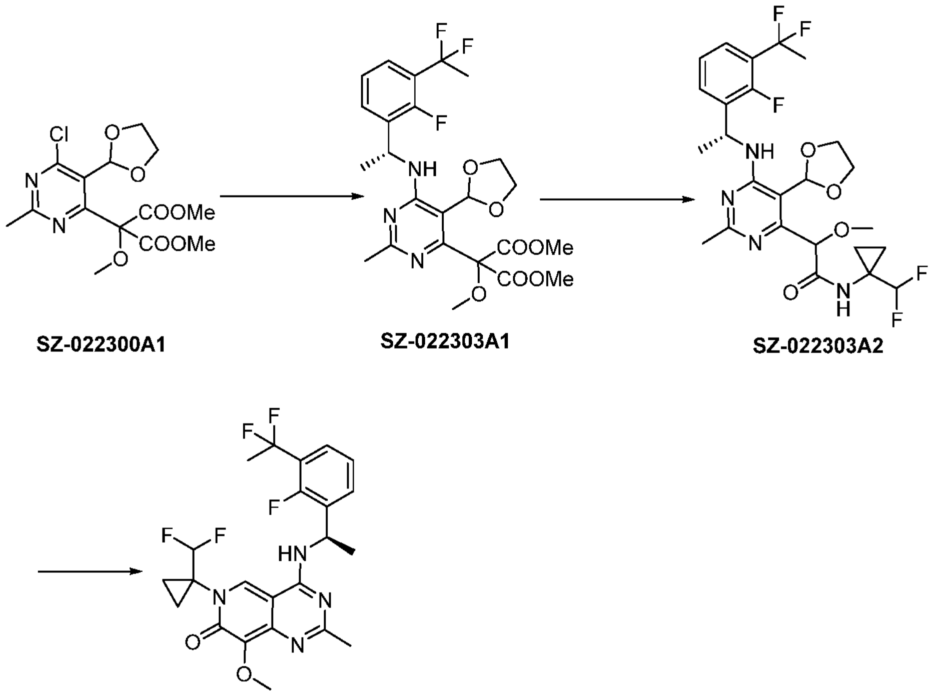

SZ-022300A1 SZ-022303A1 SZ-022303A2

SZ-022303

Step 1:

Compound **SZ-022300A1** (200 mg, 0.55 mmol), (1*R*)-1-(3-(1,1-difluoroethyl)-2-fluorophenyl)ethylamine hydrochloride (159 mg, 0.67 mmol), and triethylamine (112 mg, 1.11 mmol) were allowed to react at 60 °C for 3 h. After the starting materials were consumed as monitored by LC-MS, the reaction mixture was cooled to room temperature. Water (50 mL) and ethyl acetate (50 mL) were added to the reaction mixture for extraction, and the organic layer was separated, dried, and concentrated by rotary evaporation to give a crude product. The crude product was purified by flash silica gel column chromatography (PE:EA = 3: 1; Rf = 0.3) to give product **SZ-022303A1** as a colorless oil (130 mg). LC-MS: $[M+H]^+$ 528.06.

Step 2:

**SZ-022303A1** (130 mg, 0.25 mmol) was added to dimethyl sulfoxide (2 mL), and then a 20% sodium hydroxide solution (100 μL) was added. The mixture was allowed to react at 35 °C for 2 h. 1-(Difluoromethyl)cyclopropan-1-amine hydrochloride (46 mg, 0.32 mmol), *N,N,N,N*-tetramethyl-*O*-(7-azabenzotriazol-1-yl)uronium hexafluorophosphate (187 mg, 0.49 mmol), and triethylamine (50 mg, 0.49 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022303A2** as a light yellow viscous substance (100 mg). LC-MS: $[M+H]^+$ 545.02.

Step 3:

**SZ-022303A2** (100 mg, 0.25 mmol) was added to isopropanol (2 mL), and then a hydrochloric acid solution (2 M, 0.5 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022303** (60 mg). LC-MS: $[M+H]^+$ 483.17.

**SZ-022303:** $^1H$ NMR (400 MHz, DMSO-$d_6$) δ 8.93 (s, 1H), 8.80 (d, *J* = 7.2 Hz, 1H), 7.59 (t, *J* = 6.8 Hz, 1H), 7.45 (t, *J* = 6.8 Hz, 1H), 7.28 (t, *J* = 7.6 Hz, 1H), 6.34 (t, *J* = 56.8 Hz, 1H), 5.78 - 5.71 (m, 1H), 3.77 (s, 3H), 2.21 (s, 3H), 2.02 (t, *J* = 19.2 Hz, 3H), 1.58 (d, *J* = 7.2 Hz, 3H), 1.51 - 1.48 (m, 2H), 1.44 - 1.35 (m, 2H).

**Example 11: SZ-022328**

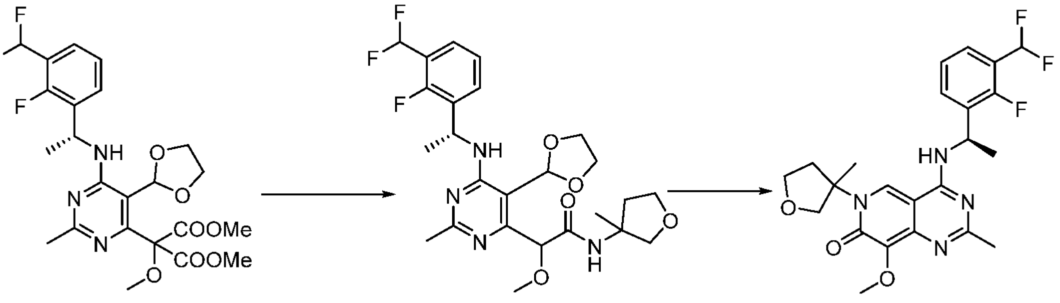

SZ-022244A1 SZ-022328A1 SZ-022328

Step 1:

**SZ-022244A1** (120 mg, 0.23 mmol) was added to dimethyl sulfoxide (2 mL), and then a 20% sodium hydroxide solution (120 μL) was added. The mixture was allowed to react at 40 °C for 2 h. 3-Methyloxolan-3-amine (36 mg, 0.35 mmol), N,N,N,N-tetramethyl-O-(7-azabenzotriazol-1-yl)uronium hexafluorophosphate (267 mg, 0.70 mmol), and triethylamine (71 mg, 0.70 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022328A1** as a yellow viscous substance (120 mg). LC-MS: $[M+H]^+$ 525.21.

Step 2:

**SZ-022328A1** (120 mg, 0.23 mmol) was added to isopropanol (2 mL), and then a hydrochloric acid solution (2 M, 0.58 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022328** (16.4 mg). LC-MS: $[M+H]^+$ 463.16.

**SZ-022328:** $^1$H NMR (400 MHz, DMSO-$d_6$) δ 8.79 - 8.72 (m, 1H), 8.64 (d, *J* = 4.8 Hz, 1H), 7.66 (t, *J* = 7.2 Hz, 1H), 7.52 (t, *J* = 7.2 Hz, 1H), 7.31 (td, *J* = 7.7, 2.8 Hz, 1H), 7.23 (t, *J* = 54.4 Hz, 1H), 5.82 - 5.73 (m, 1H), 4.42 (d, *J* = 9.2 Hz, 1H), 3.98 - 3.85 (m, 3H), 3.74 (s, 3H), 2.57 - 2.52 (m, 2H), 2.21 (s, 3H), 1.61 (s, 3H), 1.59 (d, *J* = 6.4 Hz, 3H).

Example 12: SZ-022334

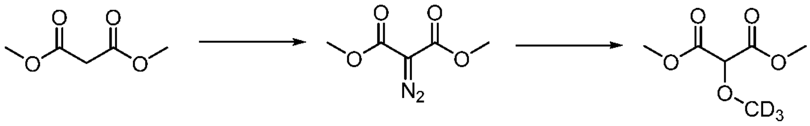

SZ-022334A1 SZ-022334A2 SZ-022334A3

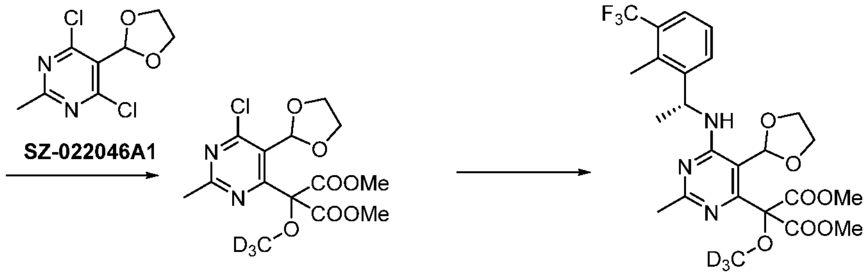

SZ-022046A1

SZ-022334A4 SZ-022334A5

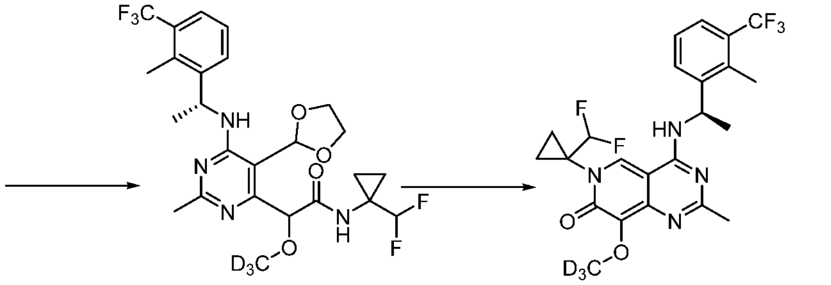

SZ-022334A6 SZ-022334

Step 1:

**SZ-022334A1** (1 g, 7.57 mmol) was added to acetonitrile (5 mL), and then triethylamine (920 mg, 9.09 mmol) was added. The mixture was cooled to 0 °C, and 4-acetamidobenzenesulfonyl azide (2.18 g, 9.09 mmol) was slowly added. The resulting mixture was allowed to react at room temperature overnight. After the starting materials were consumed as monitored by LC-MS, the mixture was filtered under vacuum. The filter cake was washed with ethyl acetate (20 mL), and water (10 mL) was added for extraction. The organic layer was separated, washed with a saturated sodium chloride solution, dried, and concentrated by rotary evaporation to give a crude product. The crude product was purified by flash silica gel column chromatography (PE:EA = 5:1; Rf = 0.3) to give product **SZ-022334A2** as a light yellow oil (1 g).

Step 2:

**SZ-022334A2** (1 g, 6.33 mmol) was added to dichloromethane (10 mL), and then deuterated methanol (250 mg, 6.96 mmol) and rhodium(II) acetate dimer (28 mg, 63 μmol) were added. The mixture was allowed to react for 3 h with the temperature maintained at 70 °C. After the starting materials were consumed as monitored by TLC, the reaction mixture was concentrated by rotary evaporation to give a crude product. The crude product was purified by flash silica gel column chromatography (PE:EA = 10: 1; Rf = 0.2) to give product **SZ-022334A3** as a colorless oil (840 mg).

Step 3:

**SZ-022046A1** (600 mg, 2.56 mmol, synthesized according to WO2019122129), **SZ-022334A3** (840 mg, 5.1 mmol), and potassium carbonate (1.1 g, 7.7 mmol) were added to dimethyl sulfoxide (10 mL), and the mixture was heated to 100 °C for reaction for 5 h. After the starting materials were consumed as monitored by LC-MS, water (100 mL) and ethyl acetate (100 mL) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA = 3:1; Rf = 0.4) to give product **SZ-**

**022334A4** as a yellow oil (220 mg). LC-MS: $[M+H]^+$ 364.22, 366.20.

Step 4:

**SZ-022334A4** (220 mg, 0.61 mmol), (1*R*)-1-(3-(trifluoromethyl)-2-methylphenyl)ethylamine hydrochloride (174 mg, 0.73 mmol, synthesized according to WO2019122129), triethylamine (184 mg, 1.82 mmol), and potassium carbonate (84 mg, 0.61 mmol) were added to dimethyl sulfoxide (5 mL), and the mixture was heated to 60 °C for reaction for 8 h. After the starting materials were consumed as monitored by LC-MS, water (100 mL) and ethyl acetate (100 mL) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA = 3:1; Rf = 0.35) to give product **SZ-022334A5** as a yellow oil (200 mg). LC-MS: $[M+H]^+$ 530.99.

Step 5:

**SZ-022334A5** (200 mg, 0.38 mmol) was added to dimethyl sulfoxide (2 mL), and then a 10% sodium hydroxide solution (750 μL) was added. The mixture was allowed to react at 40 °C for 1 h. 1-Difluoromethylcyclopropanamine hydrochloride (71 mg, 0.57 mmol), *N,N,N',N'*-tetramethyl-*O*-(7-azabenzotriazol-1-yl)uronium hexafluorophosphate (430 mg, 1.13 mmol), and triethylamine (191 mg, 1.89 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022334A6** as a yellow viscous substance (200 mg). LC-MS: $[M+H]^+$ 548.31.

Step 6:

**SZ-022334A6** (200 mg, 0.37 mmol) was added to isopropanol (5 mL), and then a hydrochloric acid solution (2 M, 0.9 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022334** (85 mg). LC-MS: $[M+H]^+$ 485.88.

**SZ-022334:** $^1H$ NMR (400 MHz, DMSO-$d_6$) δ 8.91 (s, 1H), 8.88 (d, *J* = 6.8 Hz, 1H), 7.70 (d, *J* = 7.6 Hz, 1H), 7.56 (d, *J* = 7.6 Hz, 1H), 7.39 (t, *J* = 7.6 Hz, 1H), 6.34 (t, *J* = 56.8 Hz, 1H), 5.72 - 5.65 (m, 1H), 2.57 (s, 3H), 2.21 (s, 3H), 1.54 (d, *J* = 7.2 Hz, 3H), 1.51 -1.45 (m, 2H), 1.45 - 1.30 (m, 2H).

**Example 13: SZ-022335**

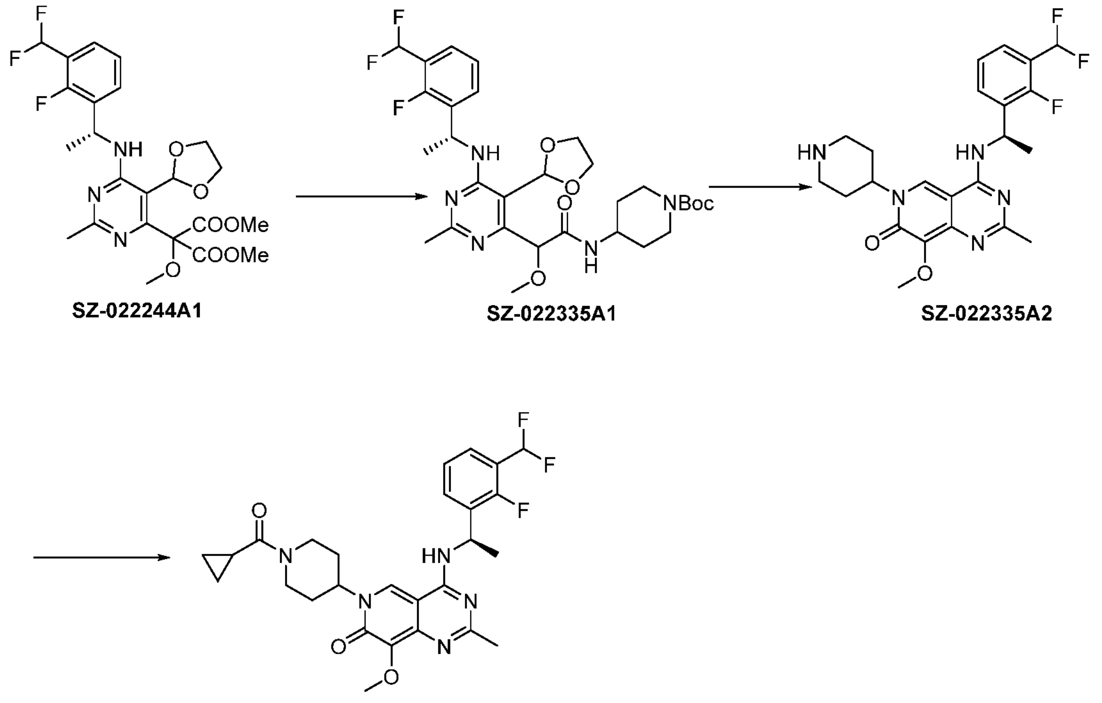

SZ-022244A1 SZ-022335A1 SZ-022335A2

SZ-022335

Step 1:

**SZ-022244A1** (145 mg, 0.28 mmol) was added to dimethyl sulfoxide (2 mL), and then a 10% sodium hydroxide solution (230 μL) was added. The mixture was allowed to react at 40 °C for 1 h. 4-Amino-1-BOC-piperidine (85 mg, 0.42 mmol), *N*,*N*,*N*',*N*'-tetramethyl-*O*-(7-azabenzotriazol-1-yl)uronium hexafluorophosphate (322 mg, 0.85 mmol), and triethylamine (86 mg, 0.85 mmol) were then added into the system, and the mixture was allowed to react at room temperature for another 8 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022335A1** as a yellow viscous substance (140 mg). LC-MS: $[M+H]^+$ 624.41.

Step 2:

**SZ-022335A1** (140 mg, 0.23 mmol) was added to isopropanol (2 mL), and then a hydrochloric acid solution (2 M, 0.50 mL) was added. The mixture was allowed to react at 50 °C for 8 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative medium-pressure reversed-phase chromatography and then lyophilized to give **SZ-022335A2** (150 mg). LC-MS: $[M+H]^+$ 461.88.

Step 3:

**SZ-022335A2** (140 mg, 0.30 mmol), cyclopropanecarboxylic acid (26 mg, 0.30 mmol), N,N,N,N-tetramethyl-O-(7-azabenzotriazol-1-yl)uronium hexafluorophosphate (173 mg, 0.45 mmol), and triethylamine (92 mg, 0.90 mmol) were added to *N*,*N*-dimethylformamide (5 mL). The mixture was allowed to react at room temperature for 3 h. After the starting materials were consumed as monitored by LC-MS, water (20 mL) and ethyl acetate (20 mL) were added for extraction, and the organic layer was separated and washed with a saturated sodium chloride solution. The solvent was removed by rotary evaporation, and the residue was subjected to preparative high performance liquid chromatography and then lyophilized to give **SZ-022335** (42 mg). LC-MS: $[M+H]^+$ 530.00.

**SZ-022335:** $^1H$ NMR (400 MHz, DMSO-$d_6$) δ 8.80 (s, 1H), 8.70 (d, *J* = 7.2 Hz, 1H), 7.64 (t, *J* = 7.2 Hz, 1H), 7.52 (t, *J* = 7.2 Hz, 1H), 7.32 (t, *J* = 7.6 Hz, 1H), 7.23 (t, *J* = 54.4 Hz, 1H), 5.78 - 5.71 (m, 1H), 5.28 - 5.18 (m, 1H), 4.74 - 4.45 (m, 2H), 3.77 (s, 3H), 2.82 - 2.68 (m, 1H), 2.22 (s, 3H), 2.11 - 2.05 (m, 1H), 2.03 - 1.82 (m, 4H), 1.59 (d, *J* = 7.2

Hz, 3H), 0.86 - 0.66 (m, 4H).

**Example 14: SZ-022336**

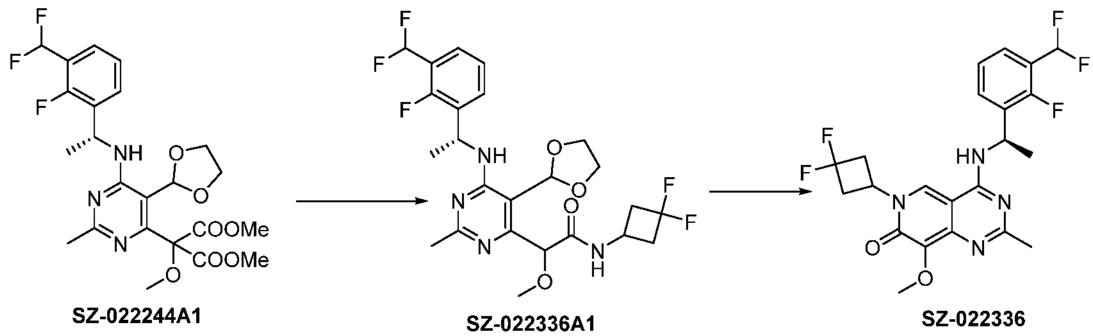

SZ-022244A1 SZ-022336A1 SZ-022336

Step 1:

**SZ-022244A1** (140 mg, 0.28 mmol) was added to dimethyl sulfoxide (2 mL), and then a 10% sodium hydroxide solution (500 μL) was added. The mixture was allowed to react at 40 °C for 1 h. 3,3-Difluorocyclobutanamine hydrochloride (60 mg, 0.42 mmol), N,N,N,N-tetramethyl-O-(7-azabenzotriazol-1-yl)uronium hexafluorophosphate (320 mg, 0.84 mmol), and triethylamine (85 mg, 0.84 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022336A1** as a yellow viscous substance (140 mg). LC-MS: $[M+H]^+$ 531.23.

Step 2:

**SZ-022336A1** (140 mg, 0.26 mmol) was added to isopropanol (2 mL), and then a hydrochloric acid solution (2 M, 0.6 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022336** (44 mg). LC-MS: $[M+H]^+$ 468.9.

**SZ-022336:** $^1H$ NMR (400 MHz, DMSO-$d_6$) δ 8.76 (s, 1H), 8.71 (d, *J* = 7.2 Hz, 1H), 7.67 (t, *J* = 7.2 Hz, 1H), 7.53 (t, *J* = 7.2 Hz, 1H), 7.32 (t, *J* = 7.6 Hz, 1H), 7.24 (t, *J* = 54.4 Hz, 1H), 5.75 - 5.82 (m, 1H), 4.97 - 4.86 (m, 1H), 3.77 (s, 3H), 3.29 - 3.10 (m, 4H), 2.23 (s, 3H), 1.60 (d, *J* = 7.2 Hz, 3H).

**Example 15:** SZ-022307

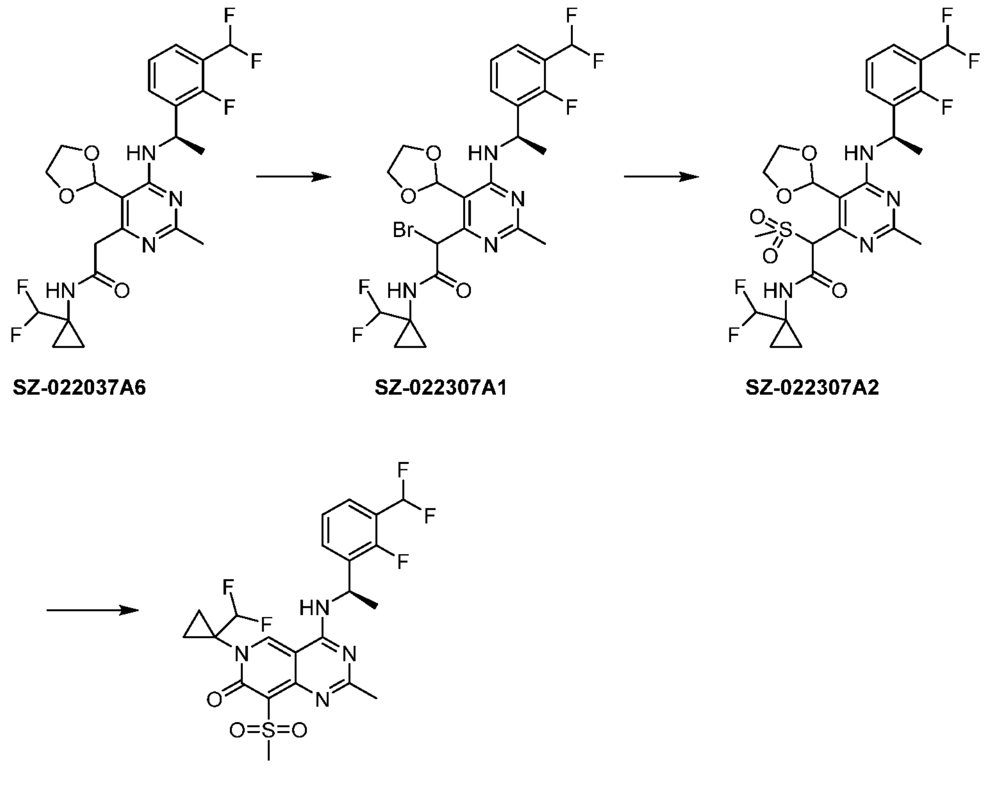

SZ-022037A6 SZ-022307A1 SZ-022307A2

SZ-022307

Step 1:

**SZ-022037A6** (650 mg, 1.30 mmol, synthesized according to WO2019122129), N-bromosuccinimide (231 mg, 1.30 mmol), and azobisisobutyronitrile (21.3 mg, 0.13 mmol) were suspended in carbon tetrachloride (15 mL) under argon atmosphere, and the mixture was stirred at 60 °C for 1 h. The reaction mixture was then concentrated to give a crude product. The crude product was purified by column chromatography (petroleum ether: ethyl acetate = 1:1, Rf = 0.5) to give **SZ-022307A1** as a white solid (500 mg). LCMS: $[M+H]^+$ 578.84, 580.76.

Step 2:

**SZ-022307A1** (100 mg, 0.17 mmol) and sodium methanesulfinate (21 mg, 0.20 mmol) were mixed in dimethyl sulfoxide (10 mL) under argon atmosphere, and then the mixture was stirred at room temperature for 2 h. After the reaction was completed, 100 mL of water was added to the reaction mixture. The resulting mixture was extracted with 50 mL of ethyl acetate, and the organic phase was dried and concentrated to give **SZ-022307A2** as a yellow liquid (100 mg). LCMS: $[M+H]^+$ 578.92.

Step 3:

**SZ-022307A2** (100 mg, 0.17 mmol) and hydrochloric acid (0.2 mL, 5 M) were mixed in isopropanol (2 mL) under argon atmosphere, and then the mixture was stirred at 40 °C for 2 h. After the reaction was completed, the reaction mixture was diluted with 10 mL of water. The dilution was extracted with 20 mL of ethyl acetate, and the organic phase was dried and concentrated to give a crude product. The crude product was purified by preparative reversed-phase chromatography and lyophilized to give **SZ-022307** as a yellow solid (13.91 mg). LCMS: $[M+H]^+$ 516.86.

**SZ-022307:** $^1$H NMR (400 MHz, DMSO-$d_6$) δ 9.46 (s, 1H), 9.18 (d, *J* = 4.0 Hz, 1H), 7.66 (t, *J* = 8.0 Hz, 1H), 7.54 (t, *J* = 8.0 Hz, 1H), 7.34 (t, *J* = 8.0 Hz, 1H), 7.23 (t, *J* = 52.0 Hz, 1H), 6.36 (t, *J* = 56.0 Hz, 1H), 5.81 - 5.76 (m, 1H), 3.33 (s, 3H), 2.28 (s, 3H), 1.60 (d, *J* = 4.0 Hz, 3H), 1.52 - 1.43 (m, 4H).

**Example 16: SZ-022312**

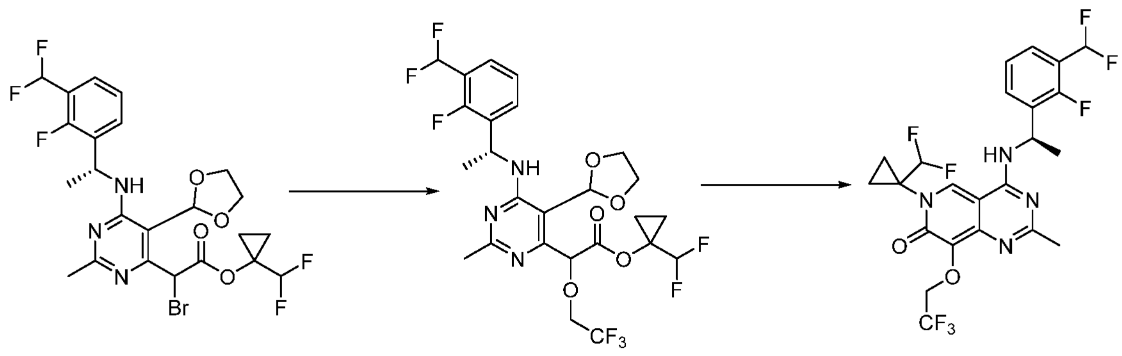

SZ-022307A1 SZ-022312A1 SZ-022312

Step 1:

Sodium hydride (14 mg, 0.3 mmol) was added to tetrahydrofuran (5 mL), and then trifluoroethanol (20 mg, 0.2 mmol) was added. The mixture was stirred at room temperature for 0.5 h. A solution of **SZ-022307A1** (57 mg, 0.1 mmol) in tetrahydrofuran (1 mL) was added dropwise to the reaction mixture, and the resulting mixture was stirred for another 1.5 h. After the starting materials were consumed as monitored by LC-MS, water (10 mL) and ethyl acetate (10 mL) were added to the reaction mixture for extraction, and the organic layer was separated, washed with a saturated sodium chloride solution, dried, and concentrated by rotary evaporation to give crude product **SZ-022312A1** as a yellow viscous substance (55 mg). LC-MS: $[M+H]^+$ 598.98.

Step 2:

**SZ-022312A1** (55 mg, 0.1 mmol) was added to isopropanol (1 mL), and then a hydrochloric acid solution (2 M, 0.3 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL) and ethyl acetate (10 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022312** (4 mg). LC-MS: $[M+H]^+$ 536.91.

**SZ-022312**: $^1$H NMR (400 MHz, DMSO - $d_6$) δ 8.94 (s, 1H), 8.88 (d, *J* = 6.8 Hz, 1H), 7.61 (t, *J* = 6.8 Hz, 1H), 7.49 (t, *J* = 6.8 Hz, 1H), 7.29 (t, *J* = 7.6 Hz, 1H), 7.19 (t, *J* = 54.4 Hz, 1H), 6.30 (t, *J*= 56.8 Hz, 1H), 5.74 - 5.70 (m, 1H), 4.62 (q, *J* = 9.2 Hz, 2H), 2.19 (s, 3H), 1.55 (d, *J* = 6.8 Hz, 4H), 1.47 (s, 3H).

**Example 17: SZ-022314**

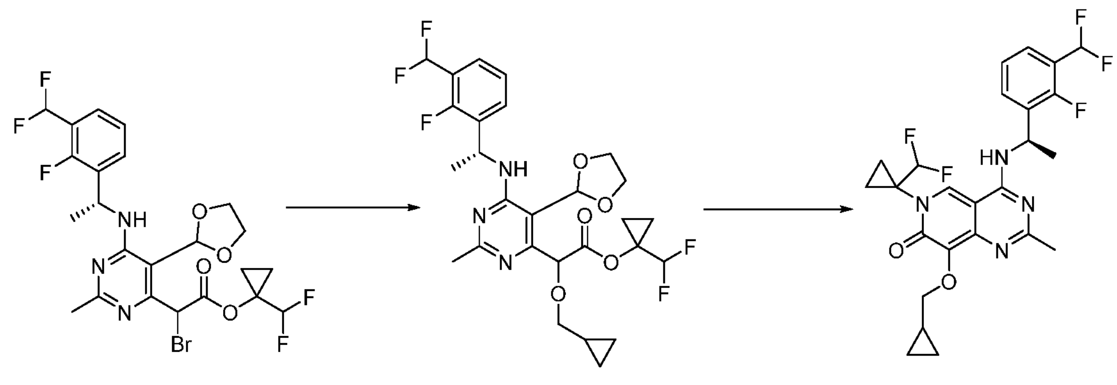

SZ-022307A1 SZ-022314A1 SZ-022314

Step 1:

Sodium hydride (14 mg, 0.3 mmol) was added to tetrahydrofuran (5 mL), and then cyclopropanemethanol (14.4 mg, 0.2 mmol) was added. The mixture was stirred at room temperature for 0.5 h. A solution of **SZ-022307A1** (57 mg, 0.1 mmol) in tetrahydrofuran (1 mL) was added dropwise to the reaction mixture, and the resulting mixture was stirred for another 1.5 h. After the starting materials were consumed as monitored by LC-MS, water (10 mL) and ethyl acetate (10 mL) were added to the reaction mixture for extraction, and the organic layer was separated, washed with a saturated sodium chloride solution, dried, and concentrated by rotary evaporation to give crude product **SZ-022314A1** as a yellow viscous substance (60 mg). LC-MS: $[M+H]^+$ 571.01.

Step 2:

**SZ-022314A1** (60 mg, 0.1 mmol) was added to isopropanol (1 mL), and then a hydrochloric acid solution (2 M, 0.3 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL) and ethyl acetate (10 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022314** (5 mg). LC-MS: $[M+H]^+$ 508.86.

**SZ-022314:** $^1H$ NMR (400 MHz, DMSO - $d_6$) $\delta$ 8.94 (s, 1H), 8.81 (d, *J* = 6.8 Hz, 1H), 7.66 (t, *J* = 7.6 Hz, 1H), 7.54 (t, *J* = 7.2 Hz, 1H), 7.34 (t, *J* = 7.6 Hz, 1H), 7.25 (t, *J* = 54.4 Hz, 1H), 6.35 (t, *J* = 57.2 Hz, 1H), 5.79 - 5.76 (m, 1H), 3.89 (d, *J* = 7.2 Hz, 2H), 2.24 (s, 3H), 1.59 (d, *J* = 7.2 Hz, 4H), 1.51 (s, 3H), 1.19 - 1.13 (m, 1H), 0.47 - 0.42 (m, *J* = 7.2 Hz, 2H), 0.27 - 0.23 (m, *J* = 7.2 Hz, 2H).

**Example 18: SZ-022306**

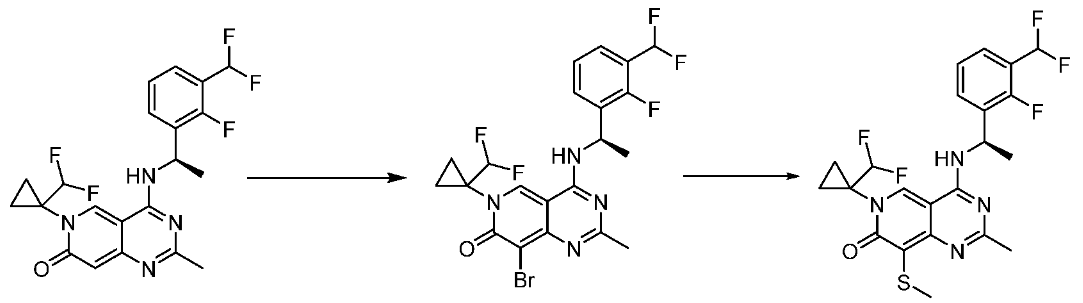

**SZ-022037** **SZ-022306A1** **SZ-022306**

Step 1:

**SZ-022037** (200 mg, 0.46 mmol, synthesized according to WO2019122129) and *N*-bromosuccinimide (96.1 mg, 0.55 mmol) were mixed in acetonitrile (5 mL) under argon atmosphere, and the mixture was stirred at room temperature for 1 h. The reaction mixture was then directly purified by medium-pressure reversed-phase chromatography (acetonitrile/water = 40%) to give **SZ-022306A1** as a white solid (160 mg). LCMS: $[M+H]^+$ 516.82, 518.79.

Step 2:

**SZ-022306A1** (50 mg, 0.10 mmol) and a sodium thiomethoxide solution (0.1 mL, 20% wt) were mixed in *N*,*N*-dimethylformamide (3 mL) under argon atmosphere, and then the mixture was stirred for half an hour in an ice-water bath. After the reaction was completed, 30 mL of water was added to the reaction mixture. The resulting mixture was extracted with 50 mL of ethyl acetate, and the organic phase was dried and concentrated to give a crude product. The crude product was purified by preparative high-pressure chromatography and lyophilized to give **SZ-022306** as a yellow solid (10 mg). LCMS: $[M+H]^+$ 485.12.

**SZ-022306:** $^1H$ NMR (400 MHz, DMSO-$d_6$) $\delta$ 9.11 (s, 1H), 8.88 (d, *J* =8.0 Hz, 1H), 7.64 (t, *J* = 8.0 Hz, 1H), 7.52 (t, *J* = 8.0 Hz, 1H), 7.32 (t, *J* = 8.0 Hz, 1H), 7.23 (t, *J* = 52.0 Hz, 1H), 6.37 (t, *J* = 56.0 Hz, 1H), 5.78 - 5.72 (m, 1H), 2.39 (s, 3H), 2.24 (s, 3H), 1.58 (d, *J* = 4.0 Hz, 3H), 1.50 - 1.40 (m, 4H).

Example 19: Intermediate SZ-022037

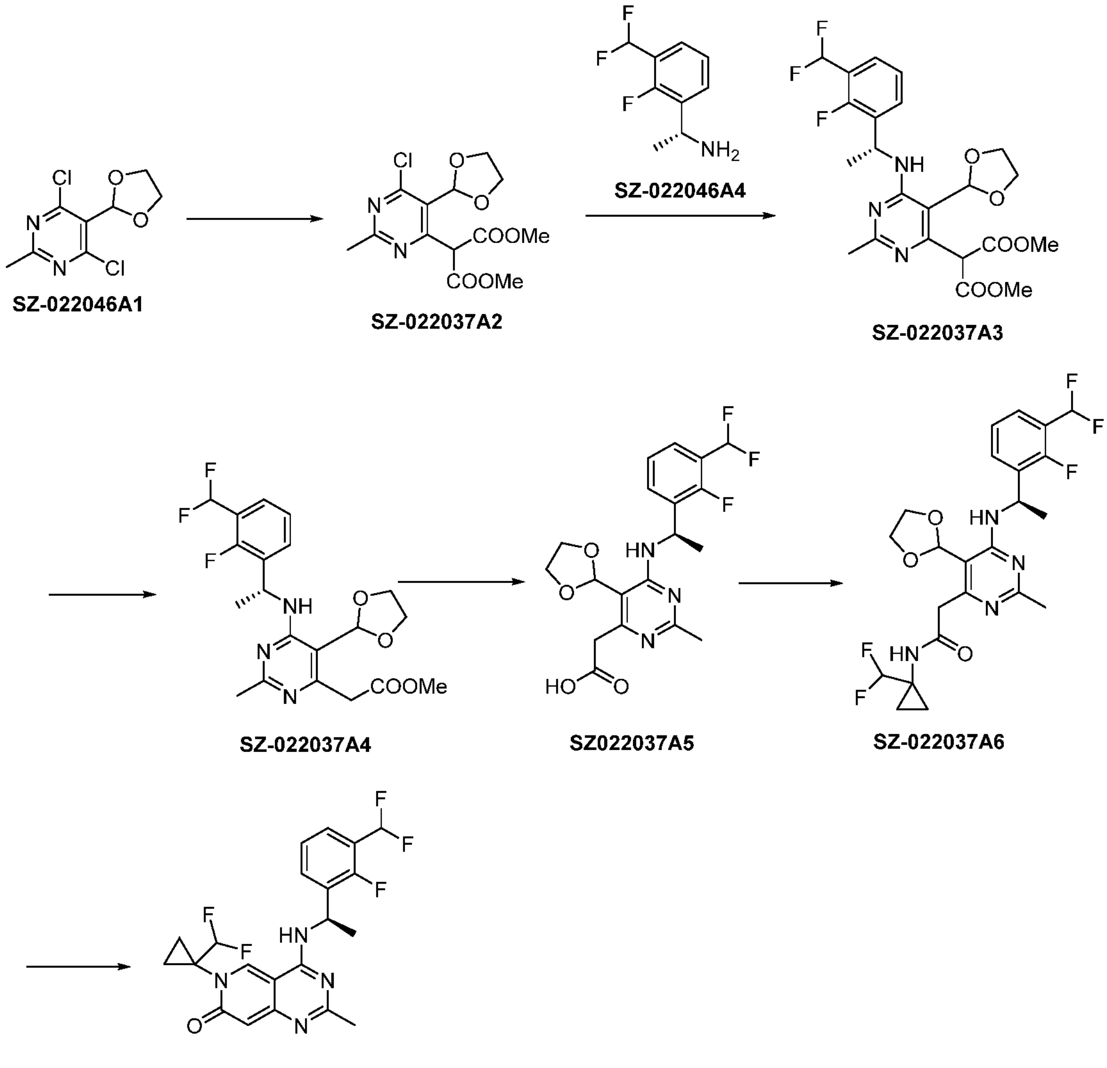

SZ-022046A1 SZ-022037A2 SZ-022046A4 SZ-022037A3

SZ-022037A4 SZ022037A5 SZ-022037A6

SZ-022037

Step 1:

SZ-022046A1 (38.0 g, 161.7 mmol), dimethyl malonate (21.8 g, 164.9 mmol), and cesium carbonate (105.3 g, 323.3 mmol) were added to dimethyl sulfoxide (300 mL), and the mixture was heated to 100 °C for reaction for 3 h. Water (1 L) and ethyl acetate (1 L) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA = 3:1; Rf = 0.2) to give product **SZ-022037A2** as an off-white solid(31.9 g). LC-MS: $[M+H]^+$ 330.69, 332.51.

Step 2:

**SZ-022037A2** (15.0 g, 45.4 mmol), **SZ-022046A4** (10.4 g, 46.3 mmol), and *N,N*-diisopropylethylamine (17.6 g, 136.0 mmol) were added to dimethyl sulfoxide (150 mL), and the mixture was heated to 80 °C for reaction for 15 h. After the starting materials were consumed as monitored by LC-MS, water (1 L) and ethyl acetate (1 L) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA = 1:1; Rf = 0.2) to give product **SZ-022037A3** as a brown-yellow oil (17.3 g).

LC-MS: $[M+H]^+$ 483.93, 484.88.

Step 3:

**SZ-022037A3** (5.4 g, 11.2 mmol) and lithium chloride (1.9 g, 44.7 mmol) were added to dimethyl sulfoxide (190 mL), and the mixture was heated to 120 °C for reaction for 15 h. After the starting materials were consumed as monitored by LC-MS, water (1.5 L) and ethyl acetate (1.5 L) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA:DCM = 1:1:0.1; Rf = 0.2) to give product **SZ-022037A4** as a brown-yellow oil (5.3 g). LC-MS: $[M+H]^+$ 426.98.

Step 4:

**SZ-022037A4** (2.9 g, 6.8 mmol) was dissolved in a mixed solvent of acetonitrile (30 mL) and dimethyl sulfoxide (45 mL), and then a 10% sodium hydroxide solution (11 mL, 27.3 mmol) was added. The mixture was allowed to react at 25 °C for 1.5 h. After the starting materials were consumed as monitored by LC-MS, the reaction mixture was used directly in the next step without work-up. LC-MS: $[M+H]^+$ 411.92.

Step 5:

1-(Difluoromethyl)cyclopropanamine hydrochloride (1.3 g, 9.4 mmol), *N,N,N',N'*-tetramethyl-*O*-(7-azabenzotriazol-1-yl)uronium hexafluorophosphate (7.6 g, 20.4 mmol), and triethylamine (2.1 g, 20.4 mmol) were added to the reaction system in the previous step, and the mixture was allowed to react at 25 °C for 1 h. After the starting materials were consumed as monitored by LC-MS, water (500 mL) and ethyl acetate (500 mL) were added to the reaction mixture for extraction, and the organic layer was separated, washed with a saturated sodium chloride solution, dried, and concentrated to dryness under reduced pressure to give crude product **SZ-022037A6** as a light yellow solid (4.0 g). LC-MS: $[M+H]^+$ 501.3.

Step 6:

Crude product **SZ-022037A6** (4.0 g, 6.8 mmol) was added to isopropanol (50 mL), and then a hydrochloric acid solution (2 M, 17 mL) was added. The mixture was allowed to react at 50 °C for 3 h. After the starting materials were consumed as monitored by LC-MS, the reaction mixture was directly concentrated to dryness under reduced pressure. A saturated sodium bicarbonate solution (50 mL) and ethyl acetate (50 mL) were then added for extraction, and the organic layer was separated, washed with a saturated sodium chloride solution, dried, and concentrated under reduced pressure to give a crude product. The crude product was separated by preparative high performance liquid chromatography and then lyophilized to give **SZ-022037** as a yellow solid (1.8 g). LC-MS: $[M+H]^+$ 438.83.

**SZ-022037:** $^1H$ NMR (400 MHz, DMSO-$d_6$) δ 9.16 (s, 1H), 8.89 (d, *J* = 6.8 Hz, 1H), 7.65 (t, *J* = 7.6 Hz, 1H), 7.53 (t, *J* = 7.2 Hz, 1H), 7.33 (t, *J* = 7.6 Hz, 1H), 7.24 (t, *J* = 54.4 Hz, 1H), 6.34 (t, *J* = 57.2 Hz, 1H), 6.09 (s, 1H), 5.78 - 5.71 (m, 1H), 2.18 (s, 3H), 1.58 (d, *J* = 7.2 Hz, 3H), 1.50 - 1.46 (m, 2H), 1.45 - 1.36 (m, 2H).

**Example 20: SZ-022251**

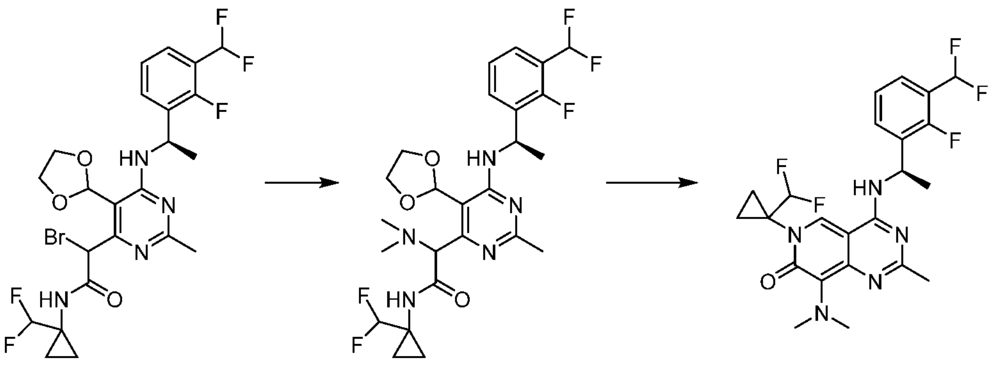

SZ-022307A1 SZ-022251A1 SZ-022251

Step 1:

**SZ-022307A1** (100 mg, 0.17 mmol), a solution of dimethylamine in tetrahydrofuran (0.10 mL, 2 M), and sodium carbonate (18.4 mg, 0.17 mmol) were mixed in tetrahydrofuran (5 mL) under argon atmosphere, and then the mixture was stirred at room temperature for 1 h. The reaction mixture was filtered and concentrated to give **SZ-022251A1** as a yellow liquid (60 mg). LCMS: $[M+H]^+$ 543.98.

Step 2:

**SZ-022251A1** (60 mg, 0.11 mmol) and hydrochloric acid (0.08 mL, 2 M) were mixed in isopropanol (3 mL) under argon atmosphere, and then the mixture was stirred at 40 °C for 1.5 h. After the reaction was completed, the reaction mixture was diluted with 10 mL of water. The dilution was extracted with 20 mL of ethyl acetate, and the organic phase was dried and concentrated to give a crude product. The crude product was purified by preparative reversed-phase chromatography and lyophilized to give **SZ-022251** as a yellow solid (2.07 mg). LCMS: $[M+H]^+$ 482.18.

**SZ-022251:** $^1H$ NMR (400 MHz, $CD_3OD$) δ 8.95 (s, 1H), 7.61 (t, *J* = 8.0 Hz, 1H), 7.53 (t, *J* = 8.0 Hz, 1H), 7.29 (t, *J* = 8.0 Hz, 1H), 7.04 (t, *J* = 56.0 Hz, 1H), 6.32 (t, *J* = 56.0 Hz, 1H), 5.87 - 5.82 (m, 1H), 2.89 (s, 6H), 2.37 (s, 3H), 1.69 (d, *J* = 4.0 Hz, 3H), 1.64 - 1.33 (m, 4H).

**Example 21: SZ-022327**

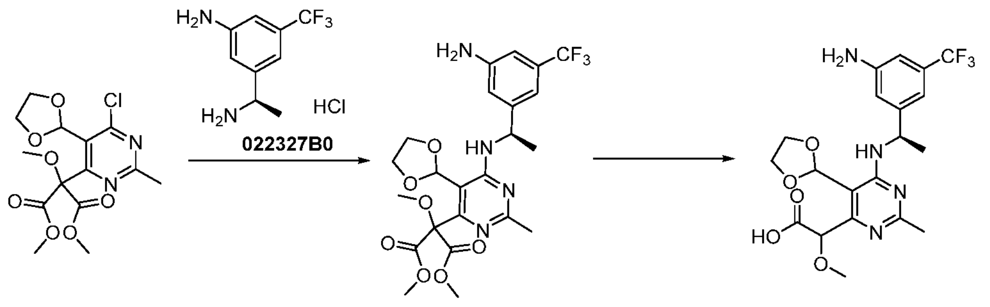


SZ-022300A1 022327B1 022327B2

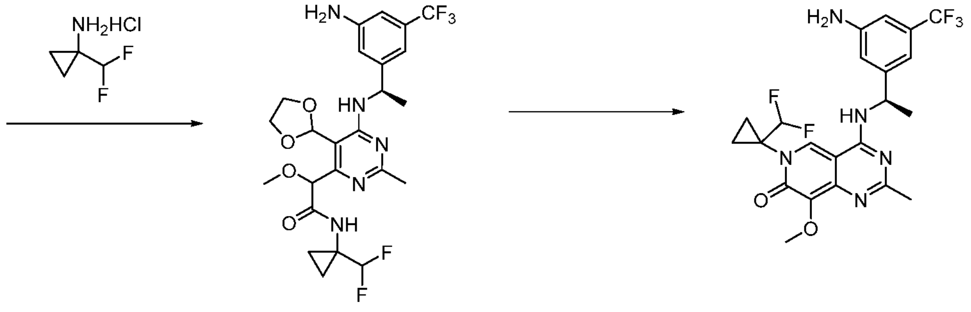

022327B3 SZ-022327

Step 1:

**SZ-022300A1** (270 mg, 0.75 mmol), **022327B0** (200 mg, 0.83 mmol), and triethylamine (400 mg, 3.75 mmol) were added to tetrahydrofuran (10 mL), and the mixture was heated to 80 °C for reaction for 16 h. After the starting materials were consumed as monitored by LC-MS, water (20 mL) and ethyl acetate (50 mL) were added to the reaction mixture for extraction, and the organic layer was separated, washed with a saturated sodium chloride solution, dried, and concentrated to dryness under reduced pressure to give a crude product. The crude product was purified by flash silica gel column chromatography (PE:EA = 1:1) to give **022327B1** as a light yellow oil (270 mg). LC-MS: $[M+H]^+$ 529.25.

Step 2:

**022327B1** (270 mg, 0.51 mmol) was added to dimethyl sulfoxide (4 mL), and then a 20% sodium hydroxide solution (500 μL) was added. The mixture was allowed to react at room temperature for 1 h. After the starting materials were consumed as monitored by LC-MS, the reaction mixture was used directly in the next step without work-up. LC-MS: $[M+H]^+$ 457.19.

Step 3:

(Difluoromethyl)cyclopropyl-1-amine hydrochloride (97 mg, 0.76 mmol), HATU (580 mg, 1.53 mmol), and triethylamine (260 mg, 2.55 mmol) were added to the reaction system in the previous step, and the mixture was allowed to react at room temperature for another 16 h. After the starting materials were consumed as monitored by LC-MS, water (20 mL) and ethyl acetate (50 mL) were added to the reaction mixture for extraction, and the organic layer was separated, washed with a saturated sodium chloride solution, dried, and concentrated to dryness under reduced pressure to give crude product **022327B3** as a light yellow oil (100 mg). LC-MS: $[M+H]^+$ 546.26.

Step 4:

**022327B3** (50 mg, 0.09 mmol) was added to isopropanol (3 mL), and then a hydrochloric acid solution (2 M, 0.52 mL) was added. The mixture was allowed to react at 50 °C for 4 h. After the starting materials were consumed as monitored by LC-MS, the reaction mixture was directly concentrated to dryness under reduced pressure. A saturated sodium bicarbonate solution (5 mL), water (10 mL), and ethyl acetate (20 mL) were then added for extraction, and the organic layer was separated, washed with a saturated sodium chloride solution, dried, and concentrated to dryness

under reduced pressure to give a crude product. The crude product was separated by preparative high performance liquid chromatography and then lyophilized to give **SZ-022327** as a yellow solid (13.0 mg). LC-MS: $[M+H]^+$ 483.98.

**SZ-022327**: $^1H$ NMR (400 MHz, $CDCl_3$) δ 8.31 (s, 1H), 7.05 (s, 1H), 6.91 (s, 1H), 6.81 (s, 2H), 6.31 (t, *J* = 58.4 Hz, 1H), 5.75 - 5.70 (m, 1H), 4.06 (s, 3H), 3.92 (s, 2H), 2.47 (s, 3H), 1.64 (d, *J* = 6.8 Hz, 3H), 1.56 (br, 2H), 1.26 (br, 2H).

**Example 22:** SZ-022329

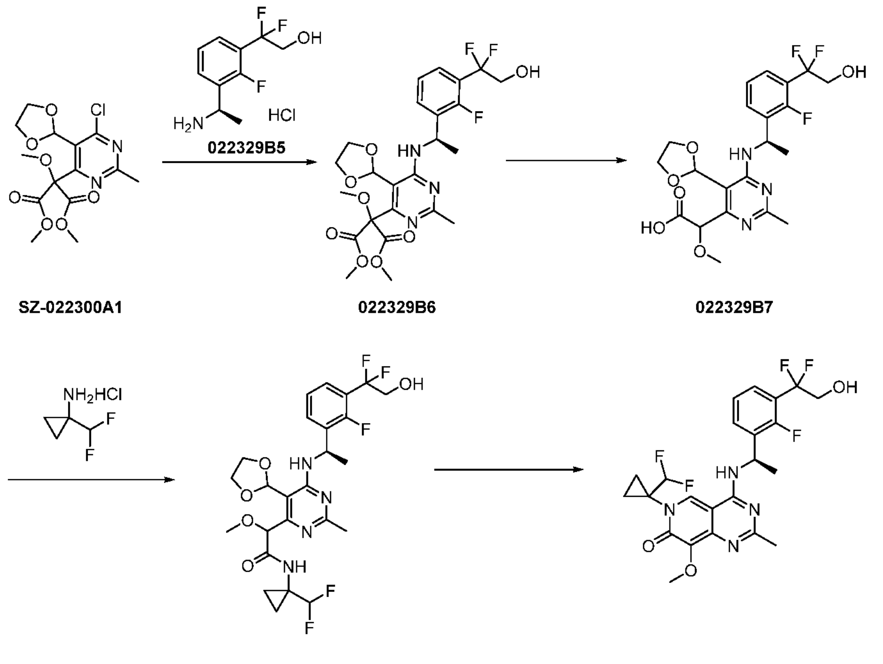

022329B5

SZ-022300A1 022329B6 022329B7

022329B8 SZ-022329

Step 1:

**SZ-022300A1** (180 mg, 0.50 mmol), **022329B5** (153 mg, 0.60 mmol, prepared by reference to the patent method), and triethylamine (253 mg, 2.50 mmol) were added to dimethyl sulfoxide (5 mL), and the mixture was heated to 70 °C for reaction for 16 h. After the starting materials were consumed as monitored by LC-MS, water (50 mL) and ethyl acetate (50 mL) were added to the reaction mixture for extraction, and the organic layer was separated, washed with a saturated sodium chloride solution, dried, and concentrated to dryness under reduced pressure to give a crude product. The crude product was purified by flash silica gel column chromatography (PE:EA= 1:2) to give compound **022329B6** as a yellow solid (70 mg). LC-MS: $[M+H]^+$ 544.23.

Step 2:

**022329B6** (70 mg, 0.13 mmol) was added to dimethyl sulfoxide (2 mL), and then a 20% sodium hydroxide solution (150 μL) was added. The mixture was allowed to react at room temperature for 3 h. After the starting materials were consumed as monitored by LC-MS, the reaction mixture was used directly in the next step without work-up. LC-MS: $[M+H]^+$ 472.17.

Step 3:

(Difluoromethyl)cyclopropyl-1-amine hydrochloride (28 mg, 0.20 mmol), HATU (148 mg, 0.39 mmol), and triethylamine (66 mg, 0.65 mmol) were added to the reaction system in the previous step, and the mixture was allowed to react at room temperature for 16 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and then concentrated to dryness under reduced pressure to give a crude product. The crude product was purified by flash silica gel column chromatography (DCM:MeOH = 20:1) to give **022329B8** as a light yellow oil (50 mg). LC-MS: $[M+H]^+$ 561.29.

Step 4:

**022329B8** (50 mg, 0.09 mmol) was added to isopropanol (2 mL), and then a hydrochloric acid solution (2 M, 0.44 mL) was added. The mixture was allowed to react at 50 °C for 4 h. After the starting materials were consumed as monitored by LC-MS, the reaction mixture was directly concentrated to dryness under reduced pressure. A saturated sodium bicarbonate solution (5 mL), water (10 mL), and ethyl acetate (20 mL) were then added for extraction, and the organic layer was separated, washed with a saturated sodium chloride solution, dried, and then concentrated to dryness under reduced pressure to give a crude product. The crude product was subjected to preparative high performance liquid chromatography and then lyophilized to give **SZ-022329** as a yellow solid (30 mg). LC-MS: $[M+H]^+$ 499.16.

**SZ-022329:** $^1$H NMR (400 MHz, DMSO-$d_6$) δ 8.92 (s, 1H), 8.78 (d, *J* = 7.2 Hz, 1H), 7.59 (t, *J* = 7.2 Hz, 1H), 7.43 (t, *J* = 7.2 Hz, 1H), 7.28 (t, *J* = 7.6 Hz, 1H), 6.34 (t, *J* = 57.2 Hz, 1H), 5.77 - 5.68 (m, 2H), 3.97 - 3.88 (m, 2H), 3.77 (s, 3H), 2.22 (s, 3H), 1.57 (d, *J* = 6.8 Hz, 3H), 1.49 - 1.39 (m, 4H).

**Example 23 and Example 24: SZ-022330 and SZ-022330B**

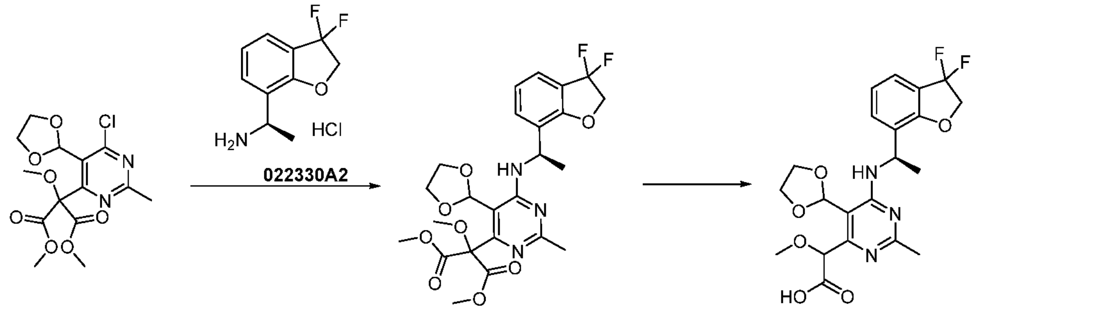


SZ-022300A1 022330A3 022330A4

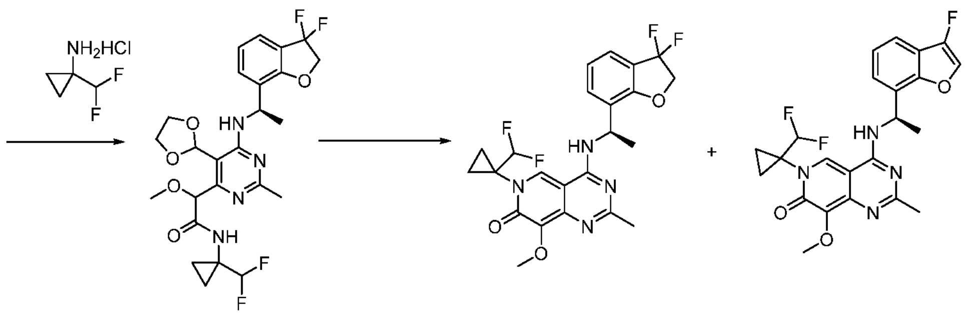

022330A5 SZ-022330 SZ-022330B

Step 1:

**SZ-022300A1** (90 mg, 0.25 mmol), **022330A2** (71 mg, 0.30 mmol, prepared by reference to the patent method), and triethylamine (126 mg, 1.25 mmol) were added to tetrahydrofuran (5 mL), and the mixture was heated to 80 °C for reaction for 16 h. After the starting materials were consumed as monitored by LC-MS, water (20 mL) and ethyl acetate (50 mL) were added to the reaction mixture for extraction, and the organic layer was separated, washed with a saturated sodium chloride solution, dried, and concentrated to dryness under reduced pressure to give a crude product. The crude product was purified by flash silica gel column chromatography (PE:EA = 1: 1) to give **022330A3** as a light yellow oil (70 mg). LC-MS: $[M+H]^+$ 524.26.

Step 2:

**022330A3** (70 mg, 0.13 mmol) was added to dimethyl sulfoxide (2 mL), and then a 20% sodium hydroxide solution (150 μL) was added. The mixture was allowed to react at room temperature for 3 h. After the starting materials were consumed as monitored by LC-MS, the reaction mixture was used directly in the next step without work-up. LC-MS: $[M+H]^+$ 432.28, 452.28.

Step 3:

(Difluoromethyl)cyclopropyl-1-amine hydrochloride (28 mg, 0.20 mmol), HATU (148 mg, 0.39 mmol), and triethylamine (66 mg, 0.65 mmol) were added to the reaction system in the previous step, and the mixture was allowed to react at room temperature for another 16 h. After the starting materials were consumed as monitored by LC-MS, water (20 mL) and ethyl acetate (30 mL) were added to the reaction mixture for extraction, and the organic layer was separated, washed with a saturated sodium chloride solution, dried, and concentrated to dryness under reduced pressure to give crude product **022330A5** as a light yellow oil (70 mg). LC-MS: $[M+H]^+$ 521.23, 541.22.

Step 4:

**022330A5** (70 mg, 0.13 mmol) was added to isopropanol (3 mL), and then a hydrochloric acid solution (2 M, 0.52 mL) was added. The mixture was allowed to react at 50 °C for 4 h. After the starting materials were consumed as monitored by LC-MS, the reaction mixture was directly concentrated to dryness under reduced pressure. A saturated sodium bicarbonate solution (5 mL), water (10 mL), and ethyl acetate (20 mL) were then added for extraction, and the organic layer was separated, washed with a saturated sodium chloride solution, dried, and concentrated to dryness under reduced pressure to give a crude product. The crude product was separated by preparative high performance liquid chromatography and then lyophilized to give **SZ-022330** as a yellow solid (14.8 mg) (LC-MS: $[M+H]^+$ 478.90) and **SZ-022330B** as a yellow solid (12.4 mg). LC-MS: $[M+H]^+$ 458.86.

**SZ-022330:** $^1$H NMR (400 MHz, $CDCl_3$) δ 7.97 (br, 1H), 7.48 - 7.44 (m, 2H), 7.05 (t, *J* = 7.6 Hz, 1H), 6.30 (t, *J* = 58.4 Hz, 1H), 5.75 - 5.70 (m, 1H), 4.77 - 4.58 (m, 2H), 4.06 (s, 3H), 2.47 (s, 3H), 1.66 (d, *J* = 6.8 Hz, 3H), 1.63 - 1.61 (m, 2H), 1.31 - 1.24 (m, 2H).

**SZ-022330B:** $^1$H NMR (400 MHz, $CDCl_3$) δ 8.05 (br, 1H), 7.62 (d, *J* = 4.4 Hz, 1H), 7.53 (d, *J* = 8.8 Hz, 1H), 7.38 (d, *J* = 7.2 Hz, 1H), 7.26 - 7.23 (m, 2H), 6.30 (t, *J* = 58.4 Hz, 1H), 6.01 - 5.96 (m, 1H), 4.04 (s, 3H), 2.46 (s, 3H), 1.78 (d, *J* = 7.2 Hz, 3H), 1.63 - 1.59 (m, 2H), 1.29 - 1.24 (m, 2H).

**Example 25: SZ-022224**

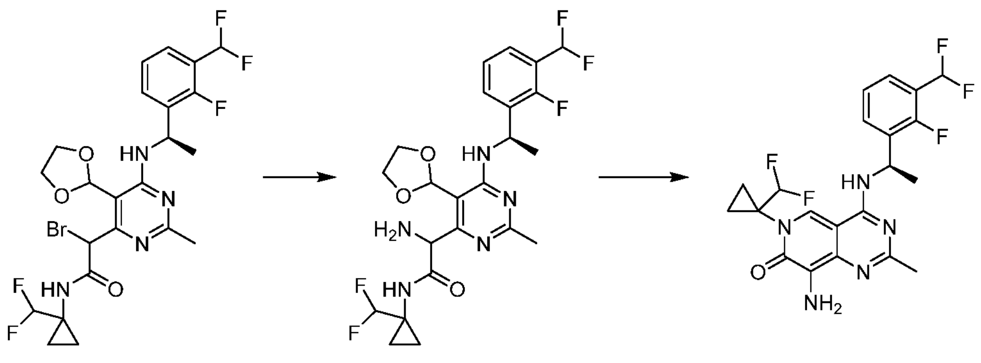

**SZ-022307A1** **SZ-022224A1** **SZ-022224**

Step 1:

**SZ-022307A1** (80 mg, 0.14 mmol) was dissolved in a solution of amine in methanol (5.00 mL, 7 M) under argon atmosphere, and then the mixture was microwaved at 130 °C for reaction for half an hour. The reaction mixture was concentrated to give **SZ-022224A1** as a yellow liquid (90 mg). LCMS: $[M+H]^+$ 515.95.

Step 2:

**SZ-022224A1** (90 mg, 0.17 mmol) and hydrochloric acid (0.2 mL, 5 M) were mixed in isopropanol (3 mL) under argon atmosphere, and then the mixture was stirred at 40 °C for half an hour. After the reaction was completed, the reaction mixture was diluted with 10 mL of water. The dilution was extracted with 20 mL of ethyl acetate, and the organic phase was dried and concentrated to give a crude product. The crude product was purified by preparative reversed-phase chromatography and lyophilized to give **SZ-022224** as a yellow solid (1.73 mg). LCMS: $[M+H]^+$ 453.91.

**SZ-022224:** $^1H$ NMR (400 MHz, $CD_3OD$) δ 7.73 (s, 1H), 7.62 (t, *J* = 8.0 Hz, 1H), 7.51 (t, *J* = 8.0 Hz, 1H), 7.27 (t, *J* = 8.0 Hz, 1H), 7.05 (t, *J* = 56.0 Hz, 1H), 6.06 (t, *J* = 56.0 Hz, 1H), 5.92 - 5.85 (m, 1H), 2.44 (s, 3H), 1.68 (d, *J* = 4.0 Hz, 3H), 1.38 - 1.08 (m, 4H).

**Example 26: SZ-022344**

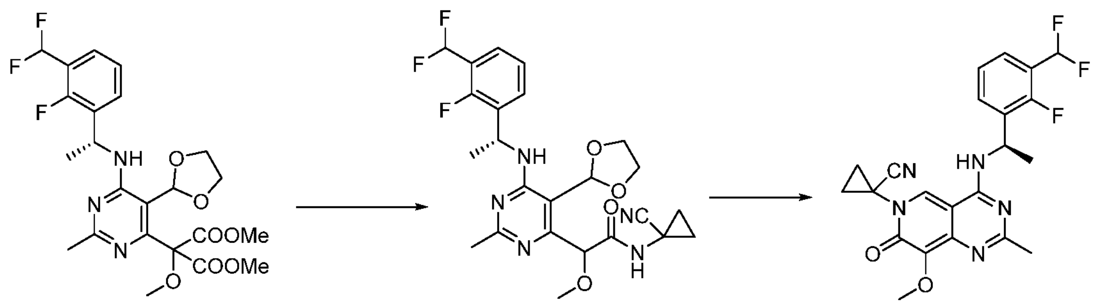

SZ-022244A1 SZ-022344A1 SZ-022344

Step 1:

**SZ-022244A1** (100 mg, 0.19 mmol) was added to dimethyl sulfoxide (2 mL), and then a 10% sodium hydroxide solution (380 μL) was added. The mixture was allowed to react at 40 °C for 1 h. 1-Amino-1-cyclopropanecarbonitrile hydrochloride (34 mg, 0.28 mmol), *N,N,N',N'*-tetramethyl-O-(7-azabenzotriazol-1-yl)uronium hexafluorophosphate (216 mg, 0.57 mmol), and triethylamine (58 mg, 0.57 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022344A1** as a yellow viscous substance (100 mg). LC-MS: $[M+H]^+$ 506.08.

Step 2:

**SZ-022344A1** (100 mg, 0.20 mmol) was added to isopropanol (2 mL), and then a hydrochloric acid solution (4 M, 0.25 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022344** (19.4 mg). LC-MS: $[M+H]^+$ 444.14.

**SZ-022344**: $^1H$ NMR (400 MHz, DMSO-$d_6$) δ 8.97 (s, 1H), 8.75 (d, *J* = 5.2 Hz, 1H) 7.68 (t, *J* = 7.2 Hz, 1H), 7.53 (t, *J* = 7.2 Hz, 1H), 7.32 (t, *J* = 7.6 Hz, 1H), 5.78 - 5.71 (m, 1H), 3.80 (s, 3H), 2.22 (s, 3H), 2.05 - 1.97 (m, 2H), 1.82 - 1.74 (m, 2H), 1.58 (d, *J* = 7.2 Hz, 3H).

**Example 27: SZ-022343**

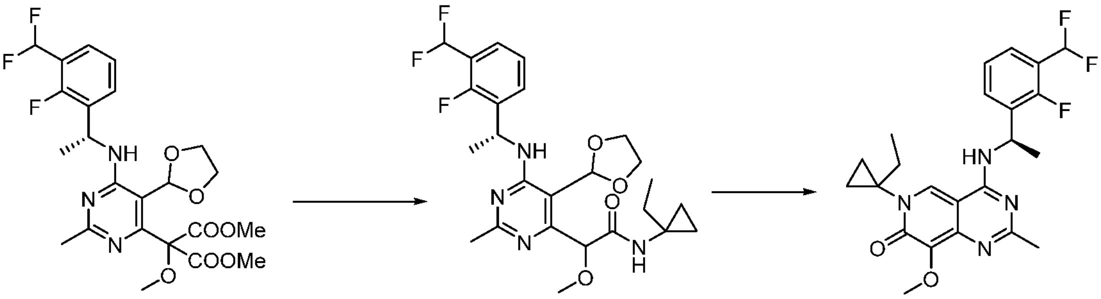

SZ-022244A1 SZ-022343A1 SZ-022343

Step 1:

**SZ-022244A1** (110 mg, 0.21 mmol) was added to dimethyl sulfoxide (2 mL), and then a 10% sodium hydroxide solution (400 μL) was added. The mixture was allowed to react at 40 °C for 1 h. 1-Ethylcyclopropanamine hydrochloride (35 mg, 0.29 mmol), *N*,*N*,*N'*,*N'*-tetramethyl-*O*-(7-azabenzotriazol-1-yl)uronium hexafluorophosphate (223 mg, 0.59 mmol), and triethylamine (59 mg, 0.59 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022343A1** as a yellow viscous substance (110 mg). LC-MS: $[M+H]^+$ 509.29.

Step 2:

**SZ-022343A1** (110 mg, 0.22 mmol) was added to isopropanol (2 mL), and then a hydrochloric acid solution (4 M, 0.25 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022343** (71 mg). LC-MS: $[M+H]^+$ 447.18.

**SZ-022343:** $^1$H NMR (400 MHz, DMSO-*d*$_6$) δ 8.91 (s, 1H), 8.73 (d, *J* = 7.2 Hz, 1H), 7.68 - 7.60 (m, 1H), 7.52 (t, *J* = 7.2 Hz, 1H), 7.31 (t, *J* = 7.6 Hz, 1H), 7.24 (t, *J* = 54.4 Hz, 1H), 5.79 - 5.71 (m, 1H), 3.74 (s, 3H), 2.20 (s, 3H), 1.58 (d, *J* = 7.2 Hz, 3H), 1.10 (br, 4H), 0.81 (t, *J* = 7.2 Hz, 3H).

**Example 28: SZ-022295**

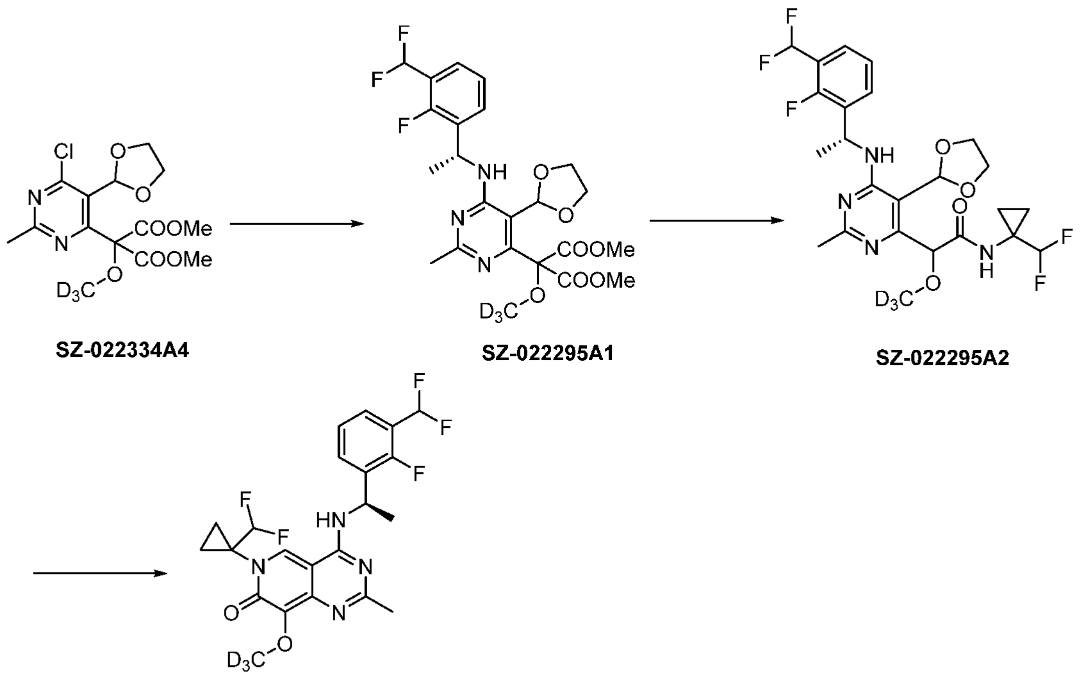

SZ-022334A4 SZ-022295A1 SZ-022295A2

SZ-022295

Step 1:

**SZ-022334A4** (120 mg, 0.33 mmol), (1*R*)-1-(2-fluoro-3-difluoromethylphenyl)ethylamine hydrochloride (112 mg, 0.49 mmol, synthesized according to WO2019122129), triethylamine (100 mg, 0.98 mmol), and potassium carbonate (46 mg, 0.33 mmol) were added to dimethyl sulfoxide (5 mL), and the mixture was heated to 60 °C for reaction for 8 h. After the starting materials were consumed as monitored by LC-MS, water (100 mL) and ethyl acetate (100 mL) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA= 3:1; Rf = 0.3) to give product **SZ-022295A1** as a yellow oil (110 mg). LC-MS: $[M+H]^+$ 516.90.

Step 2:

**SZ-022295A1** (110 mg, 0.21 mmol) was added to dimethyl sulfoxide (2 mL), and then a 10% sodium hydroxide solution (430 μL) was added. The mixture was allowed to react at 40 °C for 1 h. 1-Difluoromethylcyclopropanamine hydrochloride (40 mg, 0.32 mmol), *N,N,N',N'*-tetramethyl-*O*-(7-azabenzotriazol-1-yl)uronium hexafluorophosphate (243 mg, 0.64 mmol), and triethylamine (65 mg, 0.64 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022295A2** as a yellow viscous substance (110 mg). LC-MS: $[M+H]^+$ 534.27.

Step 3:

**SZ-022295A2** (110 mg, 0.25 mmol) was added to isopropanol (5 mL), and then a hydrochloric acid solution (2 M, 0.31 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022295** (47 mg). LC-MS: $[M+H]^+$ 472.20.

**SZ-022295:** $^1$H NMR (400 MHz, DMSO-$d_6$) δ 8.93 (s, 1H), 8.81 (d, *J* = 7.2 Hz, 1H), 7.63 (t, *J* = 7.2 Hz, 1H), 7.52 (t, *J* = 7.2 Hz, 1H), 7.32 (t, *J* = 7.6 Hz, 1H), 7.23 (t, *J* = 54.4 Hz, 1H), 6.34 (t, *J* = 57.2 Hz, 1H), 5.78 - 5.71 (m, 1H), 2.21 (s, 3H), 1.58 (d, *J* = 7.2 Hz, 3H), 1.52 - 1.45 (m, 2H), 1.45 - 1.32 (m, 2H).

**Example 29: SZ-022339**

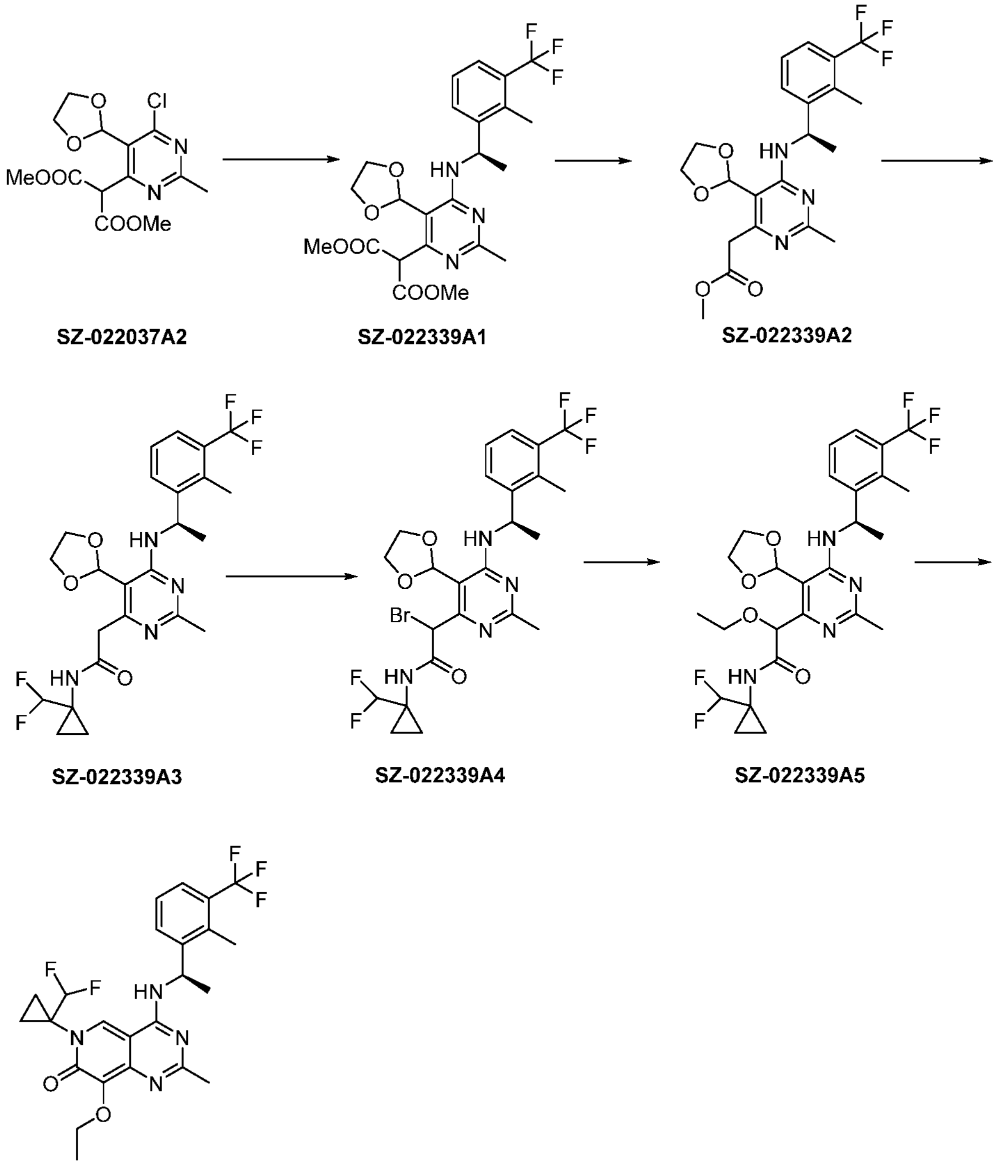

**SZ-022037A2** **SZ-022339A1** **SZ-022339A2**

**SZ-022339A3** **SZ-022339A4** **SZ-022339A5**

**SZ-022339**

Step 1:

**SZ-022037A2** (7.8 g, 23.6 mmol, synthesized according to WO2019122129), (1*R*)-1-(2-methyl-3-trifluoromethylphenyl)ethylamine hydrochloride (5.7 g, 23.6 mmol, synthesized according to WO2019122129), and diisopropylethylamine (9.2 g, 70.8 mmol) were added to *N*,*N*-dimethylformamide (80 mL), and the mixture was heated to 80 °C for reaction for 18 h. After the starting materials were consumed as monitored by LC-MS, water (500 mL) and ethyl acetate (250 mL) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA = 1:1; Rf = 0.3) to give product **SZ-022339A1** as a yellow oil (8.7 g). LC-MS: $[M+H]^+$ 498.07.

Step 2:

**SZ-022339A1** (8.7 g, 17.5 mmol) and lithium chloride (2.97 g, 70.0 mmol) were added to dimethyl sulfoxide (300 mL), and the mixture was heated to 120 °C for reaction for 16 h. After the starting materials were consumed as monitored by LC-MS, water (1500 mL) and ethyl acetate (500 mL) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA = 3:7; Rf = 0.4) to give product **SZ-022339A2** as a yellow oil (3.6 g). LC-MS: $[M+H]^+$ 440.17.

Step 3:

**SZ-022339A2** (500 mg, 1.14 mmol) and sodium hydroxide (182 mg, 4.56 mmol) were added to dimethyl sulfoxide (5 mL), and the mixture was allowed to react at room temperature for 1 h. After the starting materials were consumed as monitored by LC-MS, triethylamine (346 mg, 3.42 mmol), 1-(difluoromethylcyclopropyl)amine hydrochloride (245 mg, 1.71 mmol), and N,N,N,N-tetramethyl-O-(7-azabenzotriazol-1-yl)uronium hexafluorophosphate (1300 mg, 3.42 mmol) were added to the reaction mixture, and the resulting mixture was allowed to react at room temperature for 2 h. Water (50 mL) and ethyl acetate (50 mL) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA = 0:1; Rf = 0.5) to give **SZ-022339A3** as a white solid (500 mg). LC-MS: $[M+H]^+$ 515.21.

Step 4:

**SZ-022339A3** (500 mg, 0.97 mmol), N-bromosuccinimide (173 mg, 0.97 mmol), and azobisisobutyronitrile (16 mg, 0.097 mmol) were added to carbon tetrachloride (15 mL), and the mixture was heated to 60 °C for reaction for 1 h. After the starting materials were consumed as monitored by LC-MS, the reaction mixture was concentrated by rotary evaporation to give a crude product. The crude product was purified by flash silica gel column chromatography (PE:EA = 3: 7; Rf = 0.5) to give product **SZ-022339A4** as a yellow oil (360 mg). LC-MS: $[M+H]^+$ 592.88, 594.79.

Step 5:

Ethanol (340 mg, 7.40 mmol) was dissolved in tetrahydrofuran (10 mL), and sodium hydride (88 mg, 2.22 mmol) was slowly added under argon atmosphere in an ice-water bath. The reaction mixture was stirred for half an hour, and a solution of **SZ-022339A4** (360 mg, 0.61 mmol) in tetrahydrofuran (1 mL) was slowly added dropwise into the above system. The resulting mixture was warmed to room temperature and stirred for 2 h. After the starting materials were consumed as monitored by LC-MS, a saturated ammonium chloride solution (50 mL) and ethyl acetate (100 mL) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA = 0:1; Rf = 0.5) to give product **SZ-022339A5** as a yellow oil (210 mg). LC-MS: $[M+H]^+$ 559.23.

Step 6:

**SZ-022339A5** (210 mg, 0.38 mmol) and hydrochloric acid (0.2 mL, 5 M) were added to isopropanol (2 mL), and the mixture was heated to 50 °C for reaction for 6 h. After the starting materials were consumed as monitored by LC-MS, water (50 mL) and ethyl acetate (50 mL) were added to the reaction mixture for extraction, and the organic layer was separated, washed with a saturated sodium chloride solution, dried, and concentrated by rotary evaporation to give a crude product. The crude product was subjected to preparative high performance liquid chromatography and then lyophilized to give **SZ-022339** (28.49 mg). LC-MS: $[M+H]^+$ 497.2.

**SZ-022339:** $^1$H NMR (400 MHz, DMSO-$d_6$) $\delta$ 8.90 (s, 1H), 8.87 (d, *J* = 8.0 Hz, 1H), 7.70 (d, *J* = 8.0 Hz, 1H), 7.56 (d, *J* = 8.0 Hz, 1H), 7.39 (t, *J* = 8.0 Hz, 1H), 6.34 (t, *J* = 56.0 Hz, 1H), 5.70 - 5.67 (m, 1H), 4.06 (q, *J* = 8.0 Hz, 2H), 2.57 (s, 3H), 2.20 (s, 3H), 1.53 (d, *J* = 8.0 Hz, 3H), 1.50 - 1.35 (m, 4H), 1.21 (t, *J* = 8.0 Hz, 3H).

**Example 30: SZ-022340**

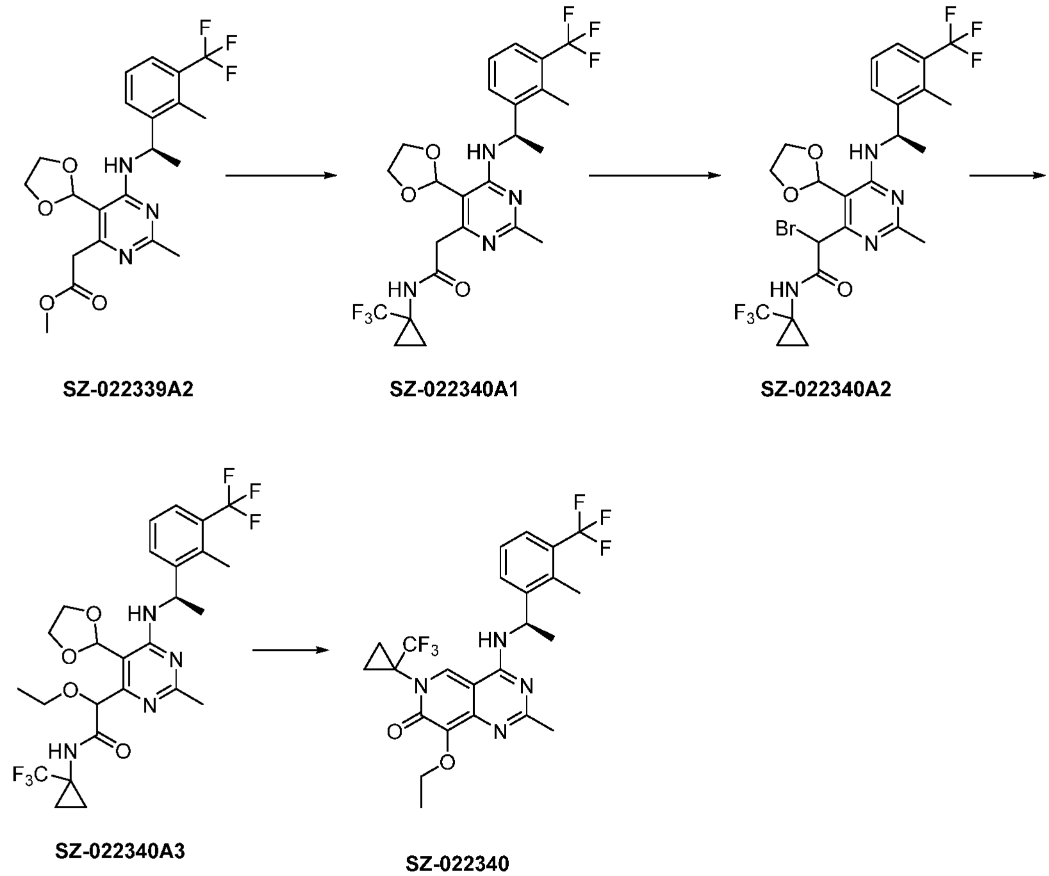

SZ-022339A2 SZ-022340A1 SZ-022340A2

SZ-022340A3 SZ-022340

Step 1:

**SZ-022339A2** (500 mg, 1.14 mmol) and sodium hydroxide (182 mg, 4.56 mmol) were added to dimethyl sulfoxide (5 mL), and the mixture was allowed to react at room temperature for 1 h. After the starting materials were consumed as monitored by LC-MS, triethylamine (346 mg, 3.42 mmol), 1-(trifluoromethylcyclopropyl)amine hydrochloride (276 mg, 1.71 mmol), and *N*,*N*,*N'*,*N'*-tetramethyl-*O*-(7-azabenzotriazol-1-yl)uronium hexafluorophosphate (1300 mg, 3.42 mmol) were added to the reaction mixture, and the resulting mixture was allowed to react at room temperature for 2 h. Water (50 mL) and ethyl acetate (50 mL) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA = 0:1; Rf = 0.5) to give **SZ-022340A1** as a yellow solid (440 mg). LC-MS: $[M+H]^+$ 533.12.

Step 2:

**SZ-022340A1** (440 mg, 0.83 mmol), N-bromosuccinimide (147 mg, 0.83 mmol), and azobisisobutyronitrile (14 mg, 0.083 mmol) were added to carbon tetrachloride (15 mL), and the mixture was heated to 60 °C for reaction for 1 h. After the starting materials were consumed as monitored by LC-MS, the reaction mixture was concentrated by rotary evaporation to give a crude product. The crude product was purified by flash silica gel column chromatography (PE:EA = 3: 7; Rf = 0.5) to give **SZ-022340A2** as a white solid (280 mg). LC-MS: $[M+H]^+$ 610.90, 612.83.

Step 3:

Ethanol (210 mg, 4.60 mmol) was dissolved in tetrahydrofuran (10 mL), and sodium hydride (56 mg, 1.38 mmol) was slowly added under argon atmosphere in an ice-water bath. The reaction mixture was stirred for half an hour, and a solution of **SZ-022340A2** (280 mg, 0.46 mmol) in tetrahydrofuran (1 mL) was slowly added dropwise into the above system. The resulting mixture was warmed to room temperature and stirred for 2 h. After the starting materials were consumed as monitored by LC-MS, a saturated ammonium chloride solution (50 mL) and ethyl acetate (100 mL) were added to the reaction mixture for extraction, and the organic layer was separated, washed with a saturated sodium chloride solution, dried, and concentrated by rotary evaporation to give a crude product. The crude product was purified

by flash silica gel column chromatography (PE:EA = 0: 1; Rf = 0.5) to give product **SZ-022340A3** as a yellow oil (70 mg). LC-MS: $[M+H]^+$ 577.20.

Step 4:

**SZ-022340A3** (70 mg, 0.12 mmol) and hydrochloric acid (0.1 mL, 5 M) were added to isopropanol (2 mL), and the mixture was heated to 50 °C for reaction for 6 h. After the starting materials were consumed as monitored by LC-MS, water (50 mL) and ethyl acetate (50 mL) were added to the reaction mixture for extraction, and the organic layer was separated, washed with a saturated sodium chloride solution, dried, and concentrated by rotary evaporation to give a crude product. The crude product was subjected to preparative high performance liquid chromatography and then lyophilized to give **SZ-022340** (13.02 mg). LC-MS: $[M+H]^+$ 515.18.

**SZ-022340:** $^1$H NMR (400 MHz, DMSO-$d_6$) δ 8.96 (s, 1H), 8.87 (d, *J* = 8.0 Hz, 1H), 7.70 (d, *J* = 8.0 Hz, 1H), 7.56 (d, *J* = 8.0 Hz, 1H), 7.39 (t, *J* = 8.0 Hz, 1H), 5.70 - 5.66 (m, 1H), 4.05 (q, *J* = 8.0 Hz, 2H), 2.57 (s, 3H), 2.20 (s, 3H), 1.81 - 1.65 (m, 4H), 1.54 (d, *J* = 8.0 Hz, 3H), 1.20 (t, *J* = 8.0 Hz, 3H).

**Example 31: SZ-022345**

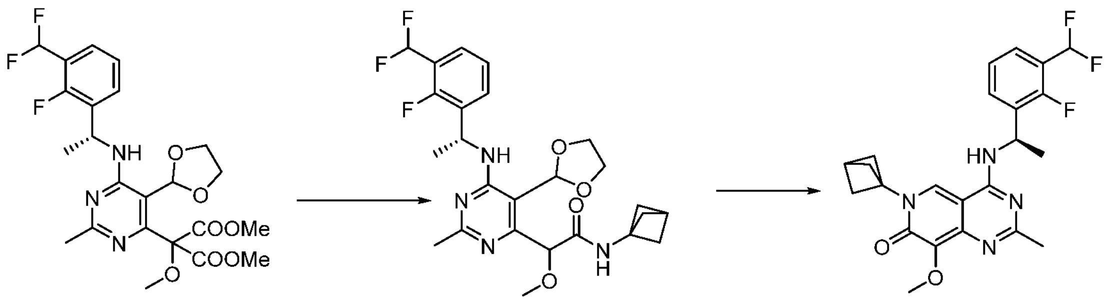

SZ-022244A1 SZ-022345A1 SZ-022345

Step 1:

**SZ-022244A1** (100 mg, 0.19 mmol) was added to dimethyl sulfoxide (2 mL), and then a 10% sodium hydroxide solution (500 μL) was added. The mixture was allowed to react at 40 °C for 1 h. 1-Bicyclo[1,1,1]pentylamine hydrochloride (34 mg, 0.28 mmol), *N*,*N*,*N'*,*N'*-tetramethyl-O-(7-azabenzotriazol-1-yl)uronium hexafluorophosphate (216 mg, 0.57 mmol), and triethylamine (58 mg, 0.57 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022345A1** as a yellow viscous substance (210 mg). LC-MS: $[M+H]^+$ 507.13.

Step 2:

**SZ-022345A1** (210 mg, 0.19 mmol) was added to isopropanol (2 mL), and then a hydrochloric acid solution (2 M, 0.6 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022345** (29 mg). LC-MS: $[M+H]^+$ 444.89.

**SZ-022345:** $^1$H NMR (400 MHz, DMSO-$d_6$) δ 8.77 (d, *J* = 7.2 Hz, 1H), 8.60 (s, 1H), 7.65 (t, *J* = 7.2 Hz, 1H), 7.52 (t, *J* = 6.8 Hz, 1H), 7.32 (t, *J* = 8.0 Hz, 1H), 7.24 (t, *J* = 54.4 Hz, 1H), 5.81 - 5.74 (m, 1H), 3.75 (s, 3H), 2.72 (s, 1H), 2.21 (s, 3H), 1.59 (d, *J*= 7.2 Hz, 3H).

**Example 32: SZ-022346**

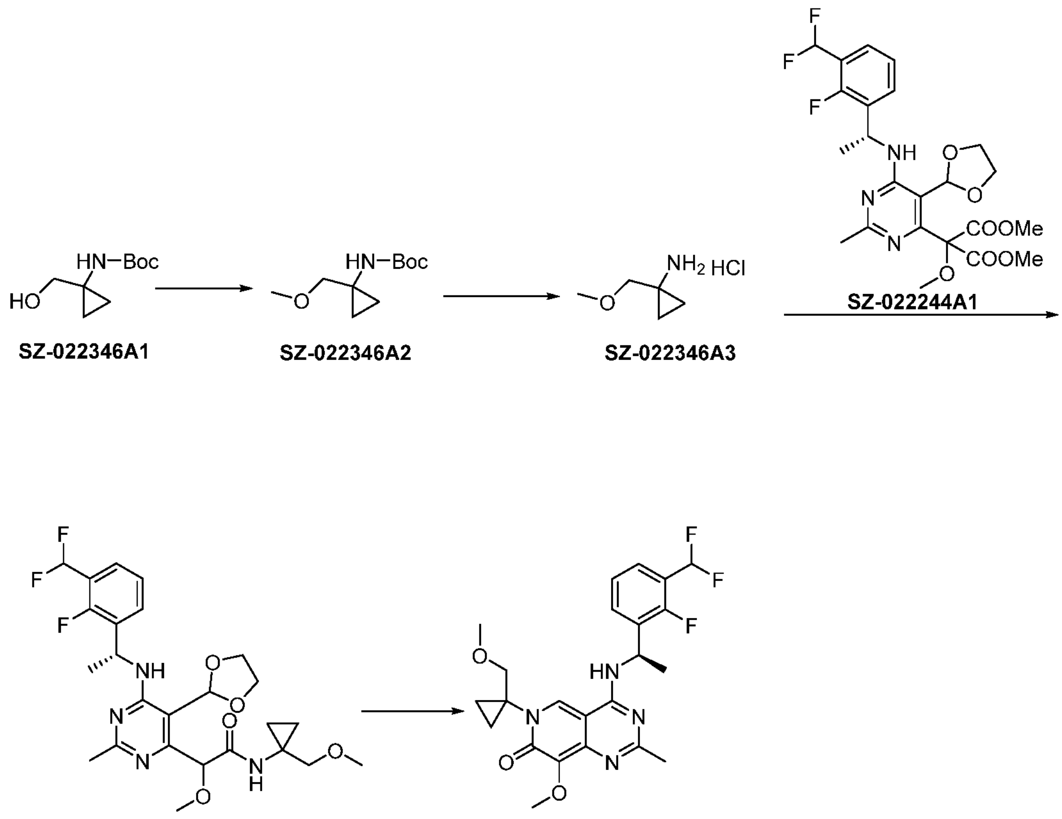

SZ-022346A1 SZ-022346A2 SZ-022346A3 SZ-022244A1

SZ-022346A4 SZ-022346

Step 1:

**SZ-022346A1** (500 mg, 2.67 mmol) was added to *N*,*N*-dimethylformamide (5 mL), and the mixture was cooled to 0 °C under argon atmosphere. Sodium hydride (128 mg, 3.20 mmol) was added, and the mixture was stirred at 0 °C for 0.5 h. Methyl iodide (417 mg, 2.94 mmol) was then added dropwise, and the resulting mixture was naturally warmed to room temperature and stirred for 15 h. After the starting materials were consumed as monitored by TLC, water (50 mL) and ethyl acetate (50 mL) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA = 10:1; Rf = 0.5) to give product **SZ-022346A2** as a colorless oil (220 mg).

**SZ-022346A2:** $^1$H NMR (400 MHz, $CDCl_3$) $\delta$ 5.03 (s, 1H), 4.14-4.09 (m, 1H), 3.38 (s, 2H), 3.36 (s, 3H), 1.44 (s, 9H), 0.83-0.72 (m, 4H).

Step 2:

**SZ-022346A2** (220 mg, 0.99 mmol) was added to a solution of hydrogen chloride in 1,4-dioxane (4 M, 5 mL), and the mixture was allowed to react at room temperature for 1 h. After the starting materials were consumed as monitored by TLC, the reaction mixture was concentrated by rotary evaporation to give **SZ-022346A3** as an off-white solid (150 mg). The crude product was used directly in the next step.

Step 3:

**SZ-022244A1** (100 mg, 0.19 mmol) was added to dimethyl sulfoxide (5 mL), and then a 10% sodium hydroxide solution (500 μL) was added. The mixture was allowed to react at 40 °C for 1 h. **SZ-022346A3** (150 mg, 0.99 mmol), *N*,*N*,*N'*,*N'*-tetramethyl-*O*-(7-azabenzotriazol-1-yl)uronium hexafluorophosphate (216 mg, 0.57 mmol), and triethylamine (58 mg, 0.57 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022346A4** as a light yellow viscous substance (180 mg). LC-MS: $[M+H]^+$ 525.12.

Step 4:

**SZ-022346A4** (180 mg, 0.19 mmol) was added to isopropanol (2 mL), and then a hydrochloric acid solution (2 M, 0.5 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022346** (60 mg). LC-MS: $[M+H]^+$ 462.96.

**SZ-022346:** $^1$H NMR (400 MHz, DMSO-$d_6$) δ 8.94 (s, 1H), 8.84 (d, *J* = 6.8 Hz, 1H), 7.66 (t, *J* = 7.2 Hz, 1H), 7.55 (t, *J* = 7.2 Hz, 1H), 7.35 (t, *J* = 8.0 Hz, 1H), 7.26 (t, *J* = 54.4 Hz, 1H), 5.82 - 5.75 (m, 1H), 3.78 (s, 3H), 3.24 (s, 3H), 2.24 (s, 3H), 1.61 (d, *J* = 7.2 Hz, 3H), 1.20 (s, 4H).

**Example 33: SZ-022347**

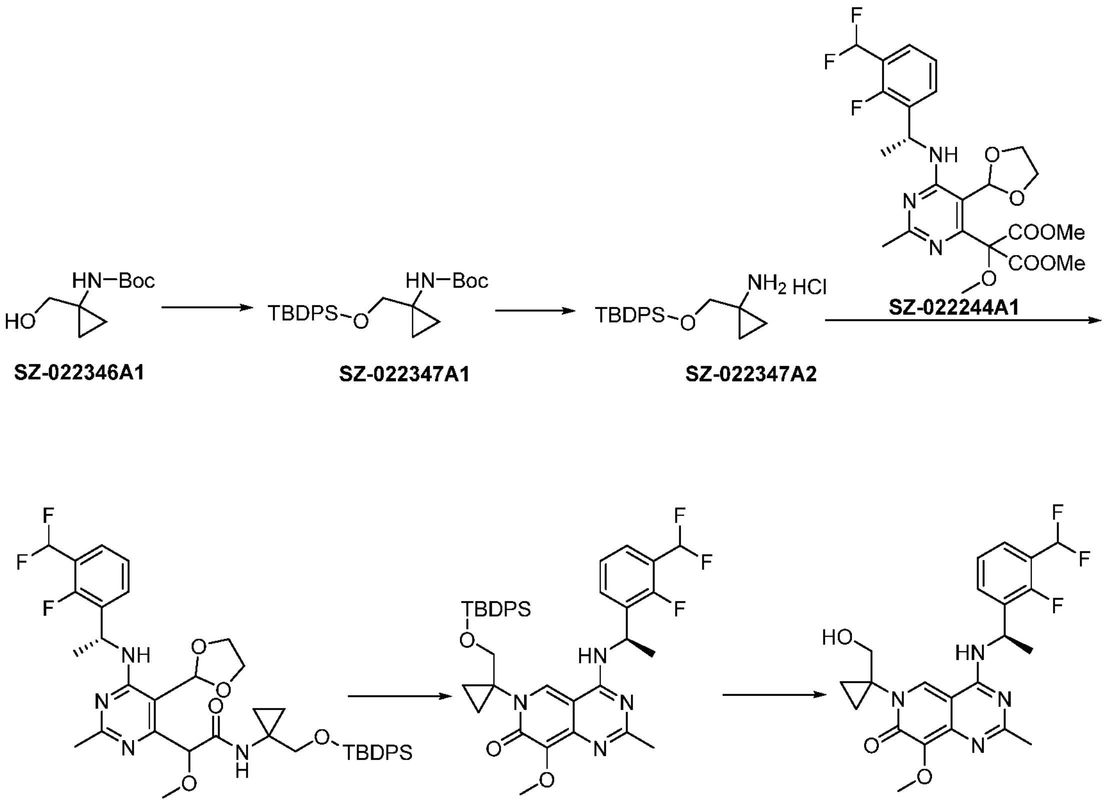

**SZ-022346A1** **SZ-022347A1** **SZ-022347A2** **SZ-022244A1**

**SZ-022347A3** **SZ-022347A4** **SZ-022347**

Step 1:

**SZ-022346A1** (500 mg, 2.67 mmol), *tert*-butyldiphenylchlorosilane (771 mg, 2.80 mmol), and imidazole (382 mg, 5.61 mmol) were added to dichloromethane (5 mL), and the mixture was stirred at room temperature for 15 h. After the starting materials were consumed as monitored by TLC, water (50 mL) and dichloromethane (50 mL) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA = 10:1; Rf = 0.5) to give product **SZ-022347A1** as a colorless oil (1.1 g).

**SZ-022347A1:** $^1$H NMR (400 MHz, DMSO-$d_6$) δ 7.62-7.59 (m, 4H), 7.46-7.39 (m, 6H), 7.20 (s, 1H), 3.65 (s, 2H), 1.35 (s, 9H), 0.99 (s, 9H), 0.78-0.65 (m, 2H), 0.62-0.59 (m, 2H).

Step 2:

**SZ-022347A1** (1.1 g, 2.58 mmol) was added to a solution of hydrogen chloride in 1,4-dioxane (4 M, 5 mL), and the mixture was allowed to react at room temperature for 1 h. After the starting materials were consumed as monitored by TLC, the reaction mixture was concentrated by rotary evaporation to give **SZ-022347A2** as an off-white solid (800 mg). The crude product was used directly in the next step.

Step 3:

**SZ-022244A1** (100 mg, 0.19 mmol) was added to dimethyl sulfoxide (5 mL), and then a 10% sodium hydroxide solution (500 μL) was added. The mixture was allowed to react at 40 °C for 1 h. **SZ-022347A2** (800 mg, 2.21 mmol), *N*,*N*,*N*',*N*'-tetramethyl-*O*-(7-azabenzotriazol-1-yl)uronium hexafluorophosphate (216 mg, 0.57 mmol), and triethylamine (58 mg, 0.57 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA = 1: 1; Rf = 0.4) to give product **SZ-022347A3** as a light yellow oil (200 mg). LC-MS: $[M+H]^+$ 749.97.

Step 4:

**SZ-022347A3** (200 mg, 0.26 mmol) was added to isopropanol (2 mL), and then a hydrochloric acid solution (2 M, 0.5 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation to give **SZ-022347A4** as a light yellow oil (160 mg). LC-MS: $[M+H]^+$ 687.87.

Step 5:

**SZ-022347A4** (160 mg, 0.23 mmol) was added to a solution of tetrabutylammonium fluoride in tetrahydrofuran (1 M, 4 mL), and the mixture was allowed to react at room temperature for 3 h. After the starting materials were consumed as monitored by LC-MS, the reaction mixture was directly concentrated by rotary evaporation. Water (20 mL) and ethyl acetate (20 mL) were then added for extraction, and the organic layer was separated, washed with a saturated sodium chloride solution, dried, and concentrated by rotary evaporation. The residue was subjected to preparative high performance liquid chromatography and then lyophilized to give **SZ-022347** (24 mg). LC-MS: $[M+H]^+$.

**SZ-022347:** $^1$H NMR (400 MHz, DMSO-$d_6$) δ 8.90 (s, 1H), 8.79 (d, *J* = 6.8 Hz, 1H), 7.66 (t, *J* = 7.2 Hz, 1H), 7.52 (t, *J* = 6.8 Hz, 1H), 7.32 (t, *J* = 7.6 Hz, 1H), 7.23 (t, *J* = 54.4 Hz, 1H), 5.81 - 5.74 (m, 1H), 5.01 (t, *J* = 6.0 Hz, 1H), 3.75 (s, 3H), 3.21 (s, 3H), 1.57 (d, *J*= 6.8 Hz, 3H), 1.22-1.10 (m, 4H).

**Example 34: SZ-022337**

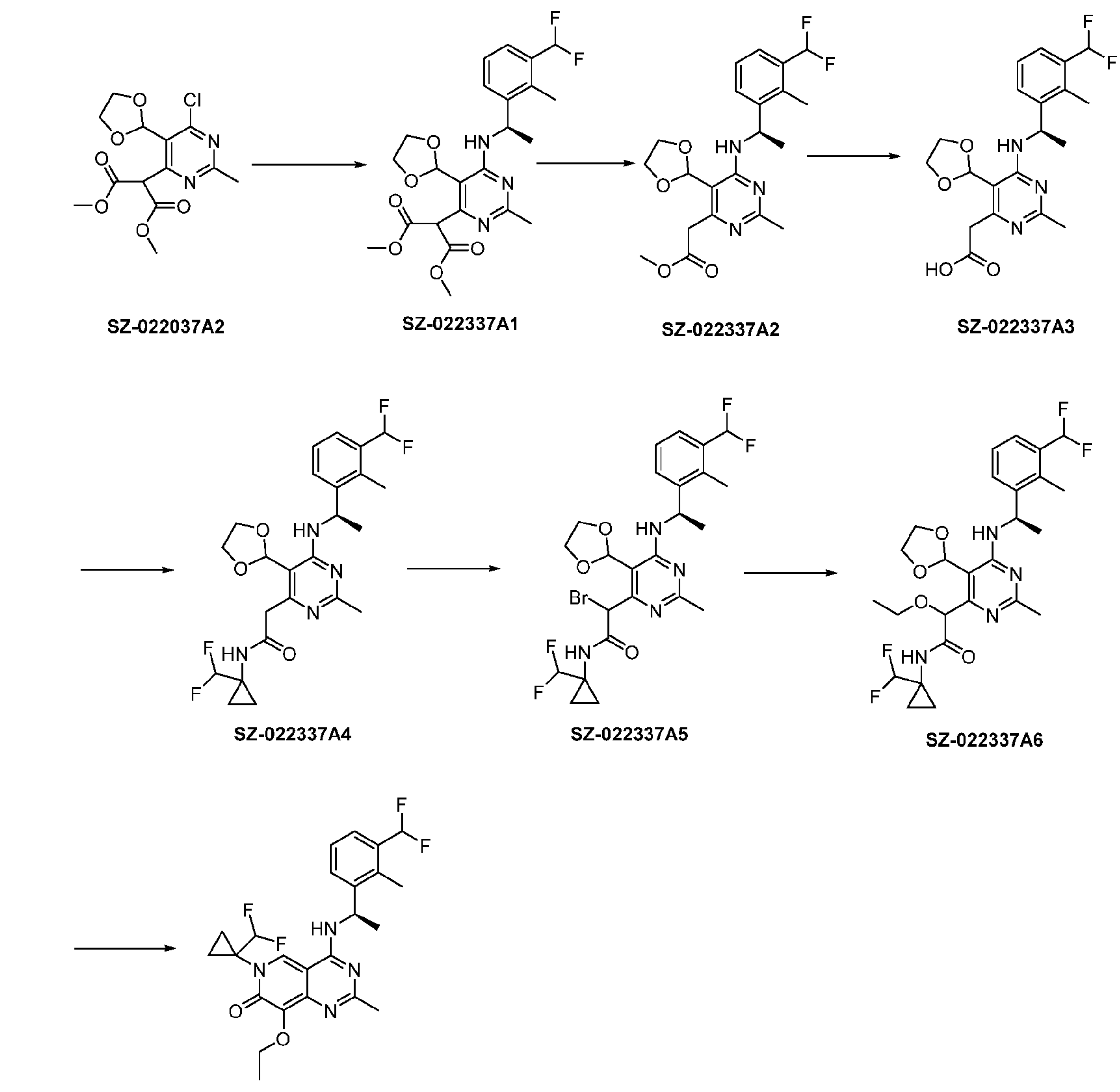

SZ-022037A2 SZ-022337A1 SZ-022337A2 SZ-022337A3

SZ-022337A4 SZ-022337A5 SZ-022337A6

SZ-022337

Step 1:

**SZ-022037A2** (1 g, 3.02 mmol, synthesized according to WO2019122129) and (1*R*)-1-(3-(difluoromethyl)-2-methylphenyl)ethylamine hydrochloride (670 mg, 3.02 mmol, synthesized according to WO2019122129) were added to *N*,*N*-dimethylformamide (10 mL), and then diisopropylethylamine (1.2 g, 9.07 mmol) was added. The mixture was heated to 80 °C for reaction for 8 h. After the starting materials were consumed as monitored by LC-MS, water (50 mL) and ethyl acetate (50 mL) were added to the reaction mixture for extraction, and the organic layer was separated, washed with a saturated sodium chloride solution, dried, and concentrated to dryness under reduced pressure to give a crude product. The crude product was purified by flash silica gel column chromatography (PE:EA = 3:2) to give **SZ-022337A1** as a light yellow oil (1.2 g). LC-MS: $[M+H]^+$ 480.04.

Step 2:

**SZ-022337A1** (1.2 g, 2.50 mmol) was added to dimethyl sulfoxide (10 mL), and then anhydrous lithium chloride (425 mg, 10.0 mL) was added. The mixture was heated to 120 °C for reaction for 16 h. After the starting materials were consumed as monitored by LC-MS, water (40 mL) and ethyl acetate (40 mL) were added to the reaction mixture for extraction, and the organic layer was separated, washed with a saturated sodium chloride solution, dried, and concentrated to dryness under reduced pressure to give a crude product. The crude product was purified by flash silica gel column chromatography (PE:EA = 2:3) to give **SZ-022337A2** as a light yellow oil (320 mg). LC-MS: $[M+H]^+$ 421.94.

Step 3:

**SZ-022337A2** (320 mg, 0.76 mmol) was added to dimethyl sulfoxide (3 mL), and then a 10% sodium hydroxide solution (1.2 mL, 3.04 mmol) was added. The mixture was allowed to react at room temperature for 1 h. After the starting materials were consumed as monitored by LC-MS, the reaction mixture was used directly in the next step without work-up. LC-MS: $[M+H]^+$ 407.91.

Step 4:

1-Difluoromethylcyclopropanamine hydrochloride (78 mg, 0.55 mmol), *N,N,N',N'*-tetramethyl-*O*-(7-azabenzotriazol-1-yl)uronium hexafluorophosphate (415 mg, 1.09 mmol), and triethylamine (110 mg, 1.09 mmol) were added to the reaction system in the previous step, and the mixture was allowed to react at room temperature for 1 h. After the starting materials were consumed as monitored by LC-MS, water (20 mL) and ethyl acetate (30 mL) were added to the reaction mixture for extraction, and the organic layer was separated, washed with a saturated sodium chloride solution, dried, and concentrated to dryness under reduced pressure to give a crude product. The crude product was purified by flash silica gel column chromatography (PE:EA = 2:3) to give **SZ-022337A4** as a light yellow oil (220 mg). LC-MS: $[M+H]^+$ 497.07.

Step 5:

**SZ-022337A4** (220 mg, 0.44 mmol) was added to carbon tetrachloride (4 mL), and then N-bromosuccinimide (80 mg, 0.44 mmol) and azobisisobutyronitrile (7 mg, 0.044 mmol) were added. The mixture was heated to 60 °C for reaction for 1 h. After the starting materials were consumed as monitored by LC-MS, the reaction mixture was directly purified by flash silica gel column chromatography (PE:EA = 4:1) to give **SZ-022337A5** as a light yellow oil (120 mg). LC-MS: $[M+H]^+$ 574.74.

Step 6:

Sodium hydride (25 mg, 0.63 mmol) was added to tetrahydrofuran (3 mL), and then ethanol (19 mg, 0.42 mmol) was added. The mixture was stirred at 0 °C for 0.5 h. A solution of **SZ-022337A5** (120 mg, 0.21 mmol) in tetrahydrofuran (1.5 mL) was added dropwise to the reaction mixture, and the resulting mixture was stirred for another 2.5 h. After the starting materials were consumed as monitored by LC-MS, water (10 mL) and ethyl acetate (10 mL) were added to the reaction mixture for extraction, and the organic layer was separated, washed with a saturated sodium chloride solution, dried, and concentrated by rotary evaporation to give crude product **SZ-022337A6** as a yellow oil (85 mg). LC-MS: $[M+H]^+$ 541.12.

Step 7:

**SZ-022337A6** (40 mg, 0.07 mmol) was added to isopropanol (3 mL), and then a hydrochloric acid solution (2 M, 1.5 mL) was added. The mixture was allowed to react at 50 °C for 5 h. After the starting materials were consumed as monitored by LC-MS, the reaction mixture was directly concentrated by rotary evaporation. A saturated sodium bicarbonate solution (5 mL) and ethyl acetate (10 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022337** (50 mg). LC-MS: $[M+H]^+$ 478.94.

**SZ-022337:** $^1H$ NMR (400 MHz, $CD_3OD$) δ 8.92 (s, 1H), 7.56 (d, *J*= 7.6 Hz, 1H), 7.42 (d, *J* = 7.6 Hz, 1H), 7.29 (t, *J* = 7.6 Hz, 1H), 6.95 (t, *J* = 55.2 Hz, 1H), 6.25 (t, *J* = 57.6 Hz, 1H), 5.87 - 5.82 (m, 1H), 4.16 (q, *J*= 7.2 Hz, 2H), 2.54 (s, 3H), 2.35 (s, 3H), 1.61 - 1.59 (m, 5H), 1.42 (br, 2H), 1.36 (t, *J* = 6.8 Hz, 3H).

**Example 35: SZ-022338**

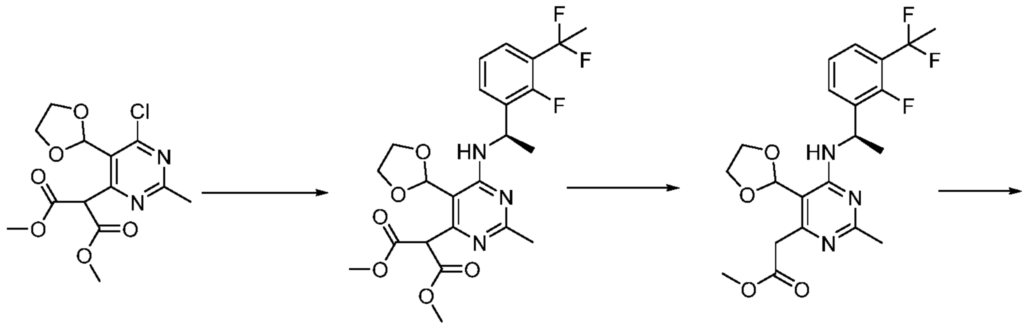

SZ-022037A2 SZ-022338A1 SZ-022338A2

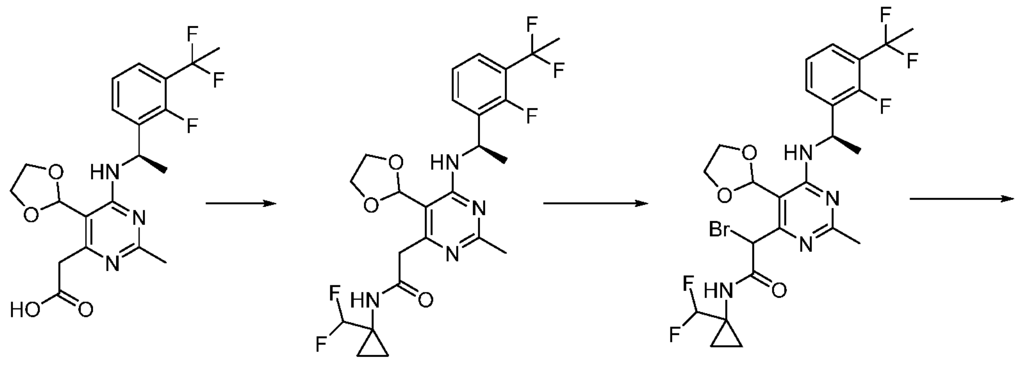

SZ-022338A3 SZ-022338A4 SZ-022338A5

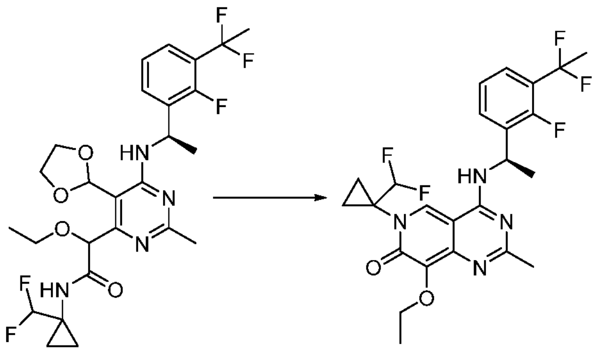

SZ-022338A6 SZ-022338

Step 1:

**SZ-022037A2** (690 mg, 1.73 mmol, synthesized according to WO2019122129) and (1*R*)-1-(3-(1,1-difluoroethyl)-2-fluorophenyl)ethylamine hydrochloride (494 mg, 1.73 mmol, synthesized according to WO2019122129) were added to *N*,*N*-dimethylformamide (6 mL), and then diisopropylethylamine (810 mg, 6.26 mmol) was added. The mixture was heated to 80 °C for reaction for 8 h. After the starting materials were consumed as monitored by LC-MS, water (30 mL) and ethyl acetate (30 mL) were added to the reaction mixture for extraction, and the organic layer was separated, washed with a saturated sodium chloride solution, dried, and concentrated to dryness under reduced pressure to give a crude product. The crude product was purified by flash silica gel column chromatography (PE:EA = 3:2) to give **SZ-022338A1** as a light yellow oil (600 mg). LC-MS: $[M+H]^+$ 498.20.

Step 2:

**SZ-022338A1** (600 mg, 1.21 mmol) was added to dimethyl sulfoxide (5 mL), and then anhydrous lithium chloride (205 mg, 4.83 mL) was added. The mixture was heated to 120 °C for reaction for 16 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated to dryness under reduced pressure to give a crude product. The crude product was purified by flash silica gel column chromatography (PE:EA = 3:2) to give **SZ-022338A2** as a light yellow oil (160 mg). LC-MS: $[M+H]^+$ 439.94.

Step 3:

**SZ-022338A2** (160 mg, 0.36 mmol) was added to dimethyl sulfoxide (2 mL), and then a 10% sodium hydroxide solution (0.58 mL, 1.46 mmol) was added. The mixture was allowed to react at room temperature for 1 h. After the starting materials were consumed as monitored by LC-MS, the reaction mixture was used directly in the next step without work-up. LC-MS: $[M+H]^+$ 426.11.

Step 4:

(Difluoromethyl)cyclopropyl-1-amine hydrochloride (78 mg, 0.55 mmol), *N,N,N',N'*-tetramethyl-*O*-(7-azabenzotriazol-1-yl)uronium hexafluorophosphate (415 mg, 1.09 mmol), and triethylamine (110 mg, 1.09 mmol) were added to the reaction system in the previous step, and the mixture was allowed to react at room temperature for 1 h. After the starting materials were consumed as monitored by LC-MS, water (20 mL) and ethyl acetate (30 mL) were added to the reaction mixture for extraction, and the organic layer was separated, washed with a saturated sodium chloride solution, dried, and concentrated to dryness under reduced pressure to give a crude product. The crude product was purified by flash silica gel column chromatography (PE:EA = 2:3) to give **SZ-022338A4** as a light yellow oil (120 mg). LC-MS: $[M+H]^+$ 515.22.

Step 5:

**SZ-022338A4** (120 mg, 0.23 mmol) was added to carbon tetrachloride (3 mL), and then N-bromosuccinimide (42 mg, 0.23 mmol) and azobisisobutyronitrile (4 mg, 0.023 mmol) were added. The mixture was heated to 120 °C for reaction for 1 h. After the starting materials were consumed as monitored by LC-MS, the reaction mixture was directly purified by flash silica gel column chromatography (PE:EA = 4:1) to give **SZ-022338A5** as a light yellow oil (90 mg). LC-MS: $[M+H]^+$ 592.73.

Step 6:

Sodium hydride (18 mg, 0.45 mmol) was added to tetrahydrofuran (2.5 mL), and then ethanol (13.8 mg, 0.3 mmol) was added. The mixture was stirred at 0 °C for 0.5 h. A solution of **SZ-022338A5** (90 mg, 0.15 mmol) in tetrahydrofuran (1 mL) was added dropwise to the reaction mixture, and the resulting mixture was stirred for another 2.5 h. After the starting materials were consumed as monitored by LC-MS, water (10 mL) and ethyl acetate (10 mL) were added to the reaction mixture for extraction, and the organic layer was separated, washed with a saturated sodium chloride solution, dried, and concentrated by rotary evaporation to give crude product **SZ-022338A6** as a yellow oil (40 mg). LC-MS: $[M+H]^+$ 559.05.

Step 7:

**SZ-022338A6** (40 mg, 0.07 mmol) was added to isopropanol (2 mL), and then a hydrochloric acid solution (2 M, 1 mL) was added. The mixture was allowed to react at 50 °C for 5 h. After the starting materials were consumed as monitored by LC-MS, the reaction mixture was directly concentrated by rotary evaporation. A saturated sodium bicarbonate solution (5 mL) and ethyl acetate (10 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022338** (18 mg). LC-MS: $[M+H]^+$ 496.99.

**SZ-022338:** $^1$H NMR (400 MHz, DMSO-$d_6$) δ 8.94 (s, 1H), 8.89 (d, *J* = 6.4 Hz, 1H), 7.59 (t, *J* = 7.2 Hz, 1H), 7.42 (t, *J* = 7.2 Hz, 1H), 7.24 (t, *J* = 7.6 Hz, 1H), 6.31 (t, *J* = 57.2 Hz, 1H), 5.75 - 5.68 (m, 1H), 4.04 (q, *J* = 6.8 Hz, 2H), 2.18 (s, 3H), 1.99 (t, *J* = 19.2 Hz, 3H), 1.55 (d, *J* = 7.2 Hz, 3H), 1.40 (br, 4H), 1.19 (t, *J* = 7.2 Hz, 3H).

**Example 36: SZ-022313**

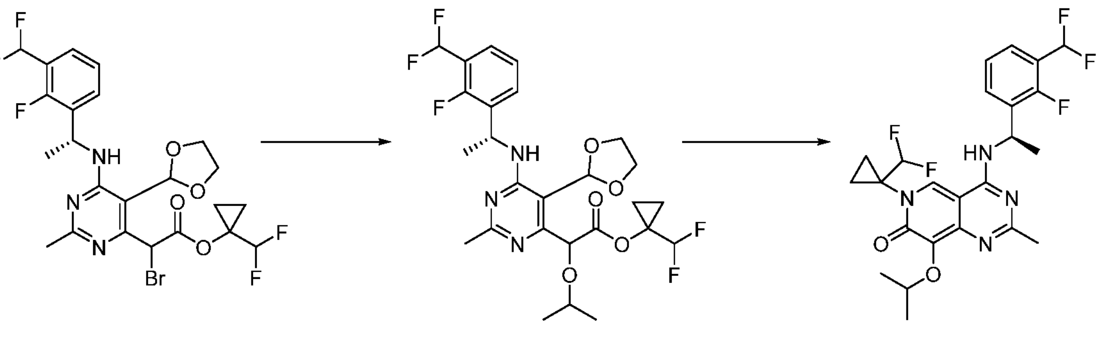

SZ-022307A1 SZ-022313A1 SZ-022313

Step 1:

Sodium hydride (14 mg, 0.3 mmol) was added to tetrahydrofuran (2 mL), and then isopropanol (12 mg, 0.2 mmol) was added. The mixture was stirred at room temperature for 0.5 h. A solution of **SZ-022307A1** (60 mg, 0.1 mmol) in tetrahydrofuran (1 mL) was added dropwise to the reaction mixture, and the resulting mixture was stirred for another 1.5 h. After the starting materials were consumed as monitored by LC-MS, water (10 mL) and ethyl acetate (10 mL) were added to the reaction mixture for extraction, and the organic layer was separated, washed with a saturated sodium chloride solution, dried, and concentrated by rotary evaporation to give crude product **SZ-022313A1** as a yellow viscous substance (29 mg). LC-MS: $[M+H]^+$ 559.22.

Step 2:

**SZ-022313A1** (29 mg, 0.1 mmol) was added to isopropanol (2 mL), and then a hydrochloric acid solution (2 M, 0.5 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL) and ethyl acetate (10 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022313** (11 mg). LC-MS: $[M+H]^+$ 496.90.

**SZ-022313:** $^1$H NMR (400 MHz, DMSO-$d_6$) $\delta$ 8.92 (s, 1H), 8.82 (d, $J$= 7.2 Hz, 1H), 7.66 (t, $J$ = 7.6 Hz, 1H), 7.51 (t, $J$ = 7.2 Hz, 1H), 7.31 (t, $J$ = 7.6 Hz, 1H), 7.22 (t, $J$ = 54.4 Hz, 1H), 6.33 (t, $J$ = 57.2 Hz, 1H), 5.76 - 5.73 (m, 1H), 4.61 - 4.64 (m, 1H), 2.19 (s, 3H), 1.57 (d, $J$ = 6.8 Hz, 3H), 1.47 (br, 4H), 1.16 (dd, $J_1$ = 4.0 Hz, $J_2$ = 6.4 Hz, 6H).

**Example 37: SZ-022323**

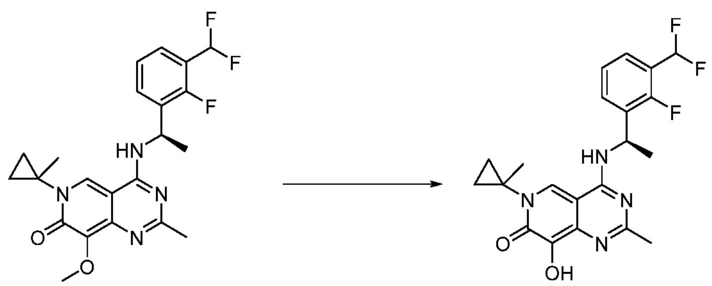

SZ-022300 SZ-022323

Step 1:

**SZ-022300** (38 mg, 88 $\mu$mol) was added to pyridine (1 mL), and then pyridine hydrochloride (102 mg, 0.88 mmol) was added. The mixture was purged with argon for 1 min and then microwaved at 150 °C for reaction for 2 h. After the starting materials were consumed as monitored by LC-MS, the reaction mixture was poured into water (10

mL), and the solution was adjusted to pH 6-7 with 1 M diluted hydrochloric acid. Ethyl acetate (20 mL) was then added for extraction, and the organic layer was separated and washed with a saturated sodium chloride solution. The solvent was removed by rotary evaporation, and the residue was subjected to preparative high performance liquid chromatography and then lyophilized to give **SZ-022323** (8 mg). LC-MS: $[M+H]^+$ 419.10.

**SZ-022323:** $^1$H NMR (400 MHz, DMSO-$d_6$) δ 8.66 (s, 1H), 8.56 (d, *J* = 7.2 Hz, 1H), 7.66 (t, *J* = 7.2 Hz, 1H), 7.51 (t, *J* = 7.2 Hz, 1H), 7.31 (t, *J* = 7.6 Hz, 1H), 7.23 (t, *J* = 54 Hz, 1H), 5.79 - 5.72 (m, 1H), 2.19 (s, 3H), 1.57 (d, *J* = 7.2 Hz, 3H), 1.52 (s, 3H), 1.16 - 1.10 (m, 2H), 1.08 - 1.01 (m, 2H).

**Example 38: SZ-022299**

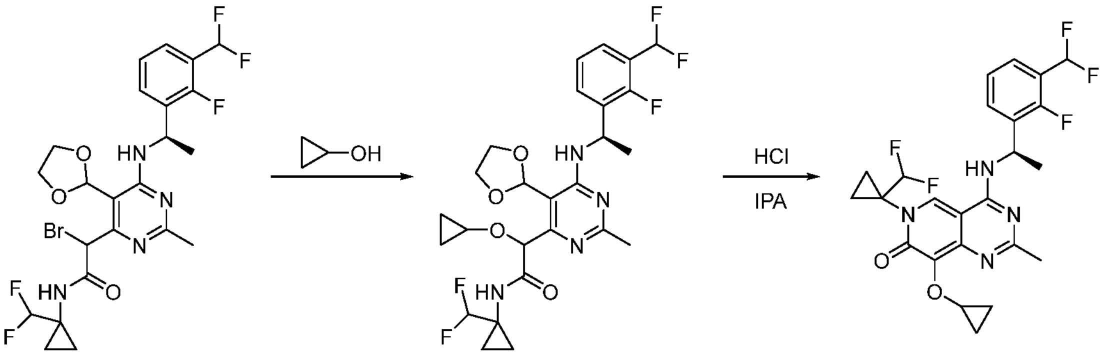


**022299A1** **022299A2** **SZ-022299**

Step 1:

60% NaH (55 mg, 1.381 mmol, 10 eq) was added to a three-necked flask, and under argon atmosphere, anhydrous tetrahydrofuran (5 mL) was added via a syringe. Cyclopropanol (88 mg, 1.519 mmol, 11 eq) was then added dropwise at room temperature, and the mixture was stirred at room temperature for 30 min. The reaction mixture was then stirred in an ice-water bath, and a solution of **022299A1** (80 mg, 0.138 mmol, 1 eq) and dry tetrahydrofuran (1 mL) was added. The resulting mixture was naturally warmed and stirred for 30 min, and then the reaction was substantially completed as indicated by LC-MS. A saturated ammonium chloride solution (10 mL × 1) and water (20 mL × 1) were added to the reaction mixture, and the mixture was extracted with ethyl acetate (20 mL × 3). The organic phases were combined, washed with saturated brine (20 mL × 1), dried over anhydrous sodium sulfate, and filtered. The filtrate was concentrated under reduced pressure, subjected to column chromatography with a 25 g silica gel column, and subjected to gradient elution with 0-65% ethyl acetate/petroleum ether. The fractions were collected and concentrated under reduced pressure to give **022299A2** as a colorless oil (71 mg). LC-MS: $[M+H]^+$ 557.05.

Step 2:

**022299A2** (71 mg, 0.128 mmol, 1 eq) and isopropanol (2 mL) were added to a 2 M hydrochloric acid solution (0.3 mL) at room temperature, and under argon atmosphere, the mixture was heated to 50 °C in an oil bath and stirred for 3 h. The reaction was completed as indicated by LC-MS, thus giving a product. The mixture was concentrated under reduced pressure to remove the solvent. Water (30 mL × 1) and a saturated sodium bicarbonate solution (5 mL × 1) were then added, and the mixture was extracted with ethyl acetate (20 mL × 3). The organic phases were combined, washed with saturated brine (20 mL × 1), dried over anhydrous sodium sulfate, and filtered. The filtrate was concentrated under reduced pressure to give a yellow oil (73 mg), which was then purified by preparative high-pressure reversed-phase chromatography to give **SZ-022299** as a yellow-green solid (25 mg). LC-MS: $[M+H]^+$ 494.99, $^1$H NMR (400 MHz, DMSO-$d_6$) δ 8.96 (s, 1H), 8.83 (d, *J* = 7.1 Hz, 1H), 7.65 (t, *J* = 7.5 Hz, 1H), 7.52 (t, *J* = 6.8 Hz, 1H), 7.32 (t, *J* = 7.6 Hz, 1H), 7.23 (t, *J* = 54.0 Hz, 1H), 6.33 (t, *J* = 56.8 Hz, 1H), 5.79-5.71 (m, 1H), 4.39-4.33 (m, 1H), 2.21 (s, 3H), 1.58 (d, *J* = 6.8 Hz, 3H), 1.53 - 1.29 (m, 4H), 0.81 - 0.72 (m, 2H), 0.41-0.36 (m, 2H).

**Example 39: SZ-022348**

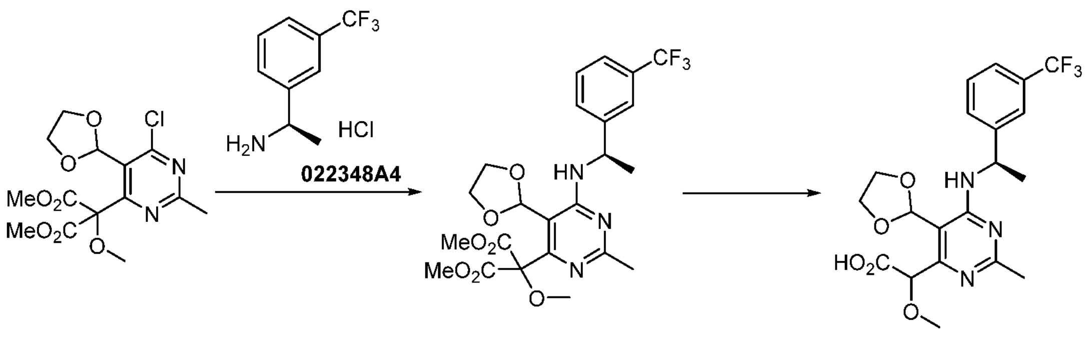


SZ-022300A1 022348A5 022348A6

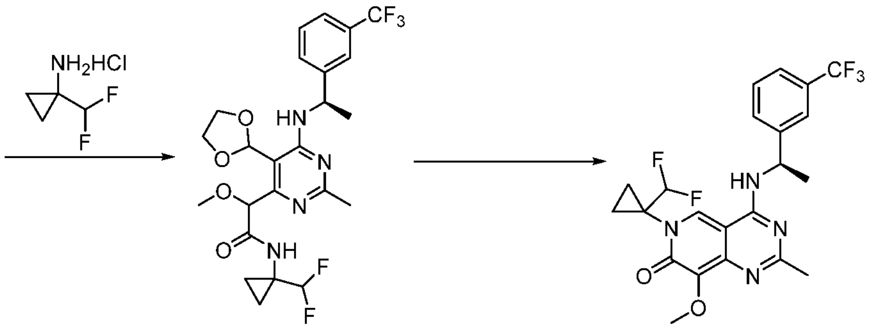

022348A7 SZ-022348

Step 1:

**SZ-022300A1** (100 mg, 0.27 mmol), **022348A4** (75 mg, 0.33 mmol), and triethylamine (140 mg, 1.39 mmol) were added to tetrahydrofuran (5 mL), and the mixture was heated to 80 °C for reaction for 16 h. After the starting materials were consumed as monitored by LC-MS, water (20 mL) and ethyl acetate (50 mL) were added to the reaction mixture for extraction, and the organic layer was separated, washed with a saturated sodium chloride solution, dried, and concentrated to dryness under reduced pressure to give a crude product. The crude product was purified by flash silica gel column chromatography (PE:EA = 1:1) to give **022348A5** as a light yellow oil (90 mg). LC-MS: $[M+H]^+$ 514.17.

Step 2:

**022348A5** (90 mg, 0.17 mmol) was added to dimethyl sulfoxide (4 mL), and then a 20% sodium hydroxide solution (0.5 mL) was added. The mixture was allowed to react at room temperature for 1 h. After the starting materials were consumed as monitored by LC-MS, the reaction mixture was used directly in the next step without work-up. LC-MS: $[M+H]^+$ 442.14.

Step 3:

(Difluoromethyl)cyclopropyl-1-amine hydrochloride (36 mg, 0.25 mmol), HATU (194 mg, 0.51 mmol), and triethylamine (86 mg, 0.85 mmol) were added to the reaction system in the previous step, and the mixture was allowed to react at room temperature for another 16 h. After the starting materials were consumed as monitored by LC-MS, water (20 mL) and ethyl acetate (50 mL) were added to the reaction mixture for extraction, and the organic layer was separated, washed with a saturated sodium chloride solution, dried, and concentrated to dryness under reduced pressure to give crude product **022348A7** as a light yellow oil (90 mg). LC-MS: $[M+H]^+$ 531.26.

Step 4:

**022348A7** (90 mg, 0.17 mmol) was added to isopropanol (3 mL), and then a hydrochloric acid solution (2 M, 0.52 mL) was added. The mixture was allowed to react at 50 °C for 4 h. After the starting materials were consumed as monitored by LC-MS, the reaction mixture was directly concentrated to dryness under reduced pressure. A saturated sodium bicarbonate solution (5 mL), water (10 mL), and ethyl acetate (20 mL) were then added for extraction, and the organic layer was separated, washed with a saturated sodium chloride solution, dried, and concentrated to dryness

under reduced pressure to give a crude product. The crude product was separated by preparative high performance liquid chromatography and then lyophilized to give **SZ-022348** as a yellow solid (30 mg). LC-MS: $[M+H]^+$ 469.14.

**SZ-022348:** $^1H$ NMR (400 MHz, DMSO-$d_6$) δ 8.88 (s, 1H), 8.80 (d, *J* = 7.6 Hz, 1H), 7.77 (s, 1H), 7.72 - 7.70 (m, 1H), 7.62 - 7.56 (m, 2H), 6.33 (t, *J* = 56.8 Hz, 1H), 5.68 - 5.61 (m, 1H), 3.77 (s, 3H), 2.25 (s, 3H), 1.59 (d, *J*= 7.2 Hz, 3H), 1.49 - 1.47 (m, 2H), 1.42 - 1.33 (m, 2H).

**Example 40: SZ-022351**

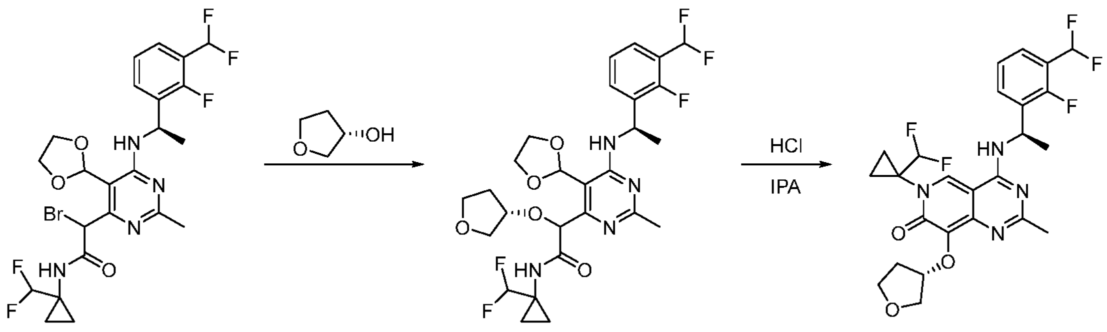


**022299A1** **022351A1** **SZ-022351**

Step 1:

60% NaH (55 mg, 1.381 mmol, 10 eq) was added to a three-necked flask, and under argon atmosphere, anhydrous tetrahydrofuran (5 mL) was added via a syringe. (*S*)-3-Hydroxytetrahydrofuran (134 mg, 1.519 mmol, 11 eq) was then added dropwise at room temperature, and the mixture was stirred at room temperature for 30 min. The reaction mixture was then stirred in an ice-water bath, and a solution of **022299A1** (80 mg, 0.138 mmol, 1 eq) and dry tetrahydrofuran (1 mL) was added. The resulting mixture was naturally warmed and stirred for 2.5 h, and then the reaction was substantially completed as indicated by LC-MS. A saturated ammonium chloride solution (10 mL × 1) and water (20 mL × 1) were added to the reaction mixture, and the mixture was extracted with ethyl acetate (20 mL × 3). The organic phases were combined, washed with saturated brine (20 mL × 1), dried over anhydrous sodium sulfate, and filtered. The filtrate was concentrated under reduced pressure, subjected to column chromatography with a 25 g silica gel column, and subjected to gradient elution with 0-100% ethyl acetate/petroleum ether. The fractions were collected and concentrated under reduced pressure to give **022351A1** as a colorless oil (51 mg), LC-MS: $[M+H]^+$ 587.02.

Step 2:

**022351A1** (51 mg, 0.087 mmol, 1 eq) and isopropanol (2 mL) were added to a 2 M hydrochloric acid solution (0.3 mL) at room temperature, and under argon atmosphere, the mixture was heated to 50 °C in an oil bath and stirred for 3 h. The reaction was completed as indicated by LC-MS, thus giving a product. The mixture was concentrated under reduced pressure to remove the solvent. Water (30 mL × 1) and a saturated sodium bicarbonate solution (5 mL × 1) were then added, and the mixture was extracted with ethyl acetate (20 mL × 3). The organic phases were combined, washed with saturated brine (20 mL × 1), dried over anhydrous sodium sulfate, and filtered. The filtrate was concentrated under reduced pressure to give a yellow oil (51 mg), which was then purified by preparative high-pressure reversed-phase chromatography to give **SZ-022351** as a yellow-green solid (21 mg). LC-MS: $[M+H]^+$ 524.95, $^1H$ NMR (400 MHz, DMSO-$d_6$) δ 8.95 (s, 1H), 8.82 (d, *J* = 7.2 Hz, 1H), 7.65 (t, *J*= 7.2 Hz, 1H), 7.52 (t, *J* = 7.2 Hz, 1H), 7.32 (t, *J* = 8.0 Hz, 1H), 7.23(t, *J* = 54.4 Hz, 1H), 6.34 (t, *J* = 56.8 Hz, 1H), 5.79-5.72 (m, 1H), 5.16 - 5.14 (m, 1H), 4.00 - 3.85 (m, 2H), 3.78 - 3.66 (m, 2H), 2.21 (s, 3H), 2.03 - 1.97 (m, 1H), 1.75-1.85 (m, 1H), 1.58 (d, *J*= 7.2 Hz, 3H), 1.53 - 1.30 (m, 4H).

**Example 41: SZ-022352**

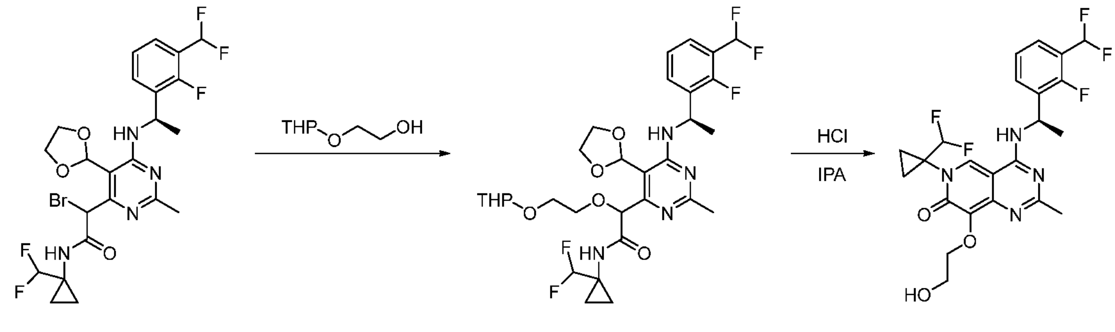


**022299A1** **022352A1** **SZ-022352**

Step 1:

60% NaH (55 mg, 1.381 mmol, 10 eq) was added to a three-necked flask, and under argon atmosphere, anhydrous tetrahydrofuran (5 mL) was added via a syringe. 2-(Tetrahydro-2*H*-pyran-2-oxy)ethanol was then added dropwise at room temperature, and the mixture was stirred at room temperature for 50 min. The reaction mixture was then stirred in an ice-water bath, and a solution of **022299A1** (80 mg, 0.138 mmol, 1 eq) and dry tetrahydrofuran (1 mL) was added. The resulting mixture was naturally warmed and stirred for 1 h, and then the reaction was substantially completed as indicated by LC-MS. A saturated ammonium chloride solution (10 mL × 1) and water (20 mL × 1) were added to the reaction mixture, and the mixture was extracted with ethyl acetate (20 mL × 3). The organic phases were combined, washed with saturated brine (20 mL × 1), dried over anhydrous sodium sulfate, and filtered. The filtrate was concentrated under reduced pressure, subjected to column chromatography with a 25 g silica gel column, and subjected to gradient elution with 0-100% ethyl acetate/petroleum ether. The fractions were collected and concentrated under reduced pressure to give **022352A1** as a colorless oil (87 mg), LC-MS: $[M+H]^+$ 644.98.

Step 2:

**022352A1** (87 mg, 0.135 mmol, 1 eq) and isopropanol (2 mL) were added to a 2 M hydrochloric acid solution (0.5 mL) at room temperature, and under argon atmosphere, the mixture was heated to 50 °C in an oil bath and stirred for 3 h. The reaction was completed as indicated by LC-MS, thus giving a product. The mixture was concentrated under reduced pressure to remove the solvent, and water (30 mL × 1) was added. The mixture was adjusted to about pH 7-8 by dropwise addition of a saturated sodium bicarbonate solution and extracted with ethyl acetate (20 mL × 3). The organic phases were combined, washed with saturated brine (20 mL × 1), dried over anhydrous sodium sulfate, and filtered. The filtrate was concentrated under reduced pressure to give a yellow oil (80 mg), which was then purified by preparative high-pressure reversed-phase chromatography to give **SZ-022352** as a yellow-green solid (37 mg). LC-MS: $[M+H]^+$ 498.99, $^1H$ NMR (400 MHz, DMSO-$d_6$) δ 9.02 - 8.94 (m, 2H), 7.65 (t, *J*= 7.6 Hz, 1H), 7.53 (t, *J* = 7.2 Hz, 1H), 7.33 (t, *J* = 7.6 Hz, 1H), 7.23 (t, *J*= 54.0 Hz, 1H), 6.33 (t, *J* = 56.8 Hz, 1H), 5.80-5.73 (m, 1H), 5.55 (br s, 1H), 4.06 - 3.98 (m, 2H), 3.48 (t, *J* = 5.2 Hz, 2H), 2.22 (s, 3H), 1.59 (d, *J* = 7.2 Hz, 3H), 1.54 - 1.32 (m, 4H).

**Example 42: SZ-022353**

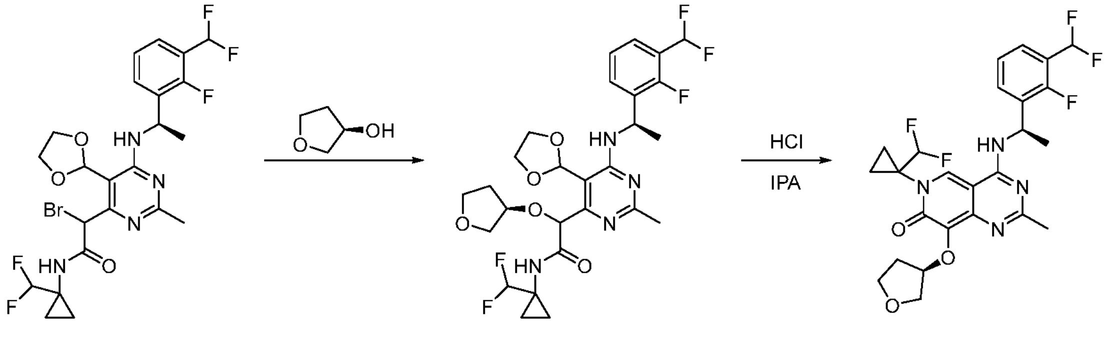


**022299A1** **022353A1** **SZ-022353**

By referring to **Example SZ-022351, SZ-022353** as a yellow-green solid (20 mg) was obtained. LC-MS: [M+H]$^+$ 525.00, $^1$H NMR (400 MHz, DMSO-$d_6$) δ 8.94 (s, 1H), 8.82 (d, *J* = 6.8 Hz, 1H), 7.65 (t, *J* = 7.6 Hz, 1H), 7.52 (t, *J* = 7.6 Hz, 1H), 7.32 (t, *J* = 7.6 Hz, 1H), 7.23(t, *J* = 54.4 Hz, 1H), 6.34 (t, *J* = 56.8 Hz, 1H), 5.79-5.72 (m, 1H), 5.18 - 5.11 (m, 1H), 4.01 - 3.85 (m, 2H), 3.79 - 3.67 (m, 2H), 2.21 (s, 3H), 2.06 - 1.97 (m, 1H), 1.87 - 1.74 (m, 1H), 1.58 (d, *J* = 6.8 Hz, 3H), 1.54 - 1.31 (m, 4H).

**Example 43: SZ-022354**

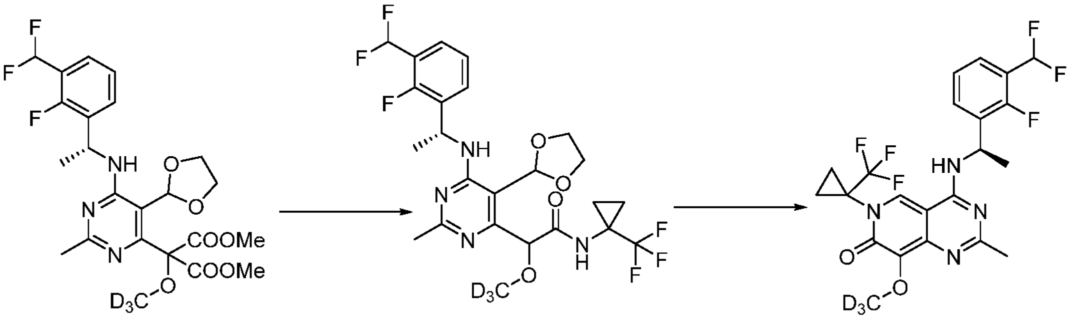

**SZ-022295A1** **SZ-022354A1** **SZ-022354**

Step 1:

**SZ-022295A1** (280 mg, 0.54 mmol) was added to dimethyl sulfoxide (5 mL), and then a 10% sodium hydroxide solution (870 μL) was added. The mixture was allowed to react at room temperature for 1 h. 1-Trifluoromethylcyclopropanamine hydrochloride (132 mg, 0.81 mmol), *N*,*N*,*N*',*N*'-tetramethyl-*O*-(7-azabenzotriazol-1-yl)uronium hexafluorophosphate (619 mg, 1.63 mmol), and triethylamine (165 mg, 1.63 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022354A1** as a yellow viscous substance (280 mg). LC-MS: [M+H]$^+$ 551.99.

Step 2:

**SZ-022354A1** (280 mg, 0.51 mmol) was added to isopropanol (5 mL), and then a hydrochloric acid solution (4 M, 0.64 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022354** (110 mg). LC-MS: [M+H]$^+$ 490.16.

**SZ-022354:** $^1$H NMR (400 MHz, DMSO-$d_6$) δ 9.00 (s, 1H), 8.83 (d, *J*= 7.2 Hz, 1H), 7.63 (t, *J* = 7.6 Hz, 1H), 7.53 (t, *J* = 7.2 Hz, 1H), 7.33 (d, *J* = 8.0 Hz, 1H), 7.24 (t, *J* = 54.4 Hz, 1H), 5.80 - 5.67 (m, 1H), 2.21 (s, 3H), 1.87 - 1.49(m, 4H), 1.59 (d, *J*= 7.2 Hz, 3H).

**Example 44: SZ-022373**

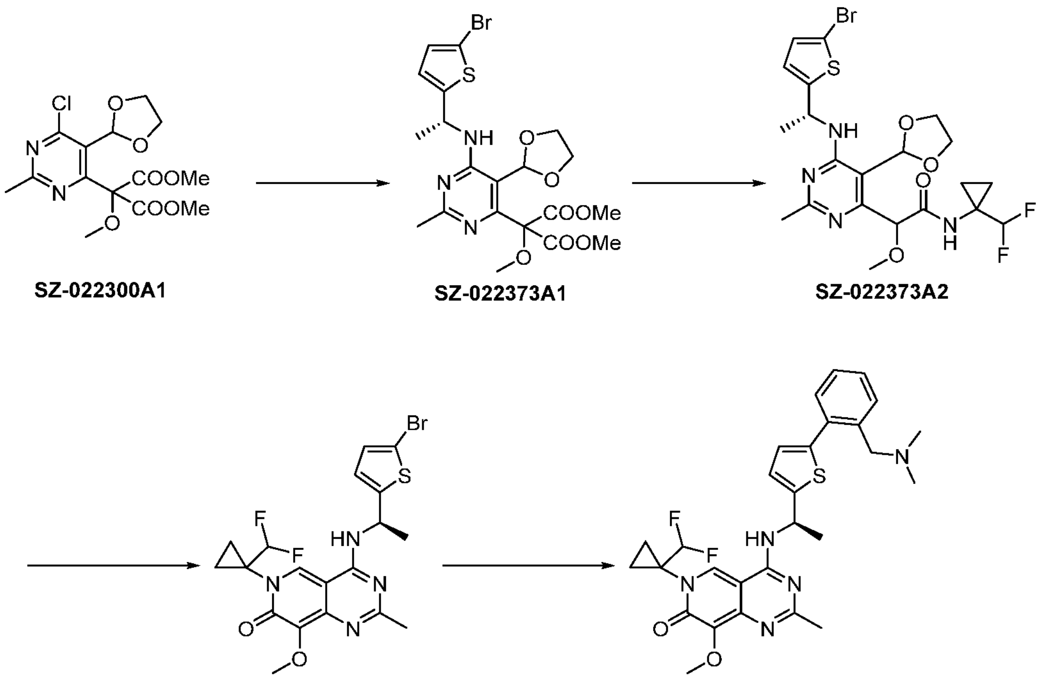

SZ-022300A1 SZ-022373A1 SZ-022373A2

SZ-022373A3 SZ-022373

Step 1:

Compound **SZ-022300A1** (500 mg, 1.39 mmol), (1*R*)-1-(5-bromothiophen-2-yl)ethylamine hydrochloride (405 mg, 1.67 mmol, synthesized according to WO2019122129), and *N*,*N*-diisopropylethylamine (900 mg, 6.94 mmol) were allowed to react at 85 °C overnight. After the starting materials were consumed as monitored by LC-MS, the reaction mixture was cooled to room temperature. Water (50 mL) and ethyl acetate (50 mL) were added to the reaction mixture for extraction, and the organic layer was separated, dried, and concentrated by rotary evaporation to give a crude product. The crude product was purified by flash silica gel column chromatography (PE:EA = 3:1; Rf = 0.2) to give product **SZ-022373A1** as a colorless oil (525 mg). LC-MS: $[M+H]^+$ 529.67, 531.64.

Step 2:

**SZ-022373A1** (525 mg, 1 mmol) was added to dimethyl sulfoxide (5 mL), and then a 10% sodium hydroxide solution (1.6 mL) was added. The mixture was allowed to react at room temperature for 1 h. 1-Difluoromethylcyclopropanamine hydrochloride (215 mg, 1.5 mmol), *N*,*N*,*N*',*N*'-tetramethyl-*O*-(7-azabenzotriazol-1-yl)uronium hexafluorophosphate (1.14 g, 3 mmol), and triethylamine (303 mg, 3 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022373A2** as a yellow viscous substance (520 mg). LC-MS: $[M+H]^+$ 546.70, 548.63.

Step 3:

**SZ-022373A2** (520 mg, 0.95 mmol) was added to isopropanol (5 mL), and then a hydrochloric acid solution (4 M, 1.2 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to medium-pressure reversed-phase column chromatography (acetonitrile:water = 1:1) and then lyophilized to give **SZ-022373A3** (350 mg). LC-MS: $[M+H]^+$ 485.07, 487.02.

Step 4:

**SZ-022373A3** (100 mg, 0.21 mmol), 2-(*N*,*N*-dimethylamino)phenylboronic acid (50 mg, 0.25 mmol), tetrakis(triphenylphosphine)palladium (30 mg, 0.021 mmol), and sodium carbonate (45 mg, 0.42 mmol) were added to 1,4-dioxane (2 mL) and water (0.4 mL), and the mixture was purged with argon for 1 min and then microwaved at 125 °C for reaction for 1.5 h. After the starting materials were consumed as monitored by LC-MS, the reaction mixture was directly concentrated by rotary evaporation. Water (10 mL) and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022373** (45 mg). LC-MS: $[M+H]^+$ 539.92.

**SZ-022373:** $^1$H NMR (400 MHz, DMSO-$d_6$) δ 8.90 (d, *J*= 8.1 Hz, 1H), 8.84 (s, 1H), 7.46 - 7.28 (m, 4H), 7.19 (d, *J* = 3.6 Hz, 1H), 7.10 (d, *J* = 3.6 Hz, 1H), 6.32 (t, *J*= 57.2 Hz, 1H), 5.96 - 5.88 (m, 1H), 3.80 (s, 3H), 3.37 (s, 2H), 2.35 (s, 3H), 2.12 (s, 6H), 1.69 (d, *J*= 6.8 Hz, 3H), 1.46 - 1.35 (m, 4H).

**Example 45: SZ-022356**

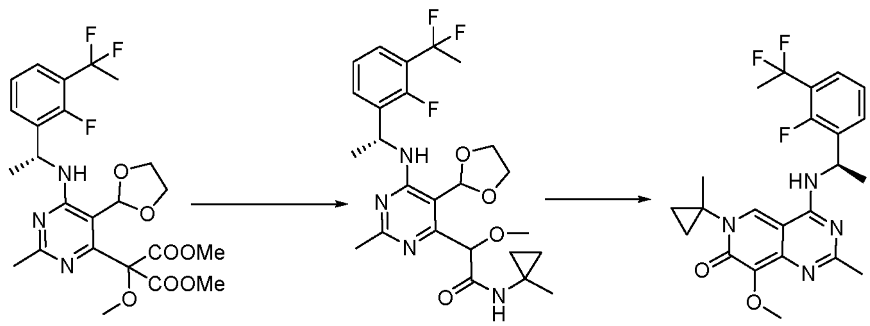

**SZ-022303A1** **SZ-022356A1** **SZ-022356**

Step 1:

**SZ-022303A1** (220 mg, 0.42 mmol) was added to dimethyl sulfoxide (4 mL), and then a 10% sodium hydroxide solution (670 μL) was added. The mixture was allowed to react at room temperature for 1 h. 1-Methylcyclopropanamine hydrochloride (67 mg, 0.63 mmol), *N,N,N',N'*-tetramethyl-*O*-(7-azabenzotriazol-1-yl)uronium hexafluorophosphate (476 mg, 1.25 mmol), and triethylamine (127 mg, 1.25 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022356A1** as a light yellow viscous substance (220 mg). LC-MS: $[M+H]^+$ 509.26.

Step 2:

**SZ-022356A1** (220 mg, 0.43 mmol) was added to isopropanol (5 mL), and then a hydrochloric acid solution (4 M, 0.55 mL) was added. The mixture was allowed to react at 50 °C for 2 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022356** (74 mg). LC-MS: $[M+H]^+$ 447.16.

**SZ-022356:** $^1$H NMR (400 MHz, DMSO-$d_6$) δ 8.99 (s, 1H), 8.69 (d, *J* = 7.2 Hz, 1H), 7.61 (t, *J* = 6.8 Hz, 1H), 7.45 (t, *J* = 6.8 Hz, 1H), 7.27 (t, *J* = 7.6 Hz, 1H), 5.77 - 5.70 (m, 1H), 3.75 (s, 3H), 2.20 (s, 3H), 2.02 (t, *J*= 19.2 Hz, 3H), 1.57 (d, *J*= 7.2 Hz, 3H), 1.52 (s, 3H), 1.17 - 1.08 (m, 2H), 1.08 - 1.00 (m, 2H).

**Example 46: SZ-022357**

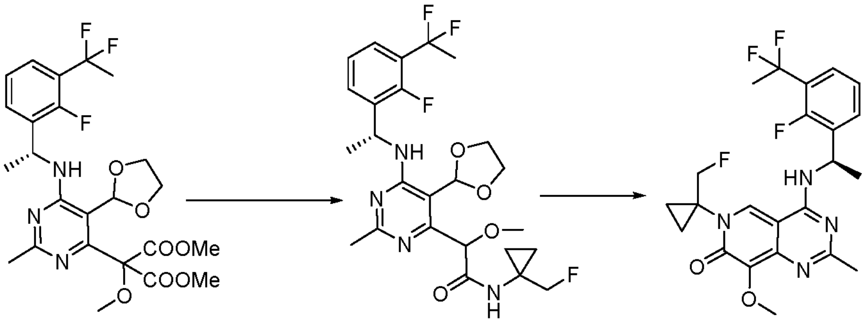

SZ-022303A1 SZ-022357A1 SZ-022357

Step 1:

**SZ-022303A1** (220 mg, 0.41 mmol) was added to dimethyl sulfoxide (4 mL), and then a 10% sodium hydroxide solution (670 μL) was added. The mixture was allowed to react at room temperature for 1 h. 1-(Difluoromethyl)cyclopropanamine hydrochloride (79 mg, 0.63 mmol), *N*,*N*,*N'*,*N'*-tetramethyl-*O*-(7-azabenzotriazol-1-yl)uronium hexafluorophosphate (476 mg, 1.25 mmol), and triethylamine (127 mg, 1.25 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022357A1** as a light yellow viscous substance (220 mg). LC-MS: $[M+H]^+$ 527.08.

Step 2:

**SZ-022357A1** (220 mg, 0.41 mmol) was added to isopropanol (5 mL), and then a hydrochloric acid solution (4 M, 0.52 mL) was added. The mixture was allowed to react at 50 °C for 2 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022357** (59 mg). LC-MS: $[M+H]^+$ 465.18.

**SZ-022357:$^1$H** NMR (400 MHz, DMSO-$d_6$) δ 8.95 (s, 1H), 8.81 (d, *J* = 7.2 Hz, 1H), 7.59 (t, *J*= 6.8 Hz, 1H), 7.45 (t, *J*= 7.6 Hz, 1H), 7.27 (t, *J*= 7.6 Hz, 1H), 5.78 - 5.71 (m, 1H), 4.79 - 4.49 (m, 2H), 3.76 (s, 3H), 2.21 (s, 3H), 2.02 (t, *J*= 19.2 Hz, 3H), 1.57 (d, *J* = 7.2 Hz, 3H), 1.38 - 1.25 (m, 4H).

**Example 47: SZ-022358**

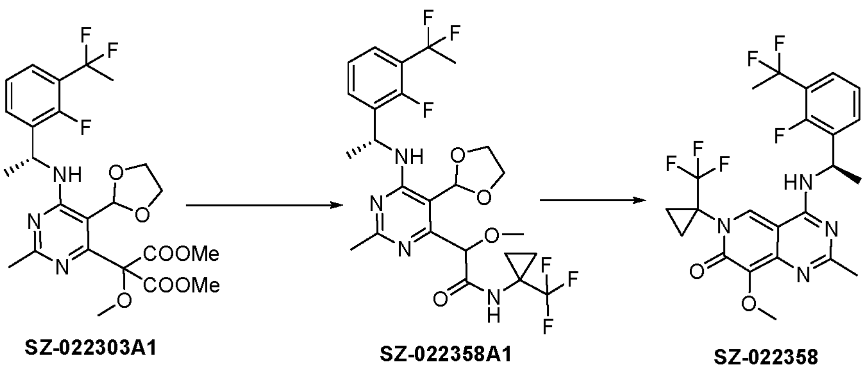

SZ-022303A1 SZ-022358A1 SZ-022358

Step 1:

**SZ-022303A1** (220 mg, 0.41 mmol) was added to dimethyl sulfoxide (2 mL), and then a 10% sodium hydroxide solution (670 μL) was added. The mixture was allowed to react at room temperature for 1 h. 1-(Trifluoromethyl)cyclopropanamine hydrochloride (101 mg, 0.62 mmol), *N*,*N*,*N*',*N*'-tetramethyl-*O*-(7-azabenzotriazol-1-yl)uronium hexafluorophosphate (476 mg, 1.25 mmol), and triethylamine (127 mg, 1.25 mmol) were then added into the system, and the mixture was allowed to react at room temperature for another 3 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022358A1** as a light yellow viscous substance (220 mg). LC-MS: $[M+H]^+$ 563.01.

Step 2:

**SZ-022358A1** (220 mg, 0.39 mmol) was added to isopropanol (5 mL), and then a hydrochloric acid solution (4 M, 0.49 mL) was added. The mixture was allowed to react at 50 °C for 2 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022358** (61 mg). LC-MS: $[M+H]^+$ 501.16.

**SZ-022358:$^1$H** NMR (400 MHz, DMSO-$d_6$) δ 9.00 (s, 1H), 8.83 (d, *J* = 6.8 Hz, 1H), 7.59 (t, *J* = 7.2 Hz, 1H), 7.45 (t, *J* = 7.2 Hz, 1H), 7.28 (d, *J* = 7.6 Hz, 1H), 5.80 - 5.69 (m, 1H), 3.76 (s, 3H), 2.21 (s, 3H), 2.02 (t, *J*= 19.2 Hz, 3H), 1.88 - 1.52 (m, 4H), 1.58 (d, *J*= 7.2 Hz, 3H).

**Example 48: SZ-022341**

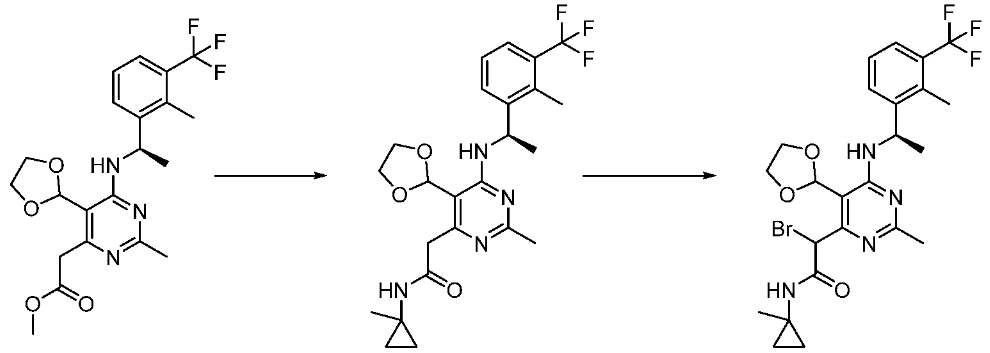

SZ-022339A2 SZ-022341A1 SZ-022341A2

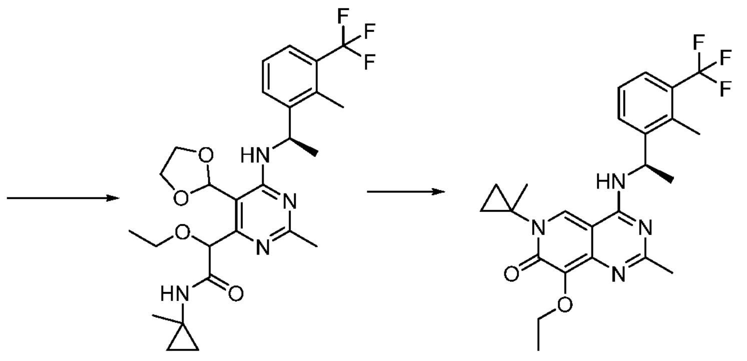

SZ-022341A3 SZ-022341

Step 1:

**SZ-022339A2** (500 mg, 1.14 mmol) and sodium hydroxide (182 mg, 4.56 mmol) were added to dimethyl sulfoxide (5 mL), and the mixture was allowed to react at room temperature for 1 h. After the starting materials were consumed as monitored by LC-MS, triethylamine (346 mg, 3.42 mmol), 1-(methylcyclopropyl)amine hydrochloride (184 mg, 1.71

mmol), and *N*,*N*,*N*',*N*'-tetramethyl-*O*-(7-azabenzotriazol-1-yl)uronium hexafluorophosphate (1300 mg, 3.42 mmol) were added to the reaction mixture, and the resulting mixture was allowed to react at room temperature for 2 h. Water (50 mL) and ethyl acetate (50 mL) were added to the reaction mixture for extraction, and the organic layer was separated, washed with a saturated sodium chloride solution, dried, and concentrated by rotary evaporation to give a crude product. The crude product was purified by flash silica gel column chromatography (DCM:MeOH = 10:1; Rf = 0.5) to give **SZ-022341A1** as a yellow solid (490 mg). LC-MS: $[M+H]^+$ 479.24.

Step 2:

**SZ-022341A1** (490 mg, 1.03 mmol), *N*-bromosuccinimide (183 mg, 1.03 mmol), and azobisisobutyronitrile (17 mg, 0.103 mmol) were added to carbon tetrachloride (15 mL), and the mixture was heated to 60 °C for reaction for 1 h. After the starting materials were consumed as monitored by LC-MS, the reaction mixture was concentrated by rotary evaporation to give a crude product. The crude product was purified by flash silica gel column chromatography (PE:EA = 3:7; Rf = 0.5) to give **SZ-022341A2** as a yellow solid (350 mg). LC-MS: $[M+H]^+$ 556.76, 558.70.

Step 3:

**SZ-022341A2** (250 mg, 0.45 mmol) was dissolved in ethanol (25 mL), and a solution of sodium ethoxide in ethanol (0.8 mL, 20% wt) was slowly added dropwise to the above system. The mixture was stirred at room temperature for 2 h. After the starting materials were consumed as monitored by LC-MS, the reaction mixture was concentrated. Water (50 mL) and ethyl acetate (50 mL) were then added for extraction, and the organic layer was separated, washed with a saturated sodium chloride solution, dried, and concentrated by rotary evaporation to give **SZ-022341A3** as a yellow solid (140 mg). LC-MS: $[M+H]^+$ 522.96.

Step 4:

**SZ-022341A3** (140 mg, 0.27 mmol) and hydrochloric acid (0.15 mL, 5 M) were added to isopropanol (2 mL), and the mixture was heated to 50 °C for reaction for 6 h. After the starting materials were consumed as monitored by LC-MS, the reaction mixture was concentrated by rotary evaporation to give a crude product. The crude product was subjected to preparative high performance liquid chromatography and then lyophilized to give **SZ-022341** (35.43 mg). LC-MS: $[M+H]^+$ 460.89.

**SZ-022341:** $^1H$ NMR (400 MHz, DMSO-$d_6$) δ 8.95 (s, 1H), 8.75 (d, *J* = 6.8 Hz, 1H), 7.73 (d, *J* = 8.0 Hz, 1H), 7.56 (d, *J* = 8.0 Hz, 1H), 7.38 (t, *J* = 8.0 Hz, 1H), 5.71 - 5.64 (m, 1H), 4.04 (q, *J* = 7.2 Hz, 2H), 2.57 (s, 3H), 2.19 (s, 3H), 1.54 - 1.51 (m, 6H), 1.20 (t, *J* = 7.2 Hz, 3H), 1.15 - 1.01 (m, 4H).

**Example 49: SZ-022342**

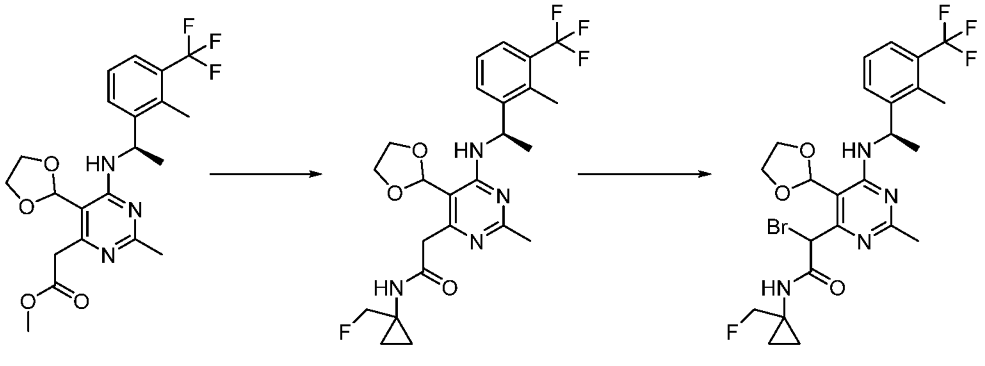

**SZ-022339A2** **SZ-022342A1** **SZ-022342A2**

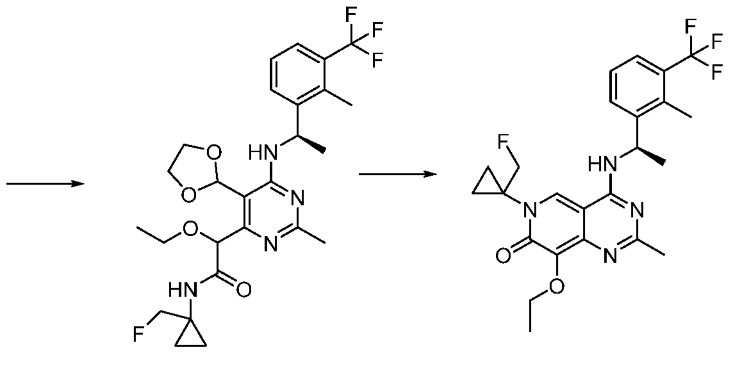

**SZ-022342A3** **SZ-022342**

Step 1:

**SZ-022339A2** (500 mg, 1.14 mmol) and sodium hydroxide (182 mg, 4.56 mmol) were added to dimethyl sulfoxide (5 mL), and the mixture was allowed to react at room temperature for 1 h. After the starting materials were consumed as monitored by LC-MS, triethylamine (346 mg, 3.42 mmol), 1-(monofluoromethylcyclopropyl)amine hydrochloride (216 mg, 1.71 mmol), and *N*,*N*,*N*',*N*'-tetramethyl-*O*-(7-azabenzotnazol-1-yl)uronium hexafluorophosphate (1300 mg, 3.42 mmol) were added to the reaction mixture, and the resulting mixture was allowed to react at room temperature for 2 h. Water (50 mL) and ethyl acetate (50 mL) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA = 0:1; Rf = 0.5) to give **SZ-022342A1** as a white solid (490 mg). LC-MS: $[M+H]^+$ 496.98.

Step 2:

**SZ-022342A1** (490 mg, 0.99 mmol), *N*-bromosuccinimide (176 mg, 0.99 mmol), and azobisisobutyronitrile (16 mg, 0.099 mmol) were added to carbon tetrachloride (15 mL), and the mixture was heated to 60 °C for reaction for 1 h. After the starting materials were consumed as monitored by LC-MS, the reaction mixture was concentrated by rotary evaporation to give a crude product. The crude product was purified by flash silica gel column chromatography (PE:EA = 1:1; Rf = 0.5) to give **SZ-022342A2** as a white solid (320 mg). LC-MS: $[M+H]^+$ 574.87, 576.77.

Step 3:

**SZ-022342A2** (320 mg, 0.56 mmol) was dissolved in ethanol (5 mL), and a solution of sodium ethoxide in ethanol (1.1 mL, 20% wt) was slowly added dropwise to the above system. The mixture was stirred at room temperature for 2 h. After the starting materials were consumed as monitored by LC-MS, the reaction mixture was concentrated. Water (50 mL) and ethyl acetate (50 mL) were then added for extraction, and the organic layer was separated, washed with a saturated sodium chloride solution, dried, and concentrated by rotary evaporation to give **SZ-022342A3** as a yellow solid (270 mg). LC-MS: $[M+H]^+$ 541.00.

Step 4:

**SZ-022342A3** (270 mg, 0.50 mmol) and hydrochloric acid (0.3 mL, 5 M) were added to isopropanol (5 mL), and the mixture was heated to 50 °C for reaction for 3 h. After the starting materials were consumed as monitored by LC-MS, water (50 mL) and ethyl acetate (50 mL) were added to the reaction mixture for extraction, and the organic layer was separated, washed with a saturated sodium chloride solution, dried, and concentrated by rotary evaporation to give a crude product. The crude product was subjected to preparative high performance liquid chromatography and then lyophilized to give **SZ-022342** (33.99 mg). LC-MS: $[M+H]^+$ 479.19.

**SZ-022342:** $^1H$ NMR (400 MHz, DMSO-$d_6$) δ 8.91 (s, 1H), 8.85 (d, *J* = 6.8 Hz, 1H), 7.71 (d, *J* = 8.0 Hz, 1H), 7.56 (d, *J* = 8.0 Hz, 1H), 7.39 (t, *J* = 8.0 Hz, 1H), 5.72 - 5.67 (m, 1H), 4.73 - 4.54 (m, 2H), 4.06 (q, *J* = 7.2 Hz, 2H), 2.57 (s, 3H), 2.20 (s, 3H), 1.53 (d, *J*= 7.2 Hz, 3H), 1.33 - 1.30 (m, 4H), 1.21 (t, *J* = 7.2 Hz, 3H).

**Example 50: SZ-022371**

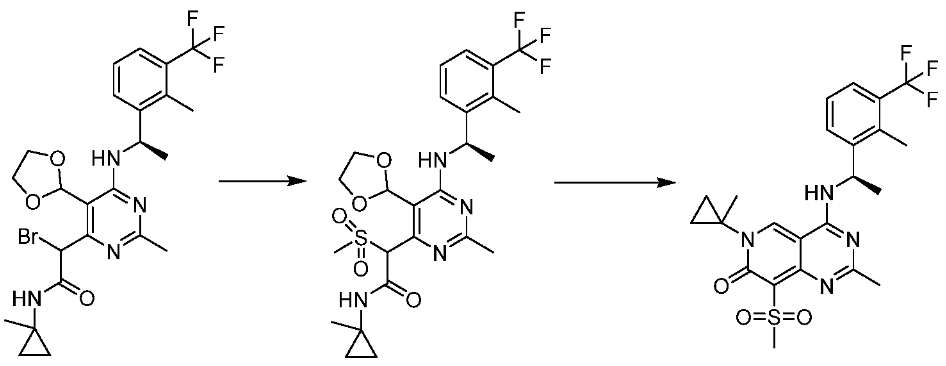

SZ-022341A2 SZ-022371A1 SZ-022371

Step 1:

**SZ-022341A2** (100 mg, 0.18 mmol) and sodium methanesulfinate (22 mg, 0.22 mmol) were dissolved in dimethyl sulfoxide (10 mL), and the solution was stirred at room temperature for 2 h. After the starting materials were consumed as monitored by LC-MS, water (100 mL) and ethyl acetate (50 mL) were added to the reaction mixture for extraction, and the organic layer was separated, washed with a saturated sodium chloride solution, dried, and concentrated by rotary evaporation to give **SZ-022371A1** as a yellow solid (80 mg). LC-MS: $[M+H]^+$ 557.26.

Step 2:

**SZ-022371A1** (80 mg, 0.14 mmol) and hydrochloric acid (0.1 mL, 5 M) were added to isopropanol (2 mL), and the mixture was heated to 50 °C for reaction for 3 h. After the starting materials were consumed as monitored by LC-MS, the reaction mixture was concentrated by rotary evaporation to give a crude product. The crude product was subjected to preparative high performance liquid chromatography and then lyophilized to give **SZ-022371** (21.51 mg). LC-MS: $[M+H]^+$ 494.84.

**SZ-022371:** $^1H$ NMR (400 MHz, DMSO-$d_6$) δ 9.50 (s, 1H), 9.10 (d, *J* = 6.8 Hz, 1H), 7.74 (d, *J* =8.0 Hz, 1H), 7.57 (d, *J* =8.0 Hz, 1H), 7.39 (t, *J* = 8.0 Hz, 1H), 5.73 - 5.66 (m, 1H), 3.31 (s, 3H), 2.56 (s, 3H), 2.25 (s, 3H), 1.56 - 1.54 (m, 6H), 1.17 - 1.05 (m, 4H).

**Example 51: SZ-022372**

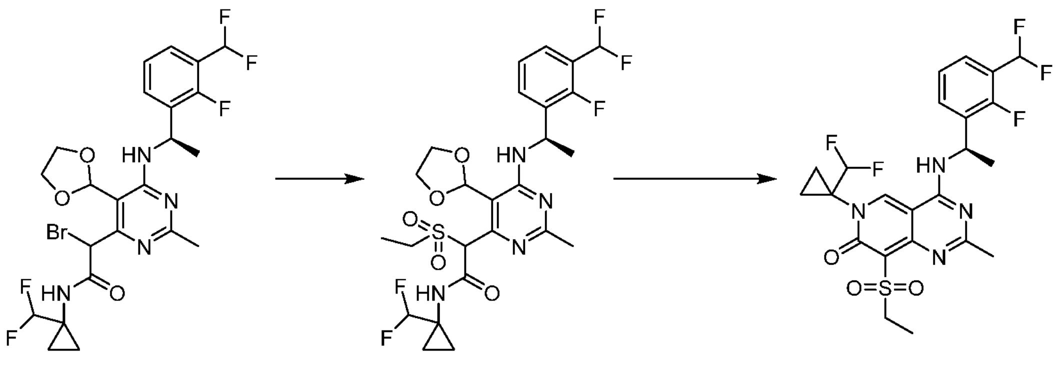

SZ-022307A1 SZ-022372A1 SZ-022372

Step 1:

**SZ-022307A1** (90 mg, 0.16 mmol) and sodium ethanesulfinate (22 mg, 0.19 mmol) were dissolved in dimethyl sulfoxide (10 mL), and the solution was stirred at room temperature for 2 h. After the starting materials were consumed as monitored by LC-MS, water (100 mL) and ethyl acetate (50 mL) were added to the reaction mixture for extraction, and the organic layer was separated, washed with a saturated sodium chloride solution, dried, and concentrated by rotary evaporation to give **SZ-022372A1** as a yellow solid (145 mg). LC-MS: $[M+H]^+$ 592.89.

Step 2:

**SZ-022372A1** (145 mg, 0.24 mmol) and hydrochloric acid (0.15 mL, 5 M) were added to isopropanol (3 mL), and the mixture was heated to 50 °C for reaction for 3 h. After the starting materials were consumed as monitored by LC-MS, the reaction mixture was concentrated by rotary evaporation to give a crude product. The crude product was subjected to preparative high performance liquid chromatography and then lyophilized to give **SZ-022372** (21.95 mg). LC-MS: $[M+H]^+$ 530.86.

**SZ-022372:** $^1$H NMR (400 MHz, DMSO-$d_6$) δ 9.47 (s, 1H), 9.20 (d, *J*= 6.8 Hz, 1H), 7.67 (t, *J* = 7.2 Hz, 1H), 7.54 (t, *J* = 7.2 Hz, 1H), 7.34 (t, *J* = 7.6 Hz, 1H), 7.24 (t, *J* = 56.0 Hz, 1H), 6.36 (t, *J* = 56.8 Hz, 1H), 5.80 - 5.76 (m, 1H), 3.54 (q, *J*= 7.4 Hz, 2H), 2.26 (s, 3H), 1.60 (d, *J*= 7.2 Hz, 3H), 1.51 - 1.45 (m, 4H), 1.10 (t, *J* = 7.2 Hz, 3H).

**Example 52: SZ-022363**

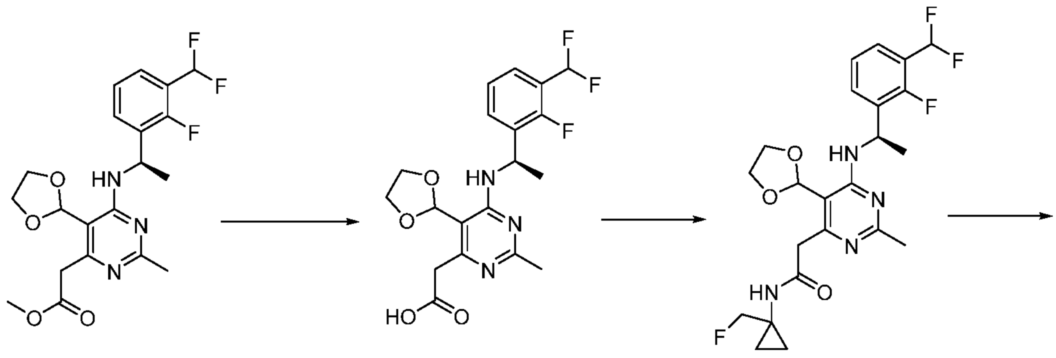

SZ022037A4 SZ022037A5 SZ022363A1

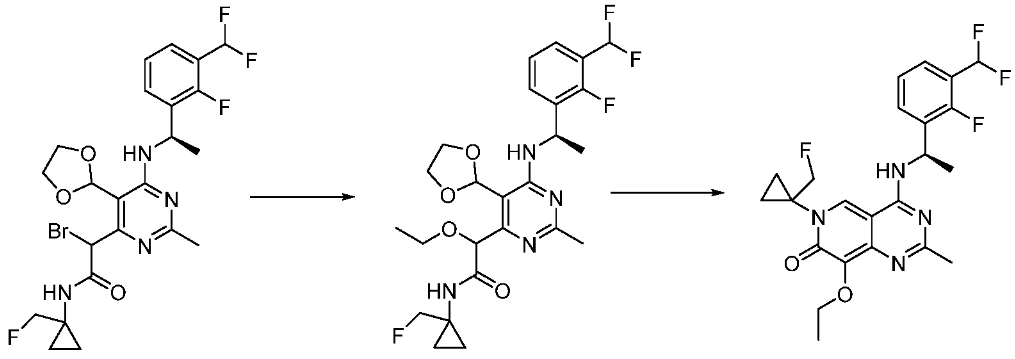

SZ022363A2 SZ022363A3 SZ-022363

Step 1:

**SZ-022037A4** (150 mg, 0.35 mmol) was added to dimethyl sulfoxide (3 mL), and then a 10% sodium hydroxide solution (0.42 mL, 1.06 mmol) was added. The mixture was allowed to react at room temperature for 1 h. After the starting materials were consumed as monitored by LC-MS, the reaction mixture was used directly in the next step without work-up. LC-MS: $[M+H]^+$ 411.89.

Step 2:

1-(Monofluoromethyl)cyclopropanamine hydrochloride (66 mg, 0.53 mmol), *N*,*N*,*N*',*N*'-tetramethyl-*O*-(7-azabenzotriazol-1-yl)uronium hexafluorophosphate (402 mg, 1.06 mmol), and triethylamine (107 mg, 1.06 mmol) were added to the reaction system in the previous step, and the mixture was allowed to react at room temperature for 1 h. After the starting materials were consumed as monitored by LC-MS, water (20 mL) and ethyl acetate (30 mL) were added to the reaction mixture for extraction, and the organic layer was separated, washed with a saturated sodium chloride solution, dried, and concentrated to dryness under reduced pressure to give a crude product. The crude product was purified by flash silica gel column chromatography (PE:EA = 2:3) to give **022363A1** as a white solid (95 mg). LC-MS: $[M+H]^+$ 483.16.

Step 3:

**SZ-022363A1** (95 mg, 0.20 mmol) was added to carbon tetrachloride (4 mL), and then N-bromosuccinimide (35 mg, 0.20 mmol) and azobisisobutyronitrile (3.2 mg, 0.019 mmol) were added. The mixture was heated to 60 °C for reaction for 1 h. After the starting materials were consumed as monitored by LC-MS, the reaction mixture was directly purified by flash silica gel column chromatography (PE:EA = 4:1) to give **022363A2** as a white solid (100 mg). LC-MS: $[M+H]^+$ 560.71.

Step 4:

Sodium hydride (71 mg, 0.18 mmol) was added to tetrahydrofuran (3 mL), and then ethanol (98 mg, 2.14 mmol) was added. The mixture was stirred at 0 °C for 0.5 h. A solution of **SZ-022363A2** (100 mg, 0.18 mmol) in tetrahydrofuran

(2 mL) was added dropwise to the reaction mixture, and the resulting mixture was stirred for another 2.5 h. After the starting materials were consumed as monitored by LC-MS, water (10 mL) and ethyl acetate (10 mL) were added to the reaction mixture for extraction, and the organic layer was separated, washed with a saturated sodium chloride solution, dried, and concentrated by rotary evaporation to give crude product **SZ-022363A3** as a yellow oil (32 mg). LC-MS: $[M+H]^+$ 527.02.

Step 5:

**SZ-022363A3** (32 mg, 0.06 mmol) was added to isopropanol (2.5 mL), and then a hydrochloric acid solution (2 M, 1 mL) was added. The mixture was allowed to react at 50 °C for 5 h. After the starting materials were consumed as monitored by LC-MS, the reaction mixture was directly concentrated by rotary evaporation. A saturated sodium bicarbonate solution (5 mL) and ethyl acetate (10 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022363** (12 mg). LC-MS: $[M+H]^+$ 464.88.

**SZ-022363:** $^1$H NMR (400 MHz, MeOH-$d_6$) δ 8.93 (s, 1H), 8.78 (d, *J*= 7.2 Hz, 1H), 7.63 (t, *J* = 7.6 Hz, 1H), 7.51 (t, *J* = 7.2 Hz, 1H), 7.31 (t, *J* = 7.6 Hz, 1H), 7.22 (t, *J*= 54.4 Hz, 1H), 5.78 - 5.71 (m, 1H), 4.66 - 4.59 (m, 2H), 4.05 (q, *J*= 7.2 Hz, 2H), 2.20 (s, 3H), 1.57 (d, *J*= 7.2 Hz, 3H) 1.32 (m, 4H), 1.21 (t, *J* = 7.2 Hz, 3H).

**Example 53: SZ-022364**

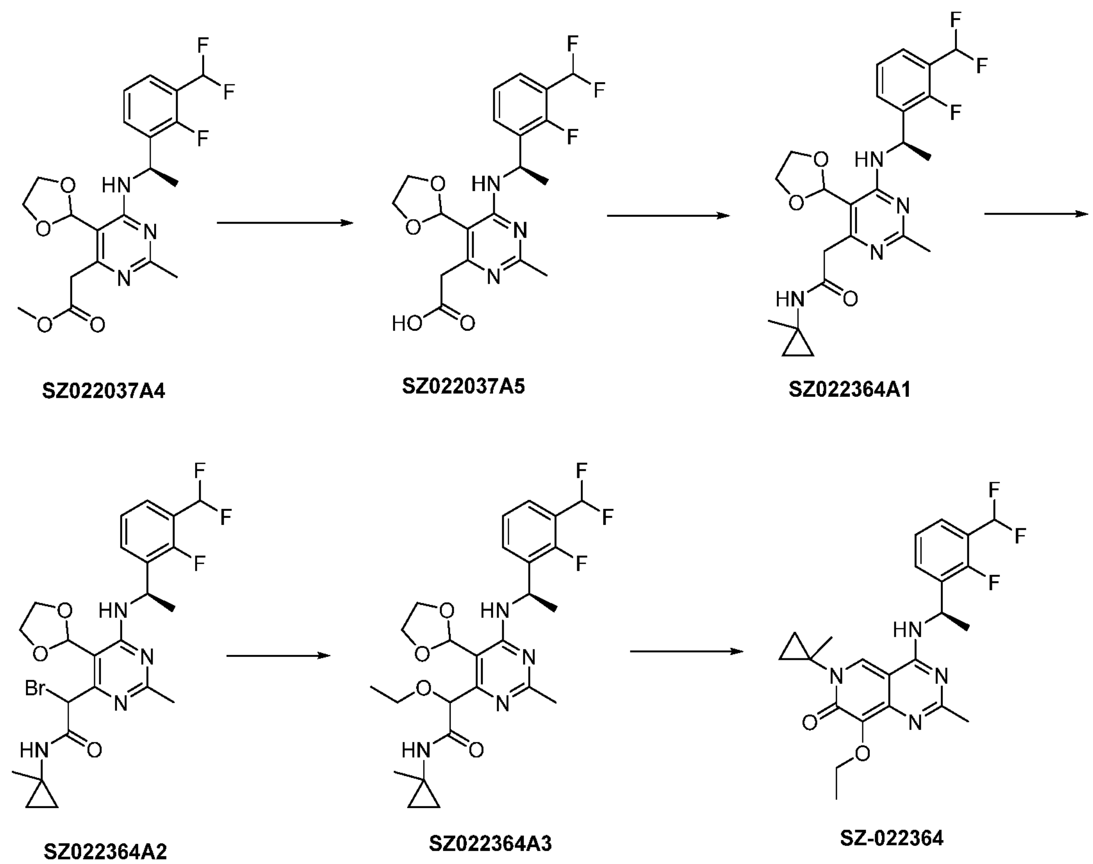

SZ022037A4 SZ022037A5 SZ022364A1

SZ022364A2 SZ022364A3 SZ-022364

Step 1:

**SZ-022037A4** (150 mg, 0.35 mmol) was added to dimethyl sulfoxide (3 mL), and then a 10% sodium hydroxide solution (0.42 mL, 1.06 mmol) was added. The mixture was allowed to react at room temperature for 1 h. After the starting materials were consumed as monitored by LC-MS, the reaction mixture was used directly in the next step without work-up. LC-MS: $[M+H]^+$ 411.92.

Step 2:

1-Methylcyclopropanamine hydrochloride (57 mg, 0.53 mmol), *N*,*N*,*N'*,*N'*-tetramethyl-*O*-(7-azabenzotriazol-1-yl)uronium hexafluorophosphate (402 mg, 1.06 mmol), and triethylamine (107 mg, 1.06 mmol) were added to the reaction system in the previous step, and the mixture was allowed to react at room temperature for 1 h. After the starting materials were consumed as monitored by LC-MS, water (20 mL) and ethyl acetate (30 mL) were added to the reaction mixture for extraction, and the organic layer was separated, washed with a saturated sodium chloride solution, dried, and concentrated to dryness under reduced pressure to give a crude product. The crude product was purified by flash silica gel column chromatography (PE:EA = 2:3) to give **022364A1** as a white solid (120 mg). LC-MS: $[M+H]^+$ 464.95.

Step 3:

**SZ-022364A1** (120 mg, 0.26 mmol) was added to carbon tetrachloride (3.5 mL), and then N-bromosuccinimide (46 mg, 0.26 mmol) and azobisisobutyronitrile (4.2 mg, 0.026 mmol) were added. The mixture was heated to 60 °C for reaction for 1 h. After the starting materials were consumed as monitored by LC-MS, the reaction mixture was directly purified by flash silica gel column chromatography (PE:EA = 4:1) to give **022364A2** as a white solid (80 mg). LC-MS: $[M+H]^+$ 542.75.

Step 4:

Sodium hydride (74 mg, 0.18 mmol) was added to tetrahydrofuran (3 mL), and then ethanol (101 mg, 2.21 mmol) was added. The mixture was stirred at 0 °C for 0.5 h. A solution of **SZ-022364A2** (100 mg, 0.18 mmol) in tetrahydrofuran (1.5 mL) was added dropwise to the reaction mixture, and the resulting mixture was stirred for another 2.5 h. After the starting materials were consumed as monitored by LC-MS, water (10 mL) and ethyl acetate (10 mL) were added to the reaction mixture for extraction, and the organic layer was separated, washed with a saturated sodium chloride solution, dried, and concentrated by rotary evaporation to give **SZ-022364A3** as a white solid (50 mg). LC-MS: $[M+H]^+$ 509.11.

Step 5:

**SZ-022364A3** (50 mg, 0.1 mmol) was added to isopropanol (2 mL), and then a hydrochloric acid solution (2 M, 1 mL) was added. The mixture was allowed to react at 50 °C for 5 h. After the starting materials were consumed as monitored by LC-MS, the reaction mixture was directly concentrated by rotary evaporation. A saturated sodium bicarbonate solution (5 mL) and ethyl acetate (10 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022364** (30 mg). LC-MS: $[M+H]^+$ 446.91.

**SZ-022364:** $^1$H NMR (400 MHz, DMSO-$d_6$) δ 9.13 (s, 1H), 9.05 (s, 1H), 7.73 (t, *J* = 7.6 Hz, 1H), 7.51 (t, *J* = 7.2 Hz, 1H), 7.30 (t, *J* = 7.7 Hz, 1H), 7.23 (t, *J* = 53.6 Hz, 1H), 5.80 - 5.73 (m, 1H), 4.05 (q, *J* = 7.2 Hz, 2H), 2.20 (s, 3H), 1.58 (d, *J* = 7.2 Hz, 3H), 1.50 (s, 3H), 1.20 (t, *J* = 7.2 Hz, 3H), 1.14 - 1.01 (m, 4H).

**Example 54: SZ-022355**

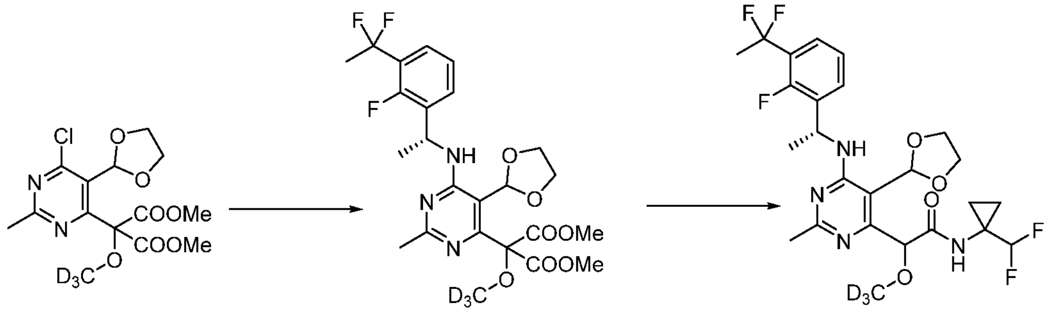

SZ-022334A4 SZ-022355A1 SZ-022355A2

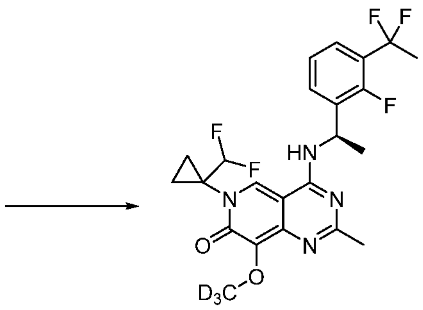

SZ-022355

Step 1:

**SZ-022334A4** (200 mg, 0.55 mmol), (1*R*)-1-(2-fluoro-3-(1,1-difluoro)ethylphenyl)ethylamine hydrochloride (139 mg, 0.58 mmol), and *N,N*-diisopropylethylamine (213 mg, 1.65 mmol) were added to dimethyl sulfoxide (2 mL), and the mixture was heated to 80 °C for reaction for 15 h. After the starting materials were consumed as monitored by LC-MS, water (100 mL) and ethyl acetate (100 mL) were added to the reaction mixture for extraction, and the organic layer was separated, washed with a saturated sodium chloride solution, dried, and concentrated by rotary evaporation to give a crude product. The crude product was purified by flash silica gel column chromatography (PE:EA = 3:1; Rf = 0.3) to give product **SZ-022355A1** as a light yellow oil (120 mg). LC-MS: $[M+H]^+$ 530.99.

Step 2:

**SZ-022355A1** (120 mg, 0.22 mmol) was added to dimethyl sulfoxide (2 mL), and then a 10% sodium hydroxide solution (500 μL) was added. The mixture was allowed to react at 25 °C for 1 h. 1-Difluoromethylcyclopropanamine hydrochloride (49 mg, 0.34 mmol), *N,N,N',N'*-tetramethyl-*O*-(7-azabenzotriazol-1-yl)uronium hexafluorophosphate (258 mg, 0.68 mmol), and triethylamine (69 mg, 0.68 mmol) were then added into the system, and the mixture was allowed to react at room temperature for another 2 h. After the starting materials were consumed as monitored by LC-MS, water (20 mL) and ethyl acetate (20 mL) were added to the reaction mixture for extraction, and the organic layer was separated, washed with a saturated sodium chloride solution, dried, and concentrated by rotary evaporation to give crude product **SZ-022355A2** as a light yellow oil (190 mg). LC-MS: $[M+H]^+$ 548.05.

Step 3:

**SZ-022355A2** (190 mg, 0.22 mmol) was added to isopropanol (2 mL), and then a hydrochloric acid solution (2 M, 0.5 mL) was added. The mixture was allowed to react at 50 °C for 3 h. After the starting materials were consumed as monitored by LC-MS, the reaction mixture was directly concentrated by rotary evaporation. A saturated sodium bicarbonate solution (5 mL), water (10 mL), and ethyl acetate (20 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022355** (60 mg). LC-MS: $[M+H]^+$ 486.18.

**SZ-022355:** $^1H$ NMR (400 MHz, DMSO-$d_6$) δ 8.93 (s, 1H), 8.80 (d, *J* = 6.8 Hz, 1H), 7.59 (t, *J* = 6.8 Hz, 1H), 7.45 (t, *J* = 6.8 Hz, 1H), 7.28 (t, *J* = 7.6 Hz, 1H), 6.34 (t, *J* = 57.2 Hz, 1H), 5.78 - 5.71 (m, 1H), 2.21 (s, 3H), 2.02 (t, *J*= 19.2 Hz, 3H), 1.57 (d, *J*= 6.8 Hz, 3H), 1.51 - 1.47 (m, 2H), 1.45 - 1.33 (m, 2H).

**Example 55: SZ-022349**

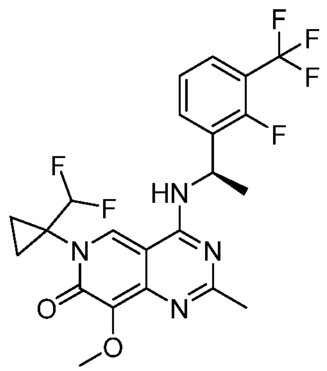

**SZ-022349**

By referring to Example **SZ-022348** with **022348A4** replaced by (*R*)-1-(2-fluoro-3-(trifluoromethyl)phenyl)ethyl-1-amine hydrochloride, separation was performed by preparative high performance liquid chromatography, followed by lyophilization to give **SZ-022349** as a yellow solid (30 mg). LC-MS: $[M+H]^+$ 486.85. $^1H$ NMR (400 MHz, DMSO-$d_6$) δ 8.92 (s, 1H), 8.85 (d, *J* = 6.8 Hz, 1H), 7.76 (t, *J* = 7.2 Hz, 1H), 7.66 (t, *J* = 7.6 Hz, 1H), 7.38 (t, *J* = 7.6 Hz, 1H), 6.34 (t, *J* = 56.8 Hz, 1H), 5.74 - 5.67 (m, 1H), 3.76 (s, 3H), 2.19 (s, 3H), 1.59 (d, *J* = 7.2 Hz, 3H), 1.51 - 1.47 (m, 2H), 1.42 - 1.35 (m, 2H).

**Example 56: SZ-022350**

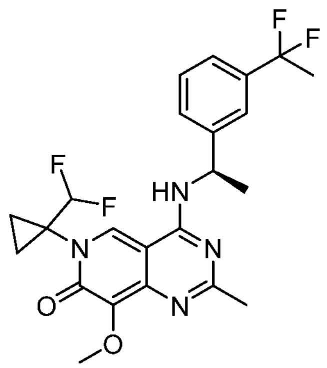

**SZ-022350**

By referring to Example **SZ-022348** with **022348A4** replaced by (*R*)-1-(3-(1,1-difluoroethyl)phenyl)ethyl-1-amine hydrochloride, separation was performed by preparative high performance liquid chromatography, followed by lyophilization to give **SZ-022350** as a yellow solid (20 mg). LC-MS: $[M+H]^+$ 465.21. $^1H$ NMR (400 MHz, $CDCl_3$) δ 8.22 (s, 1H), 7.60 (s, 1H), 7.51-7.54 (m, 1H), 7.40-7.42 (m, 2H), 6.67 (br, 1H), 6.32 (t, *J* = 58.8 Hz, 1H), 5.78 (br, 1H), 4.06 (s, 3H), 2.486 (s, 3H), 1.94 (t, *J* = 18.0 Hz, 3H), 1.71 (d, *J* = 7.2 Hz, 3H), 1.54-1.56 (m, 2H), 1.25 (br, 2H).

**Example 57: SZ-022368**

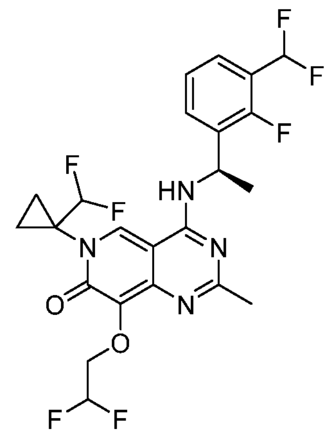

**SZ-022368**

By referring to Example **SZ-022351** with (*S*)-3-hydroxytetrahydrofuran replaced by 2,2-difluoroethanol, separation was performed by preparative high performance liquid chromatography, followed by lyophilization to give **SZ-022368** as a yellow solid (30 mg). LC-MS: $[M+H]^+$ 519.17. $^1H$ NMR (400 MHz, DMSO-$d_6$) $\delta$ 8.98 (s, 1H), 8.88 (d, *J*= 7.1 Hz, 1H), 7.64 (t, *J* = 7.4 Hz, 1H), 7.53 (t, *J* = 7.1 Hz, 1H), 7.33 (t, *J* = 7.7 Hz, 1H), 7.23 (t, *J* = 54.2 Hz, 1H), 6.35 (t, *J* = 56.8 Hz, 1H), 6.32 (tt, *J* = 55.4, 4.0 Hz, 1H), 5.79 - 5.72 (m, 1H), 4.26 (td, *J* = 14.5, 4.1 Hz, 2H), 2.23 (s, 3H), 1.59 (d, *J*= 7.1 Hz, 3H), 1.53 - 1.33 (m, 4H).

**Example 58: SZ-022362**

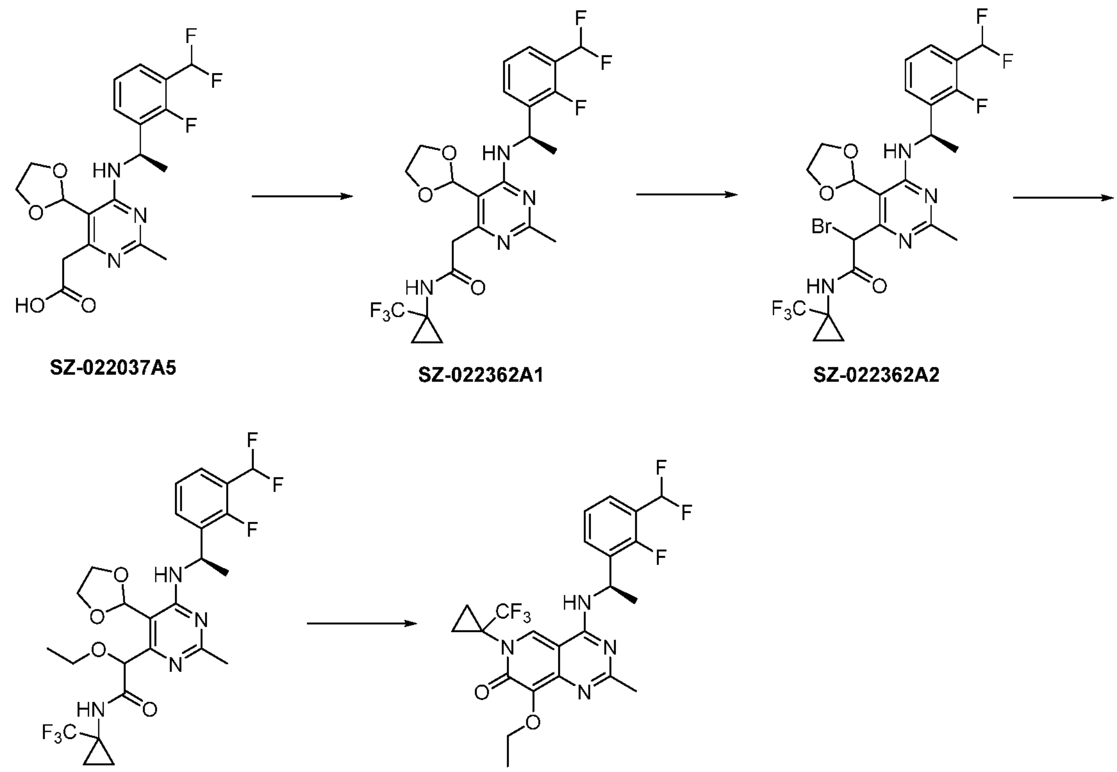

**SZ-022037A5** **SZ-022362A1** **SZ-022362A2**

**SZ-022362A3** **SZ-022362**

Step 1:

**SZ-022037A5** (411 mg, 1 mmol) was added to *N*,*N*-dimethylformamide (10 mL), and then 1-trifluoromethylcyclopropanamine hydrochloride (242 mg, 1.5 mmol), *N*,*N*,*N'*,*N'*-tetramethyl-*O*-(7-azabenzotriazol-1-yl)uronium hexafluorophosphate (1.14 mg, 3 mmol), and triethylamine (303 mg, 3 mmol) were added. The mixture was allowed to react at room temperature for 1 h. After the starting materials were consumed as monitored by LC-MS, water (30 mL) and ethyl acetate (50 mL) were added to the reaction mixture for extraction, and the organic layer was separated, washed with a saturated sodium chloride solution, dried, and concentrated to dryness under reduced pressure to give a crude product. The crude product was purified by flash silica gel column chromatography (PE:EA= 2:3) to give **SZ-022362A1** as a white solid (180 mg). LC-MS: $[M+H]^+$ 519.00.

Step 2:

**SZ-022362A1** (180 mg, 0.35 mmol) was added to acetonitrile (7 ml), and then N-bromosuccinimide (62 mg, 0.35 mmol) was added. The mixture was allowed to react at room temperature for 1 h. After the starting materials were consumed as monitored by LC-MS, the reaction mixture was directly purified by flash silica gel column chromatography (PE:EA= 4:1) to give **SZ-022362A2** as a white solid (110 mg). LC-MS: $[M+H]^+$ 596.74.

Step 3:

Sodium hydride (74 mg, 1.84 mmol) was added to tetrahydrofuran (3 mL), and then ethanol (102 mg, 2.21 mmol) was added. The mixture was stirred at 0 °C for 0.5 h. A solution of **SZ-022362A2** (110 mg, 0.18 mmol) in tetrahydrofuran (2 mL) was added dropwise to the reaction mixture, and the resulting mixture was stirred for another 2.5 h. After the starting materials were consumed as monitored by LC-MS, water (15 mL) and ethyl acetate (15 mL) were added to the reaction mixture for extraction, and the organic layer was separated, washed with a saturated sodium chloride solution, dried, and concentrated by rotary evaporation. The residue was purified by flash silica gel column chromatography (PE:EA = 3:2) to give **SZ-022362A3** as a white solid (20 mg). LC-MS: $[M+H]^+$ 562.93.

Step 4:

**SZ-022362A3** (32 mg, 0.06 mmol) was added to isopropanol (2 mL), and then a hydrochloric acid solution (2 M, 1 mL) was added. The mixture was allowed to react at 50 °C for 5 h. After the starting materials were consumed as monitored by LC-MS, the reaction mixture was directly concentrated by rotary evaporation. A saturated sodium bicarbonate solution (5 mL) and ethyl acetate (10 mL) were then added for extraction, and the organic layer was separated and washed with a saturated sodium chloride solution. The solvent was removed by rotary evaporation, and the residue was subjected to preparative high performance liquid chromatography and then lyophilized to give **SZ-022362** (7 mg). LC-MS: $[M+H]^+$ 500.90.

**SZ-022362:** $^1H$ NMR (400 MHz, DMSO-*$d_6$*) δ 8.97 (s, 1H), 8.82 (d, *J* = 7.2 Hz, 1H), 7.63 (t, *J* = 7.2 Hz, 1H), 7.52 (t, *J* = 7.2 Hz, 1H), 7.32 (t, *J* = 7.6 Hz, 1H), 7.23 (t, *J* = 54.4 Hz, 1H), 5.76 - 5.73 (m, 1H), 4.05 (q, *J* = 7.2 Hz, 2H), 2.20 (s, 3H), 1.64 - 1.53 (m, 4H), 1.57 (d, *J* = 6.8 Hz, 3H), 1.20 (t, *J*= 7.2 Hz, 3H).

**Example 59: SZ-022359**

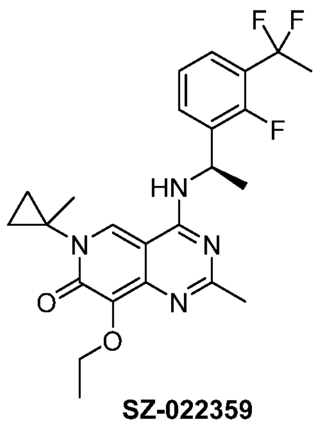

**SZ-022359**

By referring to Example **SZ-022338** with 1-difluoromethylcyclopropanamine hydrochloride replaced by 1-methylcyclopropanamine hydrochloride, separation was performed by preparative high performance liquid chromatography, followed by lyophilization to give **SZ-022359** as a yellow solid (21 mg). LC-MS: $[M+H]^+$ 461.19. $^1H$ NMR (400 MHz, DMSO-$d_6$) δ 8.98 (s, 1H), 8.69 (d, *J* = 6.8 Hz, 1H), 7.67 - 7.56 (m, 1H), 7.43 - 7.47 (m, 1H), 7.27 (t, *J* = 7.6 Hz, 1H), 5.80 - 5.68 (m, 1H), 4.05 (q, *J* = 7.2 Hz, 2H), 2.19 (s, 3H), 2.03 (t, *J* = 19.2 Hz, 3H), 1.57 (d, *J* = 7.2 Hz, 3H), 1.51 (s, 3H), 1.21 (t, *J* = 7.2 Hz, 3H), 1.17 - 0.99 (m, 4H).

**Example 60: SZ-022360**

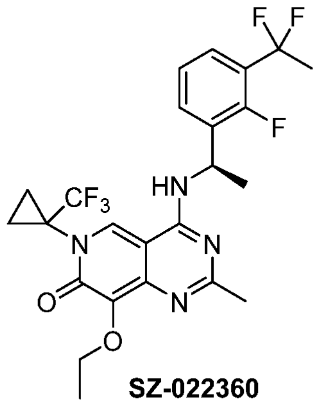

**SZ-022360**

By referring to Example **SZ-022338** with 1-difluoromethylcyclopropanamine hydrochloride replaced by 1-trifluoromethylcyclopropanamine hydrochloride, separation was performed by preparative high performance liquid chromatography, followed by lyophilization to give **SZ-022360** as a yellow solid (57 mg). LC-MS: $[M+H]^+$ 515.17. $^1H$ NMR (400 MHz, DMSO-$d_6$) δ 8.99 (s, 1H), 8.82 (d, *J* = 6.8 Hz, 1H), 7.59 (t, *J* = 6.8 Hz, 1H), 7.46 (t, *J* = 7.2 Hz, 1H), 7.32 - 7.24 (m, 1H), 5.78 - 5.70 (m, 1H), 4.06 (q, *J* = 7.2 Hz, 2H), 2.20 (s, 3H), 2.02 (t, *J* = 19.2 Hz, 3H), 1.85 - 1.77 (m, 1H), 1.73 - 1.61 (m, 2H), 1.58 (d, *J* = 7.2 Hz, 3H), 1.56 - 1.49 (m, 1H), 1.21 (t, *J* = 7.2 Hz, 3H).

**Example 61: SZ-022361**

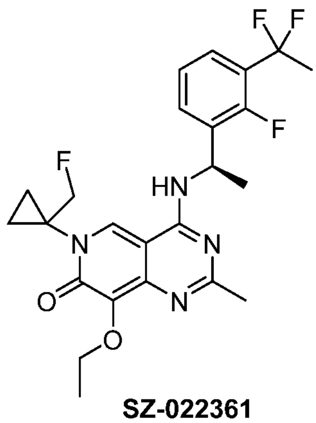

**SZ-022361**

By referring to Example **SZ-022338** with 1-difluoromethylcyclopropanamine hydrochloride replaced by 1-fluoromethylcyclopropanamine hydrochloride, separation was performed by preparative high performance liquid chromatography, followed by lyophilization to give **SZ-022361** as a yellow solid (76 mg). LC-MS: $[M+H]^+$ 479.20. $^1H$ NMR (400 MHz, DMSO-$d_6$) δ 8.94 (s, 1H), 8.84 (s, 1H), 7.59 (t, *J* = 7.6 Hz, 1H), 7.44 (t, *J* = 7.2 Hz, 1H), 7.26 (t, *J* = 8.0 Hz, 1H), 5.77 - 5.70 (m, 1H), 4.75 - 4.50 (m, 1H), 4.06 (q, *J* = 7.2 Hz, 2H), 2.20 (s, 3H), 2.01 (t, *J* = 19.2 Hz, 3H), 1.56 (d, *J* = 7.2 Hz, 3H), 1.35 - 1.25 (m, 4H), 1.21 (t, *J* = 6.8 Hz, 3H).

**Example 62: SZ-022381**

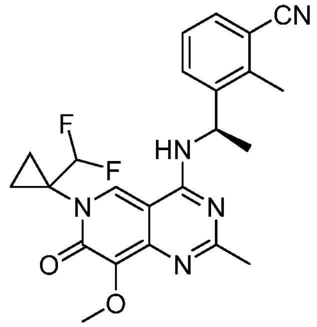

**SZ-022381**

By referring to Example **SZ-022304** with (1*R*)-1-(3-(trifluoromethyl)-2-methylphenyl)ethylamine hydrochloride replaced by (1*R*)-1-(3-cyano-2-methylphenyl)ethylamine hydrochloride, separation was performed by preparative high performance liquid chromatography, followed by lyophilization to give **SZ-022381** as a yellow solid (10 mg). LC-MS: $[M+H]^+$ 440.03. $^1H$ NMR (400 MHz, DMSO-$d_6$) $\delta$ 8.90 (s, 1H), 8.86 (d, *J* = 6.8 Hz, 1H), 7.71 (d, *J* = 7.6 Hz, 1H), 7.66 (d, *J* = 7.6 Hz, 1H), 7.39 (t, *J* = 7.6 Hz, 1H), 6.34 (t, *J* = 56.8 Hz, 1H), 5.65 - 5.57 (m, 1H), 3.76 (s, 3H), 2.66 (s, 3H), 2.22 (s, 3H), 1.52 (d, *J* = 6.8 Hz, 3H), 1.50 - 1.35 (m, 4H).

**Example 63: SZ-022382**

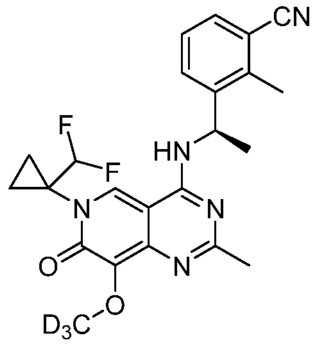

**SZ-022382**

By referring to Example **SZ-022334** with (1*R*)-1-(3-(trifluoromethyl)-2-methylphenyl)ethylamine hydrochloride replaced by (1*R*)-1-(3-cyano-2-methylphenyl)ethylamine hydrochloride, separation was performed by preparative high performance liquid chromatography, followed by lyophilization to give **SZ-022382** as a yellow solid (15 mg). LC-MS: $[M+H]^+$ 443.34. $^1H$ NMR (400 MHz, DMSO-$d_6$) $\delta$ 8.89 (s, 1H), 8.86 (d, *J* = 6.8 Hz, 1H), 7.71 (d, *J* = 7.6 Hz, 1H), 7.66 (d, *J* = 7.6 Hz, 1H), 7.39 (t, *J* = 7.6 Hz, 1H), 6.33 (t, *J* = 56.8 Hz, 1H), 5.64 - 5.68 (m, 1H), 2.66 (s, 3H), 2.22 (s, 3H), 1.52 (d, *J* = 6.8 Hz, 3H), 1.50 - 1.35 (m, 4H).

**Example 64: SZ-022383**

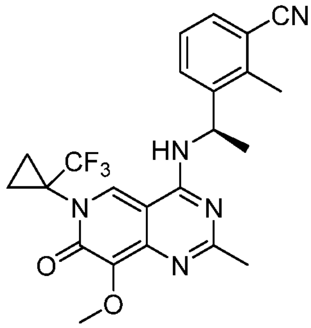

**SZ-022383**

By referring to Example **SZ-022304** with (1*R*)-1-(3-(trifluoromethyl)-2-methylphenyl)ethylamine hydrochloride replaced by (1*R*)-1-(3-cyano-2-methylphenyl)ethylamine hydrochloride and 1-(difluoromethyl)cyclopropanamine hydrochloride replaced by 1-trifluoromethylcyclopropanamine hydrochloride, separation was performed by preparative high performance liquid chromatography, followed by lyophilization to give **SZ-022383** as a yellow solid (78 mg). LC-MS: $[M+H]^+$ 458.30. $^1H$ NMR (400 MHz, DMSO-$d_6$) $\delta$ 8.96 (s, 1H), 8.87 (d, *J* = 6.4 Hz, 1H), 7.71 (d, *J* = 7.6 Hz, 1H), 7.66 (d, *J* = 7.6 Hz, 1H), 7.40 (d, *J* = 6.4 Hz, 1H), 5.66 - 5.55 (m, 1H), 3.76 (s, 3H), 2.66 (s, 3H), 2.22 (s, 3H), 1.89-1.60 (m, 4H), 1.53 (d, *J* = 6.8 Hz, 3H).

**Example 65: SZ-022384**

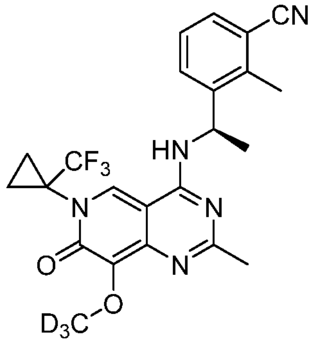

**SZ-022384**

By referring to Example **SZ-022334** with (1*R*)-1-(3-(trifluoromethyl)-2-methylphenyl)ethylamine hydrochloride replaced by (1*R*)-1-(3-cyano-2-methylphenyl)ethylamine hydrochloride and 1-(difluoromethyl)cyclopropanamine hydrochloride replaced by 1-trifluoromethylcyclopropanamine hydrochloride, separation was performed by preparative high performance liquid chromatography, followed by lyophilization to give **SZ-022384** as a yellow solid (83 mg). LC-MS: $[M+H]^+$ 461.33. $^1H$ NMR (400 MHz, DMSO-$d_6$) $\delta$ 8.99 (s, 1H), 8.90 (d, *J*= 6.0 Hz, 1H), 7.74 (d, *J*= 7.6 Hz, 1H), 7.69 (d, *J* = 7.6 Hz, 1H), 7.43 (d, *J*= 6.4 Hz, 1H), 5.70 - 5.58 (m, 1H), 2.69 (s, 3H), 2.25 (s, 3H), 1.90 - 1.60 (m, 4H), 1.56 (d, *J* = 7.2 Hz, 3H).

**Efficacy Example 1: In-cell Western Blot Assay for ERK Phosphorylation in DLD-1 Cells**

DLD-1 cells (ATCC, CCL-221) were thawed and cultured in a culture medium containing RPMI1640 (Gibco, A10491-01), 10% fetal bovine serum (FBS) (Transgene, FS201-02) and 1% antibiotic P/S (Gibco, 15140-122) for 3 days until the cell viability was good. The cells were inoculated into a 384-well plate and incubated at 37 °C with 5% $CO_2$ overnight. A test compound, a positive control compound, and a negative control were added. The compound was at concentrations of 10000 nM to 1.52 nM (3-fold dilution) and 0.051 nM, for a total of 10 concentrations. The mixture was well mixed and incubated at 37 °C with 5% $CO_2$. The cells were washed twice with PBS (Solarbio, P1010), and then a blocking buffer was added. After 1 h of blocking at room temperature, a primary antibody mixture (phospho-p44/42 MAPK (T202/Y204) Rabbit mAb (CST, 4 S), GAPDH (D4C6R) Mouse mAb) (CST, 97166S) was added, followed by

overnight incubation at 4 °C. The cells were washed 3 times with PBST (PBS containing 0.05% Tween-20 (Solarbio, T8220)), and a secondary antibody mixture (goat anti rabbit 800CW (LI-COR, 926-32211), goat anti mouse 680RD (LI-COR, 926-68070)) was added, followed by incubation at room temperature in the dark. The 384-well plate was reversed and centrifuged at 1000 rpm for 1 min, and read on an Odyssey CLx fluorescence imaging system (LI-COR) to obtain fluorescence signal values, which were corrected using DMSO and BI-3406. The specific calculation is as follows, and the results are shown in Table 1:

$$\text{Relative Signal} = \text{Signal Value (total channel 800)} / \text{Signal Value (total channel 700)}$$

$$H = \text{Ave (DMSO)}$$

$$L = \text{Ave (BI-3406)}$$

$$SD (H) = \text{STDEV (DMSO)}$$

$$SD (L) = \text{STDEV (BI-3406)}$$

$$CV\% (DMSO) = 100 * (SD_H / Ave_H)$$

$$CV\% (BI-3406) = 100 * SD_L / Ave_L$$

$$Z' = 1 - 3 * (SD_H + SD_L) / (Ave_H - Ave_L)$$

$$\text{Relative pERK} = (\text{Sample} - Ave_L) / (Ave_H - Ave_L).$$

The $IC_{50}$ values of the compounds were calculated using a four-parameter fitting algorithm, specifically as follows:

$$Y = \text{Bottom} + (\text{Top} - \text{Bottom}) / (1 + 10 \wedge ((\text{LogIC}_{50} - X) * \text{HillSlope}))$$

X: Log of cpd concentration
Y: Ave (relative pERK)
Top and Bottom: Plateaus in same units as Y
log$IC_{50}$: same log units as X
HillSlope: Slope factor or Hill slope.

Table 1

| Compound | DLD-1 p-ERK $IC_{50}$ (nM) | Compound | DLD-1 p-ERK $IC_{50}$ (nM) |
|---|---|---|---|
| **SZ-022244** | 265 | **SZ-022334** | 93 |
| **SZ-022300** | 412 | **SZ-022327** | 153 |
| **SZ-022301** | 262 | **SZ-022330** | 117 |
| **SZ-022302** | 104 | **SZ-022330B** | 400 |
| **SZ-022304** | 79 | **SZ-022295** | 256 |
| **SZ-022296** | 143 | **SZ-022345** | 885 |

(continued)

| Compound | DLD-1 p-ERK $IC_{50}$ (nM) | Compound | DLD-1 p-ERK $IC_{50}$ (nM) |
|---|---|---|---|
| **SZ-022303** | 128 | **SZ-022346** | 1016 |
| **SZ-022317** | 371 | **SZ-022348** | 614 |
| **SZ-022325** | 216 | **SZ-022337** | 174 |
| **SZ-022306** | 213 | **SZ-022339** | 186 |
| **SZ-022329** | 417 | **SZ-022338** | 334 |
| **SZ-022328** | 309 | **SZ-022340** | 367 |
| **SZ-022251** | 146 | **SZ-022313** | 1272 |
| **SZ-022307** | 253 | **SZ-022351** | 555 |
| **SZ-022312** | 601 | **SZ-022352** | 516 |
| **SZ-022314** | 563 | **SZ-022353** | 377 |
| **SZ-022354** | 138 | **SZ-022350** | 211 |
| **SZ-022342** | 57 | **SZ-022368** | 249 |
| **SZ-022349** | 193 | **SZ-022355** | 112 |
| **SZ-022341** | 161 | **SZ-022356** | 218 |
| **SZ-022363** | 171 | **SZ-022357** | 165 |
| **SZ-022364** | 303 | **SZ-022358** | 222 |
| **SZ-022371** | 274 | **SZ-022373** | 42 |
| **SZ-022362** | 77 | **SZ-022381** | 741 |
| **SZ-022359** | 231 | **SZ-022382** | 729 |
| **SZ-022360** | 221 | **SZ-022383** | 985 |
| **SZ-022361** | 164 | **SZ-022384** | 884 |
| **SZ-022299** | 751 | | |

**Efficacy Example 2: Evaluation of Inhibition of SOS1/KRAS G12C Protein Binding**

A test compound was dissolved in 100% DMSO to give a solution at a concentration of 10 mM. 10 μL of the solution was taken and 3-fold diluted with 100% DMSO to prepare test compound solutions with concentrations of 10000 nM to 0.017 nM, for a total of 11 concentrations. The compound solutions at different concentrations were then transferred to a 384-well plate at 0.1 μL/well using a sonic droplet device Echo (Labcyte), and 2 replicate wells were set for each concentration.

A 15 nM KRAS G12C (aa 1-169) protein (Pharmaron Beijing Co., Ltd., 20200707) was transferred to the 384-well reaction plate at 5 μL/well using the sonic droplet device Echo (Labcyte), and then the reaction plate was centrifuged in a centrifuge (Eppendorf, 5810R) at 1000 rpm/min for 1 min.

A 2.5 nM His-SOS1 (aa 564-1049) protein (Pharmaron Beijing Co., Ltd., 20200717) was transferred to the 384-well reaction plate at 5 μL/well using the sonic droplet device Echo (Labcyte), and then the reaction plate was centrifuged in a centrifuge (Eppendorf, 5810R) at 1000 rpm/min for 1 min and incubated at 25 °C for 15 min.

A mixed solution of Antibody 1 (MAb Anti-6his-Tb cryptate Gold, Cisbio, 61HI2TLA) and Antibody 2 (MAb Anti-GST-XL665, Cisbio, 61GSTXLA) was transferred to the 384-well reaction plate at 10 μL/well using the sonic droplet device Echo (Labcyte), and then the reaction plate was centrifuged in a centrifuge (Eppendorf, 5810R) at 1000 rpm/min for 1 min and incubated at 25 °C for 120 min.

The 665 nm/615 nm optical density ratio was read using an Envision multifunctional microplate reader (Perkin Elmer, Envision 2104).

Analysis of original data:

The $IC_{50}$ values of the compounds were fitted by a nonlinear regression equation of Graphpad Prism 8. The results are shown in Table 2:
Negative control: DMSO

The $IC_{50}$ (half maximal inhibitory concentration) values of the compounds were calculated using the following non-linear fitting equation:

$$Y = Bottom + (Top - Bottom) / (1 + 10 ^ ((LogIC_{50} - X) * HillSlope))$$

X: Log of cpd concentration
Y: 665 nm/615 nm ratio

Table 2

| Compound | KRAS-SOS1 $IC_{50}$ (nM) | Compound | KRAS-SOS 1 $IC_{50}$ (nM) |
|---|---|---|---|
| **SZ-022244** | 17.77 | **SZ-022327** | 5.045 |
| **SZ-022301** | 6.496 | **SZ-022330** | 5.018 |
| **SZ-022300** | 12.08 | **SZ-022330B** | 9.99 |
| **SZ-022302** | 3.788 | **SZ-022295** | 8.153 |
| **SZ-022304** | 6.982 | **SZ-022348** | 21.78 |
| **SZ-022296** | 9.425 | **SZ-022343** | 26.65 |
| **SZ-022303** | 9.77 | **SZ-022344** | 23.71 |
| **SZ-022317** | 4.538 | **SZ-022345** | 21.79 |
| **SZ-022325** | 15.91 | **SZ-022346** | 29.97 |
| **SZ-022326** | 10.52 | **SZ-022337** | 4.144 |
| **SZ-022306** | 12.52 | **SZ-022339** | 7.249 |
| **SZ-022329** | 2.635 | **SZ-022338** | 12.08 |
| **SZ-022328** | 9.698 | **SZ-022340** | 15.18 |
| **SZ-022336** | 36.52 | **SZ-022299** | 22.43 |
| **SZ-022307** | 25.85 | **SZ-022351** | 21.36 |
| **SZ-022312** | 17.87 | **SZ-022353** | 17.47 |
| **SZ-022314** | 17.33 | **SZ-022352** | 16 |
| **SZ-022334** | 4.178 | **SZ-022347** | 60.08 |
| **SZ-022335** | 27.87 | **SZ-022354** | 21.82 |
| **SZ-022350** | 16.73 | **SZ-022363** | 7.358 |
| **SZ-022342** | 7.147 | **SZ-022364** | 22.24 |
| **SZ-022368** | 23.45 | **SZ-022371** | 21.79 |
| **SZ-022349** | 7.234 | **SZ-022372** | 36.65 |
| **SZ-022341** | 7.755 | **SZ-022355** | 2.472 |
| **SZ-022356** | 4.358 | **SZ-022357** | 3.681 |
| **SZ-022358** | 4.793 | **SZ-022373** | 1.396 |
| **SZ-022362** | 12.44 | **SZ-022381** | 12.31 |
| **SZ-022359** | 8.568 | **SZ-022382** | 8.862 |

(continued)

| Compound | KRAS-SOS1 $IC_{50}$ (nM) | Compound | KRAS-SOS 1 $IC_{50}$ (nM) |
|---|---|---|---|
| **SZ-022360** | 9.461 | **SZ-022383** | 18.45 |
| **SZ-022361** | 4.618 | **SZ-022384** | 15.4 |
| **SZ-022313** | 43.97 | | |

## Claims

**1.** A fused ring compound represented by formula I or a pharmaceutically acceptable salt thereof:

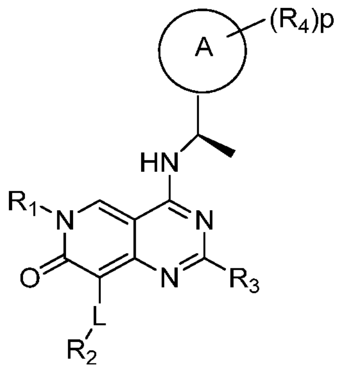

I ,

wherein,

$R_1$ is $C_{1-6}$ alkyl, $C_{1-6}$ alkyl substituted with one or more $R_{1-1a}$, $C_{3-10}$ cycloalkyl, $C_{3-10}$ cycloalkyl substituted with one or more $R_{1-1b}$, "3- to 10-membered heterocyclyl having 1, 2 or 3 heteroatoms selected from 1, 2 or 3 of N, O and S", "3- to 10-membered heterocyclyl having 1, 2 or 3 heteroatoms selected from 1, 2 or 3 of N, O and S" substituted with one or more $R_{1-1c}$, $C_{6-10}$ aryl, $C_{6-10}$ aryl substituted with one or more $R_{1-1d}$, "5- to 10-membered heteroaryl having 1, 2 or 3 heteroatoms selected from 1, 2 or 3 of N, O and S", or "5- to 10-membered heteroaryl having 1, 2 or 3 heteroatoms selected from 1, 2 or 3 of N, O and S" substituted with one or more $R_{1-1e}$;

$R_{1-1a}$, $R_{1-1b}$, $R_{1-1c}$, $R_{1-1d}$, and $R_{1-1e}$ are each independently $-OR_{1-2a}$, $-NR_{1-2b}R_{1-2c}$, halogen, - CN, $-C(O)R_{1-2d}$, $-C(O)OR_{1-2e}$, $-C(O)NR_{1-2f}R_{1-2g}$, $C_{1-6}$ alkyl, $C_{1-6}$ alkyl substituted with one or more $R_{1-3a}$, $C_{3-10}$ cycloalkyl, or "$C_{3-10}$ cycloalkyl substituted with one or more $R_{1-3b}$";

$R_{1-2a}$, $R_{1-2b}$, $R_{1-2c}$, $R_{1-2d}$, $R_{1-2e}$, $R_{1-2f}$, and $R_{1-2g}$ are each independently H, $C_{1-6}$ alkyl, $C_{1-6}$ alkyl substituted with one or more $R_{1-3c}$, $C_{3-10}$ cycloalkyl, $C_{3-10}$ cycloalkyl substituted with one or more $R_{1-3d}$, "3- to 10-membered heterocyclyl having 1, 2 or 3 heteroatoms selected from 1, 2 or 3 of N, O and S", "3- to 10-membered heterocyclyl having 1, 2 or 3 heteroatoms selected from 1, 2 or 3 of N, O and S" substituted with one or more $R_{1-3e}$, $C_{6-10}$ aryl, $C_{6-10}$ aryl substituted with one or more $R_{1-3f}$, "5- to 10-membered heteroaryl having 1, 2 or 3 heteroatoms selected from 1, 2 or 3 of N, O and S", or "5- to 10-membered heteroaryl having 1, 2 or 3 heteroatoms selected from 1, 2 or 3 of N, O and S" substituted with one or more $R_{1-3g}$;

$R_{1-3a}$, $R_{1-3b}$, $R_{1-3c}$, $R_{1-3d}$, $R_{1-3e}$, $R_{1-3f}$, and $R_{1-3g}$ are each independently $-OR_{1-4a}$, $-NR_{1-4b}R_{1-4c}$, halogen, $C_{1-6}$ alkyl substituted with one or more halogens, $C_{3-10}$ cycloalkyl, "3- to 10-membered heterocyclyl having 1, 2 or 3 heteroatoms selected from 1, 2 or 3 of N, O and S", $C_{6-10}$ aryl, or "5- to 10-membered heteroaryl having 1, 2 or 3 heteroatoms selected from 1, 2 or 3 of N, O and S";

$R_{1-4a}$, $R_{1-4b}$, and $R_{1-4c}$ are each independently hydrogen, $C_{1-6}$ alkyl, $C_{1-6}$ alkyl substituted with one or more halogens, $C_{3-10}$ cycloalkyl, "3- to 10-membered heterocyclyl having 1, 2 or 3 heteroatoms selected from 1, 2 or 3 of N, O and S", $C_{6-10}$ aryl, or "5- to 10-membered heteroaryl having 1, 2 or 3 heteroatoms selected from 1, 2 or 3 of N, O and S";

L is -O-, -S-, $-SO_2-$, or $-NR_{L-1}$; $R_{L-1}$ is hydrogen or $C_{1-6}$ alkyl;

$R_2$ is selected from hydrogen, $C_{1-6}$ alkyl, $C_{1-6}$ alkyl substituted with one or more $R_{2-1}$, $C_{3-10}$ cycloalkyl, or "3- to 10-membered heterocyclyl having 1, 2 or 3 heteroatoms selected from 1, 2 or 3 of N, O and S";

each $R_{2-1}$ is independently deuterium, halogen, $C_{3-10}$ cycloalkyl, or hydroxy;

$R_3$ is hydrogen, $C_{1-6}$ alkyl, or $C_{1-6}$ alkyl substituted with one or more halogens;
each $R_4$ is independently halogen, $-NH_2$, -CN, $C_{1-6}$ alkyl, $C_{1-6}$ alkyl substituted with one or more $R_{4-1}$, "3- to 10-membered heterocyclyl having 1, 2 or 3 heteroatoms selected from 1, 2 or 3 of N, O and S", $C_{3-6}$ cycloalkyl, $C_{6-10}$ aryl, or $C_{6-10}$ aryl substituted with one or more - $(CH_2)_mNR_{4-2}R_{4-3}$, wherein m is 0, 1, 2 or 3;
each $R_{4-1}$ is independently halogen or hydroxy;
$R_{4-2}$ and $R_{4-3}$ are each independently $C_{1-6}$ alkyl;
ring A is $C_{6-10}$ aryl, "5- to 10-membered heterocyclyl having 1, 2 or 3 heteroatoms selected from 1, 2 or 3 of N, O and S", or "5- to 10-membered heteroaryl having 1, 2 or 3 heteroatoms selected from 1, 2 or 3 of N, O and S"; and
p is 1, 2 or 3.

**2.** The fused ring compound represented by formula I or the pharmaceutically acceptable salt thereof according to claim 1, wherein,

$R_1$ is $C_{1-6}$ alkyl, $C_{1-6}$ alkyl substituted with one or more $R_{1-1a}$, $C_{3-10}$ cycloalkyl, $C_{3-10}$ cycloalkyl substituted with one or more $R_{1-1b}$, "3- to 10-membered heterocyclyl having 1, 2 or 3 heteroatoms selected from 1, 2 or 3 of N, O and S", "3- to 10-membered heterocyclyl having 1, 2 or 3 heteroatoms selected from 1, 2 or 3 of N, O and S" substituted with one or more $R_{1-1c}$, $C_{6-10}$ aryl, $C_{6-10}$ aryl substituted with one or more $R_{1-1d}$, "5- to 10-membered heteroaryl having 1, 2 or 3 heteroatoms selected from 1, 2 or 3 of N, O and S", or "5- to 10-membered heteroaryl having 1, 2 or 3 heteroatoms selected from 1, 2 or 3 of N, O and S" substituted with one or more $R_{1-1e}$;
$R_{1-1a}$, $R_{1-1b}$, $R_{1-1c}$, $R_{1-1d}$, and $R_{1-1e}$ are each independently $-OR_{1-2a}$, $-NR_{1-2b}R_{1-2c}$, halogen, - CN, $-C(O)R_{1-2d}$, $-C(O)OR_{1-2e}$, $-C(O)NR_{1-2f}R_{1-2g}$, $C_{1-6}$ alkyl, $C_{1-6}$ alkyl substituted with one or more $R_{1-3a}$, $C_{3-10}$ cycloalkyl, or "$C_{3-10}$ cycloalkyl substituted with one or more $R_{1-3b}$";
$R_{1-2a}$, $R_{1-2b}$, $R_{1-2c}$, $R_{1-2d}$, $R_{1-2e}$, $R_{1-2f}$, and $R_{1-2g}$ are each independently H, $C_{1-6}$ alkyl, $C_{1-6}$ alkyl substituted with one or more $R_{1-3c}$, $C_{3-10}$ cycloalkyl, $C_{3-10}$ cycloalkyl substituted with one or more $R_{1-3d}$, "3- to 10-membered heterocyclyl having 1, 2 or 3 heteroatoms selected from 1, 2 or 3 of N, O and S", "3- to 10-membered heterocyclyl having 1, 2 or 3 heteroatoms selected from 1, 2 or 3 of N, O and S" substituted with one or more $R_{1-3e}$, $C_{6-10}$ aryl, $C_{6-10}$ aryl substituted with one or more $R_{1-3f}$, "5- to 10-membered heteroaryl having 1, 2 or 3 heteroatoms selected from 1, 2 or 3 of N, O and S", or "5- to 10-membered heteroaryl having 1, 2 or 3 heteroatoms selected from 1, 2 or 3 of N, O and S" substituted with one or more $R_{1-3g}$;
$R_{1-3a}$, $R_{1-3b}$, $R_{1-3c}$, $R_{1-3d}$, $R_{1-3e}$, $R_{1-3f}$, and $R_{1-3g}$ are each independently $-OR_{1-4a}$, $-NR_{1-4b}R_{1-4c}$, halogen, $C_{1-6}$ alkyl substituted with one or more halogens, $C_{3-10}$ cycloalkyl, "3- to 10-membered heterocyclyl having 1, 2 or 3 heteroatoms selected from 1, 2 or 3 of N, O and S", $C_{6-10}$ aryl, or "5- to 10-membered heteroaryl having 1, 2 or 3 heteroatoms selected from 1, 2 or 3 of N, O and S";
$R_{1-4a}$, $R_{1-4b}$, and $R_{1-4c}$ are each independently hydrogen, $C_{1-6}$ alkyl, $C_{1-6}$ alkyl substituted with one or more halogens, $C_{3-10}$ cycloalkyl, "3- to 10-membered heterocyclyl having 1, 2 or 3 heteroatoms selected from 1, 2 or 3 of N, O and S", $C_{6-10}$ aryl, or "5- to 10-membered heteroaryl having 1, 2 or 3 heteroatoms selected from 1, 2 or 3 of N, O and S";
L is -O-, -S-, $-SO_2-$, or $-NR_{L-1}$; $R_{L-1}$ is hydrogen or $C_{1-6}$ alkyl;
$R_2$ is selected from hydrogen, $C_{1-6}$ alkyl, $C_{1-6}$ alkyl substituted with one or more $R_{2-1}$, $C_{3-10}$ cycloalkyl, or "3- to 10-membered heterocyclyl having 1, 2 or 3 heteroatoms selected from 1, 2 or 3 of N, O and S";
each $R_{2-1}$ is independently deuterium, halogen, $C_{3-10}$ cycloalkyl, or hydroxy;
$R_3$ is hydrogen, $C_{1-6}$ alkyl, or $C_{1-6}$ alkyl substituted with one or more halogens;
each $R_4$ is independently halogen, $-NH_2$, $C_{1-6}$ alkyl, $C_{1-6}$ alkyl substituted with one or more $R_{4-1}$, "3- to 10-membered heterocyclyl having 1, 2 or 3 heteroatoms selected from 1, 2 or 3 of N, O and S", $C_{3-6}$ cycloalkyl, $C_{6-10}$ aryl, or $C_{6-10}$ aryl substituted with one or more $-(CH_2)_mNR_{4-2}R_{4-3}$, wherein m is 0, 1, 2 or 3;
each $R_{4-1}$ is independently halogen or hydroxy;
$R_{4-2}$ and $R_{4-3}$ are each independently $C_{1-6}$ alkyl;
ring A is $C_{6-10}$ aryl, "5- to 10-membered heterocyclyl having 1, 2 or 3 heteroatoms selected from 1, 2 or 3 of N, O and S", or "5- to 10-membered heteroaryl having 1, 2 or 3 heteroatoms selected from 1, 2 or 3 of N, O and S"; and
p is 1, 2 or 3.

**3.** The fused ring compound represented by formula I or the pharmaceutically acceptable salt thereof according to claim 1, wherein each $R_4$ is independently -CN.

**4.** The fused ring compound represented by formula I or the pharmaceutically acceptable salt thereof according to claim 1 or 2, wherein the fused ring compound represented by formula I or the pharmaceutically acceptable salt thereof satisfies one or more of the following conditions:

(1) $R_1$ is $C_{3-10}$ cycloalkyl, $C_{3-10}$ cycloalkyl substituted with one or more $R_{1-1b}$, "3- to 10-membered heterocyclyl having 1, 2 or 3 heteroatoms selected from 1, 2 or 3 of N, O and S", or "3- to 10-membered heterocyclyl having 1, 2 or 3 heteroatoms selected from 1, 2 or 3 of N, O and S" substituted with one or more $R_{1-1c}$;
(2) $R_{1-1a}$, $R_{1-1b}$, $R_{1-1c}$, $R_{1-1d}$, and $R_{1-1e}$ are each independently halogen, -CN, -C(O)$R_{1-2d}$, $C_{1-6}$ alkyl, or $C_{1-6}$ alkyl substituted with one or more $R_{1-3a}$;
(3) $R_{1-2a}$, $R_{1-2b}$, $R_{1-2c}$, $R_{1-2d}$, $R_{1-2e}$, $R_{1-2f}$, and $R_{1-2g}$ are each independently $C_{3-10}$ cycloalkyl;
(4) $R_{1-3a}$, $R_{1-3b}$, $R_{1-3c}$, $R_{1-3d}$, $R_{1-3e}$, $R_{1-3f}$, and $R_{1-3g}$ are each independently -O$R_{1-4a}$ or halogen;
(5) $R_{1-4a}$, $R_{1-4b}$, and $R_{1-4c}$ are each independently hydrogen or $C_{1-6}$ alkyl;
(6) $R_3$ is $C_{1-6}$ alkyl; and
(7) each $R_4$ is independently halogen, -$NH_2$, $C_{1-6}$ alkyl, $C_{1-6}$ alkyl substituted with one or more $R_{4-1}$, or $C_{6-10}$ aryl substituted with one or more -$(CH_2)_m NR_{4-2}R_{4-3}$.

**5.** The fused ring compound represented by formula I or the pharmaceutically acceptable salt thereof according to claim 1 or 2, wherein the fused ring compound represented by formula I or the pharmaceutically acceptable salt thereof satisfies one or more of the following conditions:

(1) $R_1$ is $C_{3-10}$ cycloalkyl substituted with one or more $R_{1-1b}$, "3- to 10-membered heterocyclyl having 1, 2 or 3 heteroatoms selected from 1, 2 or 3 of N, O and S", or "3- to 10-membered heterocyclyl having 1, 2 or 3 heteroatoms selected from 1, 2 or 3 of N, O and S" substituted with one or more $R_{1-1c}$;
(2) $R_{1-1a}$, $R_{1-1b}$, $R_{1-1c}$, $R_{1-1d}$, and $R_{1-1e}$ are each independently $C_{1-6}$ alkyl or $C_{1-6}$ alkyl substituted with one or more $R_{1-3a}$;
(3) $R_{1-3a}$, $R_{1-3b}$, $R_{1-3c}$, $R_{1-3d}$, $R_{1-3e}$, $R_{1-3f}$, and $R_{1-3g}$ are each independently halogen;
(4) $R_2$ is $C_{1-6}$ alkyl, $C_{1-6}$ alkyl substituted with one or more $R_{2-1}$, $C_{3-10}$ cycloalkyl, or "3- to 10-membered heterocyclyl having 1, 2 or 3 heteroatoms selected from 1, 2 or 3 of N, O and S", preferably $C_{1-6}$ alkyl or $C_{1-6}$ alkyl substituted with one or more $R_{2-1}$;
(5) each $R_4$ is independently halogen, -$NH_2$, -CN, $C_{1-6}$ alkyl, or $C_{1-6}$ alkyl substituted with one or more $R_{4-1}$; and
(6) L is -O- or -S-.

**6.** The fused ring compound represented by formula I or the pharmaceutically acceptable salt thereof according to claim 1 or 2, wherein,

$R_1$ is $C_{3-10}$ cycloalkyl substituted with one or more $R_{1-1b}$, "3- to 10-membered heterocyclyl having 1, 2 or 3 heteroatoms selected from 1, 2 or 3 of N, O and S", or "3- to 10-membered heterocyclyl having 1, 2 or 3 heteroatoms selected from 1, 2 or 3 of N, O and S" substituted with one or more $R_{1-1c}$;
each $R_{1-1b}$ is independently $C_{1-6}$ alkyl substituted with one or more $R_{1-3a}$;
each $R_{1-1c}$ is independently $C_{1-6}$ alkyl;
each $R_{1-3a}$ is independently halogen;
L is -O-;
$R_2$ is $C_{1-6}$ alkyl or $C_{1-6}$ alkyl substituted with one or more $R_{2-1}$;
$R_{2-1}$ is deuterium;
$R_3$ is $C_{1-6}$ alkyl;
ring A is $C_{6-10}$ aryl, "5- to 10-membered heterocyclyl having 1, 2 or 3 heteroatoms selected from 1, 2 or 3 of N, O and S", or "5- to 10-membered heteroaryl having 1, 2 or 3 heteroatoms selected from 1, 2 or 3 of N, O and S";
each $R_4$ is independently halogen, -$NH_2$, $C_{1-6}$ alkyl, $C_{1-6}$ alkyl substituted with one or more $R_{4-1}$, or $C_{6-10}$ aryl substituted with one or more -$(CH_2)_m NR_{4-2}R_{4-3}$; and
each $R_{4-1}$ is independently halogen or hydroxy.

**7.** The fused ring compound represented by formula I or the pharmaceutically acceptable salt thereof according to claim 1 or 2, wherein,

$R_1$ is $C_{3-10}$ cycloalkyl substituted with one or more $R_{1-1b}$, "3- to 10-membered heterocyclyl having 1, 2 or 3 heteroatoms selected from 1, 2 or 3 of N, O and S", or "3- to 10-membered heterocyclyl having 1, 2 or 3 heteroatoms selected from 1, 2 or 3 of N, O and S" substituted with one or more $R_{1-1c}$;
each $R_{1-1b}$ is independently $C_{1-6}$ alkyl substituted with one or more $R_{1-3a}$;
each $R_{1-1c}$ is independently $C_{1-6}$ alkyl;
each $R_{1-3a}$ is independently halogen;
L is -O-;
$R_2$ is $C_{1-6}$ alkyl or $C_{1-6}$ alkyl substituted with one or more $R_{2-1}$;

$R_{2-1}$ is deuterium;
$R_3$ is $C_{1-6}$ alkyl;
ring A is $C_{6-10}$ aryl, "5- to 10-membered heterocyclyl having 1, 2 or 3 heteroatoms selected from 1, 2 or 3 of N, O and S", or "5- to 10-membered heteroaryl having 1, 2 or 3 heteroatoms selected from 1, 2 or 3 of N, O and S";
each $R_4$ is independently halogen, $-NH_2$, -CN, $C_{1-6}$ alkyl, $C_{1-6}$ alkyl substituted with one or more $R_{4-1}$, or $C_{6-10}$ aryl substituted with one or more $-(CH_2)_mNR_{4-2}R_{4-3}$; and
each $R_{4-1}$ is independently halogen or hydroxy.

**8.** The fused ring compound represented by formula I or the pharmaceutically acceptable salt thereof according to claim 1 or 2, wherein the fused ring compound represented by formula I or the pharmaceutically acceptable salt thereof satisfies one or more of the following conditions:

(1) in $R_1$, $R_{1-1a}$, $R_{1-1b}$, $R_{1-1c}$, $R_{1-1d}$, $R_{1-1e}$, $R_{1-2a}$, $R_{1-2b}$, $R_{1-2c}$, $R_{1-2d}$, $R_{1-2e}$, $R_{1-2f}$, $R_{1-2g}$, $R_{1-3a}$, $R_{1-3b}$, $R_{1-3c}$, $R_{1-3d}$, $R_{1-3e}$, $R_{1-3f}$, $R_{1-3g}$, $R_{1-4a}$, $R_{1-4b}$, $R_{1-4c}$, $R_{L-1}$, $R_2$, $R_3$, $R_4$, $R_{4-2}$, and $R_{4-3}$, the "$C_{1-6}$ alkyl" in each of the "$C_{1-6}$ alkyl substituted with one or more $R_{1-1a}$", the "$C_{1-6}$ alkyl substituted with one or more $R_{1-3a}$", the "$C_{1-6}$ alkyl substituted with one or more $R_{1-3c}$", the "$C_{1-6}$ alkyl substituted with one or more halogens", the "$C_{1-6}$ alkyl", the "$C_{1-6}$ alkyl substituted with one or more $R_{2-1}$", and the "$C_{1-6}$ alkyl substituted with one or more $R_{4-1}$" is independently methyl, ethyl, n-propyl, isopropyl, *n*-butyl, isobutyl, *sec*-butyl, or *tert*-butyl;
(2) in $R_1$, $R_{1-1a}$, $R_{1-1b}$, $R_{1-1c}$, $R_{1-1d}$, $R_{1-1e}$, $R_{1-2a}$, $R_{1-2b}$, $R_{1-2c}$, $R_{1-2d}$, $R_{1-2e}$, $R_{1-2f}$, $R_{1-2g}$, $R_{1-3a}$, $R_{1-3b}$, $R_{1-3c}$, $R_{1-3d}$, $R_{1-3e}$, $R_{1-3f}$, $R_{1-3g}$, $R_{1-4a}$, $R_{1-4b}$, $R_{1-4c}$, $R_2$, and $R_{2-1}$, the "$C_{3-10}$ cycloalkyl" in each of the "$C_{3-10}$ cycloalkyl", the "$C_{3-10}$ cycloalkyl substituted with one or more $R_{1-1b}$", the "$C_{3-10}$ cycloalkyl substituted with one or more $R_{1-3b}$", and the "$C_{3-10}$ cycloalkyl substituted with one or more $R_{1-3d}$" is independently $C_{3-6}$ cycloalkyl;
(3) in $R_1$, $R_{1-2a}$, $R_{1-2b}$, $R_{1-2c}$, $R_{1-2d}$, $R_{1-2e}$, $R_{1-2f}$, $R_{1-2g}$, $R_{1-3a}$, $R_{1-3b}$, $R_{1-3c}$, $R_{1-3d}$, $R_{1-3e}$, $R_{1-3f}$, $R_{1-3g}$, $R_{1-4a}$, $R_{1-4b}$, $R_{1-4c}$, $R_2$, and $R_4$, the "3- to 10-membered heterocyclyl" in each of the "3- to 10-membered heterocyclyl having 1, 2 or 3 heteroatoms selected from 1, 2 or 3 of N, O and S", the "'3- to 10-membered heterocyclyl having 1, 2 or 3 heteroatoms selected from 1, 2 or 3 of N, O and S' substituted with one or more $R_{1-1c}$", and the "'3- to 10-membered heterocyclyl having 1, 2 or 3 heteroatoms selected from 1, 2 or 3 of N, O and S' substituted with one or more $R_{1-3e}$" is independently 5- to 6-membered heterocyclyl;
(4) in $R_1$, $R_{1-2a}$, $R_{1-2b}$, $R_{1-2c}$, $R_{1-2d}$, $R_{1-2e}$, $R_{1-2f}$, $R_{1-2g}$, $R_{1-3a}$, $R_{1-3b}$, $R_{1-3c}$, $R_{1-3d}$, $R_{1-3e}$, $R_{1-3f}$, $R_{1-3g}$, $R_{1-4a}$, $R_{1-4b}$, $R_{1-4c}$, $R_4$, and ring A, the "$C_{6-10}$ aryl" in each of the "$C_{6-10}$ aryl", the "$C_{6-10}$ aryl substituted with one or more $R_{1-1d}$", the "$C_{6-10}$ aryl substituted with one or more $R_{1-3f}$", and the "$C_{6-10}$ aryl substituted with one or more $-(CH_2)_mNR_{4-2}R_{4-3}$" is independently phenyl;
(5) in $R_1$, $R_{1-2a}$, $R_{1-2b}$, $R_{1-2c}$, $R_{1-2d}$, $R_{1-2e}$, $R_{1-2f}$, $R_{1-2g}$, $R_{1-3a}$, $R_{1-3b}$, $R_{1-3c}$, $R_{1-3d}$, $R_{1-3e}$, $R_{1-3f}$, $R_{1-3g}$, $R_{1-4a}$, $R_{1-4b}$, $R_{1-4c}$, and ring A, the "5- to 10-membered heteroaryl" in each of the "5- to 10-membered heteroaryl having 1, 2 or 3 heteroatoms selected from 1, 2 or 3 of N, O and S", the "'5- to 10-membered heteroaryl having 1, 2 or 3 heteroatoms selected from 1, 2 or 3 of N, O and S' substituted with one or more $R_{1-1e}$", and the "'5- to 10-membered heteroaryl having 1, 2 or 3 heteroatoms selected from 1, 2 or 3 of N, O and S' substituted with one or more $R_{1-3g}$" is independently "9- to 10-membered heteroaryl";
(6) in ring A, the "5- to 10-membered heterocyclyl" in the "5- to 10-membered heterocyclyl having 1, 2 or 3 heteroatoms selected from 1, 2 or 3 of N, O and S" is "9- to 10-membered heterocyclyl"; and
(7) in $R_{1-1a}$, $R_{1-1b}$, $R_{1-1c}$, $R_{1-1d}$, $R_{1-1e}$, $R_{1-3a}$, $R_{1-3b}$, $R_{1-3c}$, $R_{1-3d}$, $R_{1-3e}$, $R_{1-3f}$, $R_{1-3g}$, $R_{2-1}$, $R_3$, $R_4$, and $R_{4-1}$, the "halogen" in each of the "halogen" and the "$C_{1-6}$ alkyl substituted with one or more halogens" is independently fluorine, chlorine, bromine, or iodine.

**9.** The fused ring compound represented by formula I or the pharmaceutically acceptable salt thereof according to claim 8, wherein the fused ring compound represented by formula I or the pharmaceutically acceptable salt thereof satisfies one or more of the following conditions:

(1) in $R_1$, $R_{1-1a}$, $R_{1-1b}$, $R_{1-1c}$, $R_{1-1d}$, $R_{1-1e}$, $R_{1-2a}$, $R_{1-2b}$, $R_{1-2c}$, $R_{1-2d}$, $R_{1-2e}$, $R_{1-2f}$, $R_{1-2g}$, $R_{1-3a}$, $R_{1-3b}$, $R_{1-3c}$, $R_{1-3d}$, $R_{1-3e}$, $R_{1-3f}$, $R_{1-3g}$, $R_{1-4a}$, $R_{1-4b}$, $R_{1-4c}$, $R_{L-1}$, $R_2$, $R_3$, $R_4$, $R_{4-2}$, and $R_{4-3}$, the "$C_{1-6}$ alkyl" in each of the "$C_{1-6}$ alkyl substituted with one or more $R_{1-1a}$", the "$C_{1-6}$ alkyl substituted with one or more $R_{1-3a}$", the "$C_{1-6}$ alkyl substituted with one or more $R_{1-3c}$", the "$C_{1-6}$ alkyl substituted with one or more halogens", the "$C_{1-6}$ alkyl", the "$C_{1-6}$ alkyl substituted with one or more $R_{2-1}$", and the "$C_{1-6}$ alkyl substituted with one or more $R_{4-1}$" is independently methyl, ethyl, n-propyl, or isopropyl;
(2) in $R_1$, $R_{1-1a}$, $R_{1-1b}$, $R_{1-1c}$, $R_{1-1d}$, $R_{1-1e}$, $R_{1-2a}$, $R_{1-2b}$, $R_{1-2c}$, $R_{1-2d}$, $R_{1-2e}$, $R_{1-2f}$, $R_{1-2g}$, $R_{1-3a}$, $R_{1-3b}$, $R_{1-3c}$, $R_{1-3d}$, $R_{1-3e}$, $R_{1-3f}$, $R_{1-3g}$, $R_{1-4a}$, $R_{1-4b}$, $R_{1-4c}$, $R_2$, and $R_{2-1}$, the "$C_{3-10}$ cycloalkyl" in each of the "$C_{3-10}$ cycloalkyl", the "$C_{3-10}$ cycloalkyl substituted with one or more $R_{1-1b}$", the "$C_{3-10}$ cycloalkyl substituted with one or more $R_{1-3b}$", and the "$C_{3-10}$ cycloalkyl substituted with one or more $R_{1-3d}$" is cyclopropyl, cyclobutyl, or cyclopentyl (e.g.,

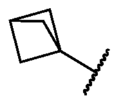

), preferably

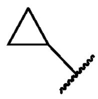

;

(3) in $R_1$, $R_{1-2a}$, $R_{1-2b}$, $R_{1-2c}$, $R_{1-2d}$, $R_{1-2e}$, $R_{1-2f}$, $R_{1-2g}$, $R_{1-3a}$, $R_{1-3b}$, $R_{1-3c}$, $R_{1-3d}$, $R_{1-3e}$, $R_{1-3f}$, $R_{1-3g}$, $R_{1-4a}$, $R_{1-4b}$, $R_{1-4c}$, $R_2$, and $R_4$, the "3- to 10-membered heterocyclyl" in each of the "3- to 10-membered heterocyclyl having 1, 2 or 3 heteroatoms selected from 1, 2 or 3 of N, O and S", the "'3- to 10-membered heterocyclyl having 1, 2 or 3 heteroatoms selected from 1, 2 or 3 of N, O and S' substituted with one or more $R_{1-1c}$", and the "'3- to 10-membered heterocyclyl having 1, 2 or 3 heteroatoms selected from 1, 2 or 3 of N, O and S' substituted with one or more $R_{1-3e}$" is

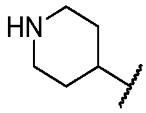

,

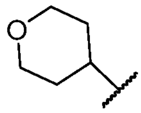

, or

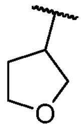

;

(4) in ring A, the "5- to 10-membered heterocyclyl" in the "5- to 10-membered heterocyclyl having 1, 2 or 3 heteroatoms selected from 1, 2 or 3 of N, O and S" is 2,3-dihydrobenzofuranyl, preferably

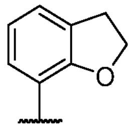

;

(5) in $R_{1-1a}$, $R_{1-1b}$, $R_{1-1c}$, $R_{1-1d}$, $R_{1-1e}$, $R_{1-3a}$, $R_{1-3b}$, $R_{1-3c}$, $R_{1-3d}$, $R_{1-3e}$, $R_{1-3f}$, $R_{1-3g}$, $R_{2-1}$, $R_3$, $R_4$, and $R_{4-1}$, the "halogen" in each of the "halogen" and the "$C_{1-6}$ alkyl substituted with one or more halogens" is fluorine; and

(6) in $R_1$, $R_{1-2a}$, $R_{1-2b}$, $R_{1-2c}$, $R_{1-2d}$, $R_{1-2e}$, $R_{1-2f}$, $R_{1-2g}$, $R_{1-3a}$, $R_{1-3b}$, $R_{1-3c}$, $R_{1-3d}$, $R_{1-3e}$, $R_{1-3f}$, $R_{1-3g}$, $R_{1-4a}$, $R_{1-4b}$, $R_{1-4c}$, and ring A, the "5- to 10-membered heteroaryl" in each of the "5- to 10-membered heteroaryl having 1, 2 or 3 heteroatoms selected from 1, 2 or 3 of N, O and S", the "'5- to 10-membered heteroaryl having 1, 2 or 3 heteroatoms selected from 1, 2 or 3 of N, O and S' substituted with one or more $R_{1-1e}$", and the "'5- to 10-membered heteroaryl having 1, 2 or 3 heteroatoms selected from 1, 2 or 3 of N, O and S' substituted with one or more $R_{1-3g}$" is independently benzofuranyl, preferably

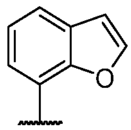

.

**10.** The fused ring compound represented by formula I or the pharmaceutically acceptable salt thereof according to claim 1 or 2, wherein the fused ring compound represented by formula I or the pharmaceutically acceptable salt thereof satisfies one or more of the following conditions:

(1) $R_1$ is

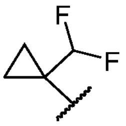, 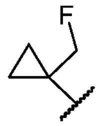, 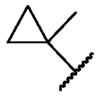, 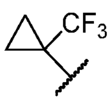, 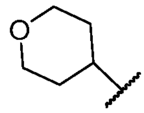, 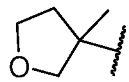,

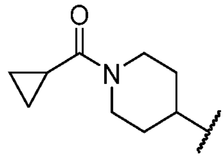, 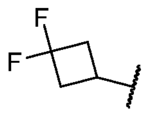, 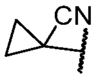, 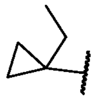, 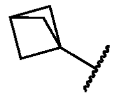, 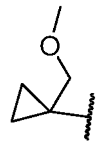, or 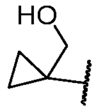;

(2) $R_2$ is hydrogen, methyl, ethyl, $-CD_3$, isopropyl, $-CH_2CF_3$,

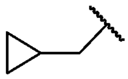,

cyclopropyl,

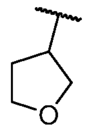, 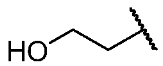, or 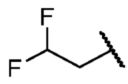;

(3) $R_3$ is methyl;

(4) $R_4$ is

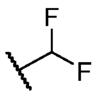, 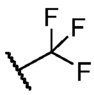, 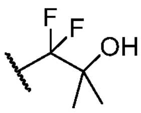, 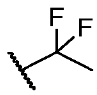, 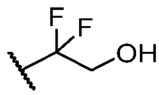

, -F, $-CH_3$, $-NH_2$,

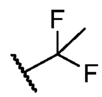,

or

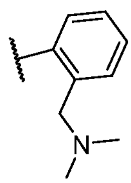;

and

(5) ring A is phenyl, benzofuranyl, 2,3-dihydrobenzofuranyl, or thienyl.

**11.** The fused ring compound represented by formula I or the pharmaceutically acceptable salt thereof according to claim 10, wherein the fused ring compound represented by formula I or the pharmaceutically acceptable salt thereof satisfies one or more of the following conditions:

(1) $R_1$ is

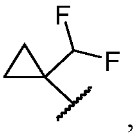, 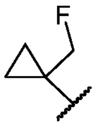, 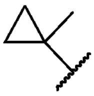, 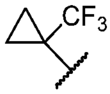, 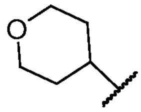, or 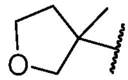;

(2) $R_2$ is methyl, ethyl, or $-CD_3$;

(3) $R_4$ is

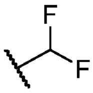, -F, $-CH_3$, 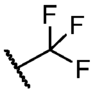, 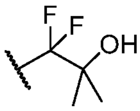, 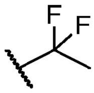,

$-NH_2$,

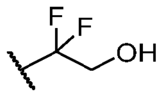,

or

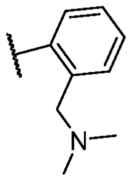;

and

(4) ring A is phenyl,

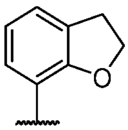, 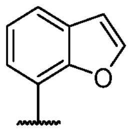, or 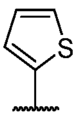.

**12.** The fused ring compound represented by formula I or the pharmaceutically acceptable salt thereof according to claim 1 or 2, wherein the fused ring compound represented by formula I or the pharmaceutically acceptable salt thereof satisfies one or more of the following conditions:

(1) $-L-R_2$ is $-OCH_3$, $-OCH_2CH_3$, $-OCD_3$, $-SO_2CH_3$, $-OCH_2CF_3$,

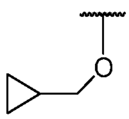,

$-SCH_3$, $-N(CH_3)(CH_3)$, $-NH_2$,

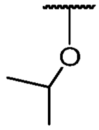,

-OH,

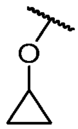, 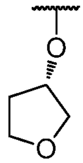, 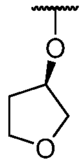, 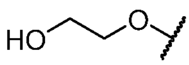, 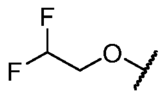,

or - $SO_2CH_2CH_3$; and

(2)

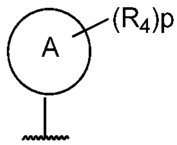

is

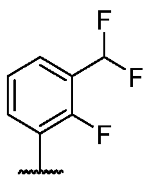, 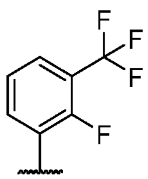, 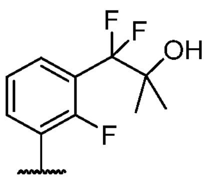, 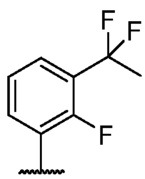,

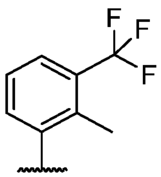, 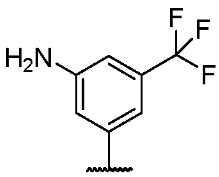, 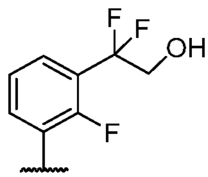, 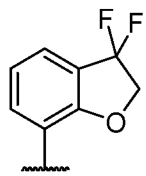, 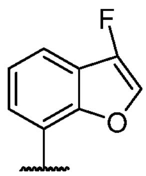, 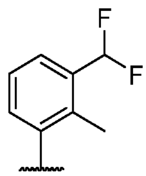,

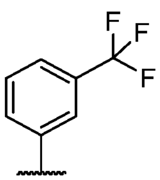, 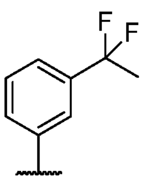, 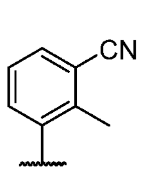, or 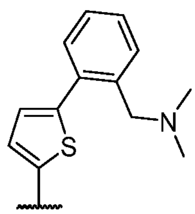.

**13.** The fused ring compound represented by formula I or the pharmaceutically acceptable salt thereof according to claim 12, wherein the fused ring compound represented by formula I or the pharmaceutically acceptable salt thereof satisfies one or more of the following conditions:

(1) $-L-R_2$ is $-OCH_3$, $-OCH_2CH_3$, $-OCD_3$, or $-SCH_3$; and

(2)

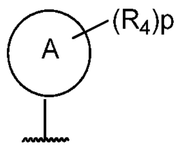

is

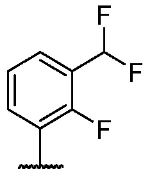 , 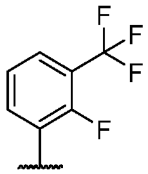 , 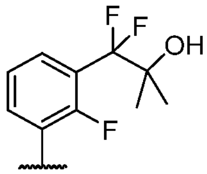 , 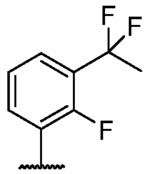 ,

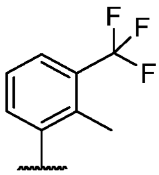 , 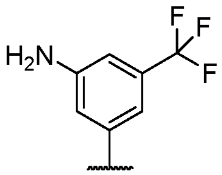 , 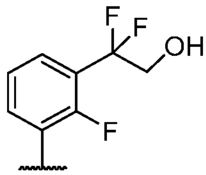 , 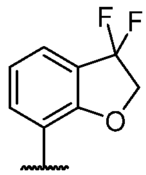 , 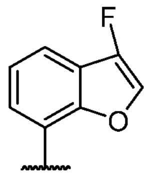 , 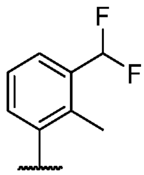 ,

or

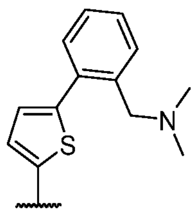 .

**14.** The fused ring compound represented by formula I or the pharmaceutically acceptable salt thereof according to claim 1, wherein the fused ring compound represented by formula I is any one of the following compounds:

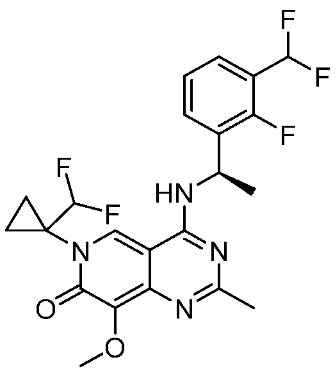

**SZ-022244** ,

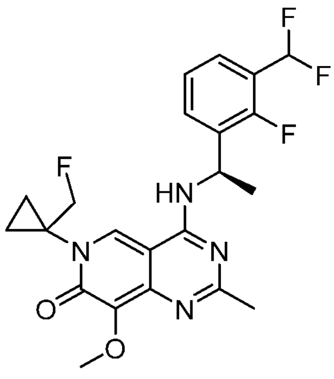

**SZ-022301** ,

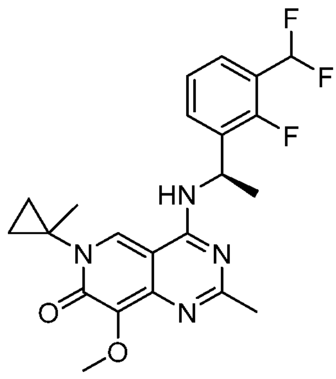

**SZ-022300** ,

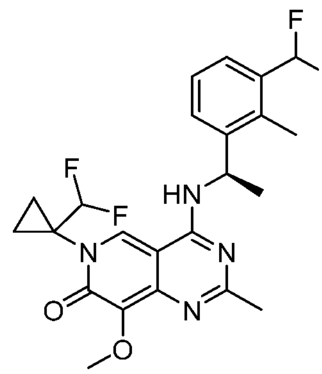

**SZ-022302** ,

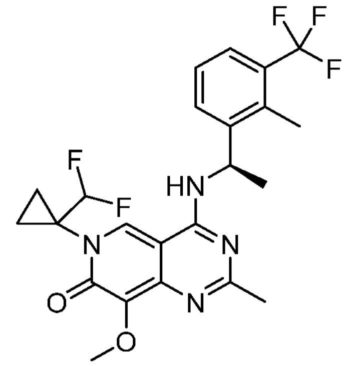

**SZ-022304** ,

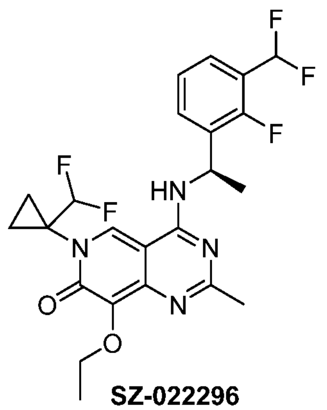

**SZ-022296** ,

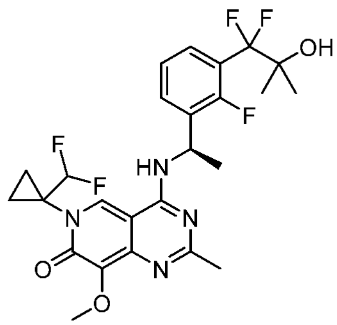

**SZ-022317** ,

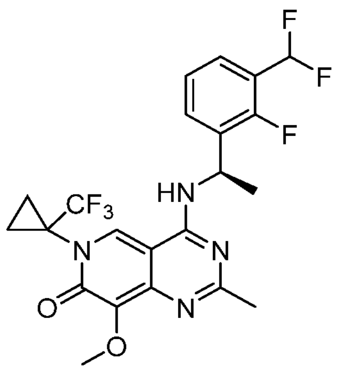

**SZ-022325** ,

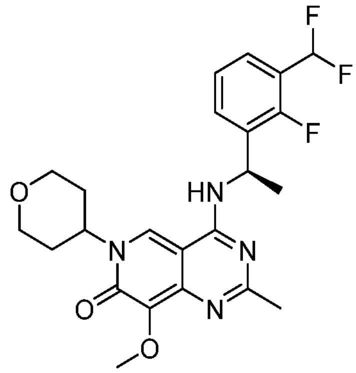

**SZ-022326** ,

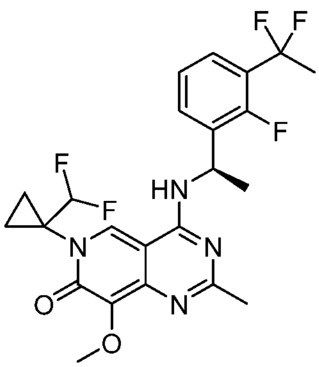

**SZ-022303** ,

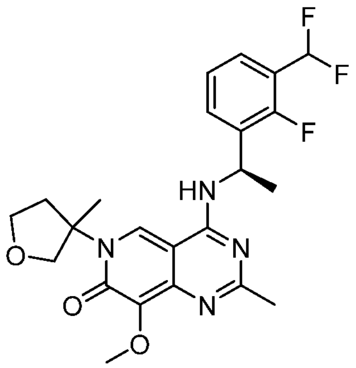

**SZ-022328** ,

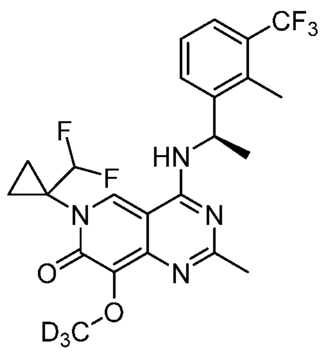

**SZ-022334** ,

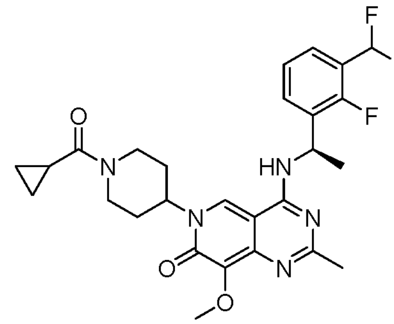

SZ-022335,

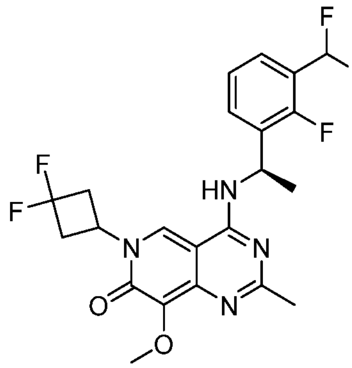

SZ-022336,

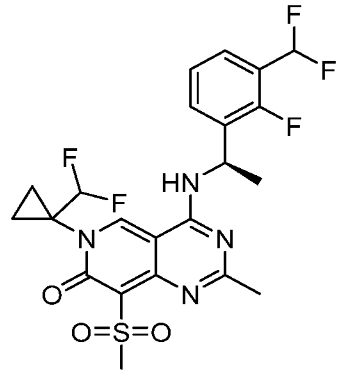

SZ-022307,

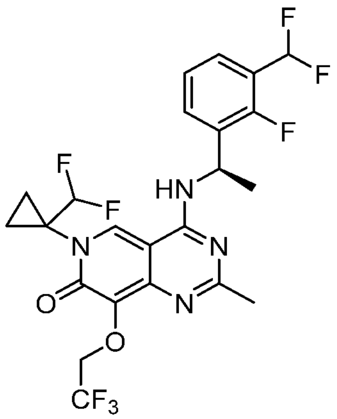

SZ-022312,

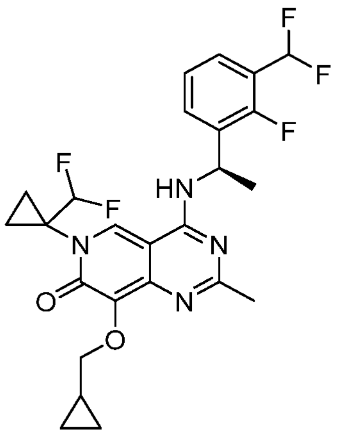

SZ-022314,

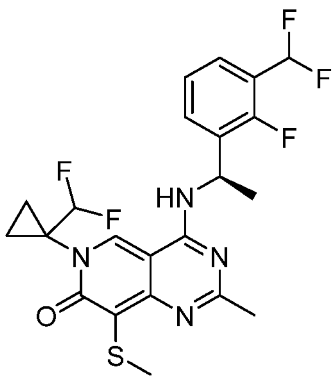

SZ-022306,

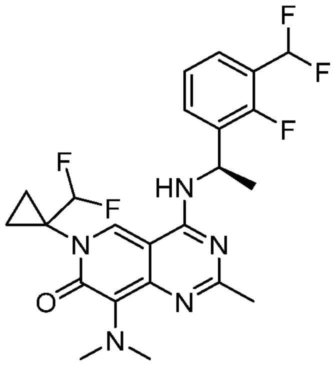

SZ-022251,

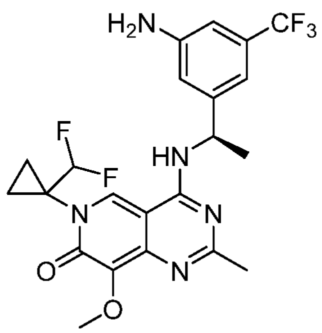

SZ-022327,

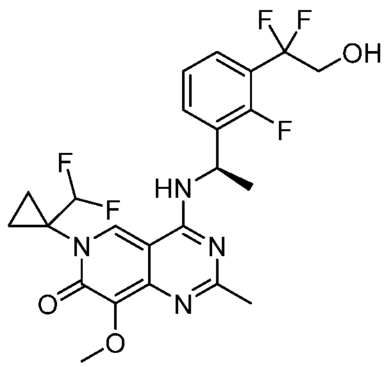

SZ-022329,

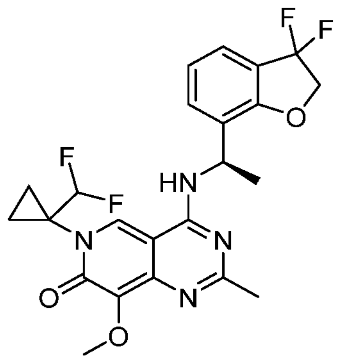

**SZ-022330** ,

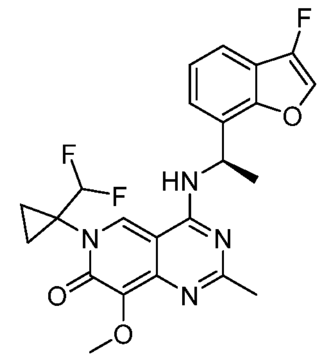

**SZ-022330B** ,

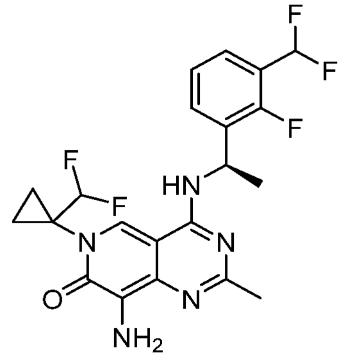

**SZ-022224** ,

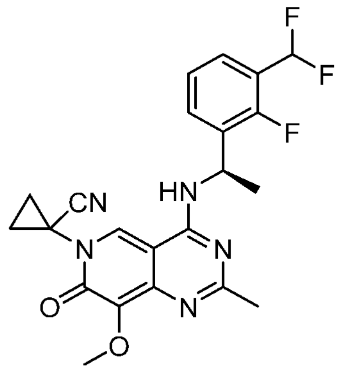

**SZ-022344** ,

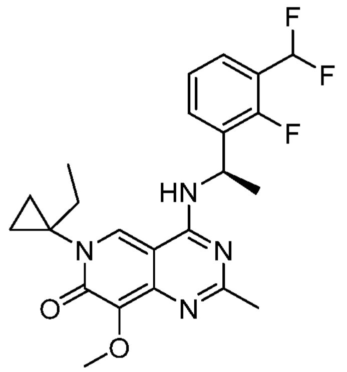

**SZ-022343** ,

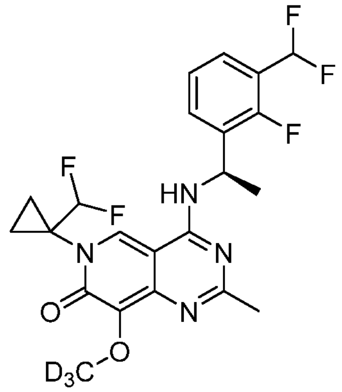

**SZ-022295** ,

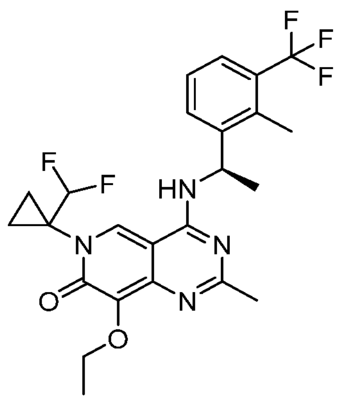

**SZ-022339** ,

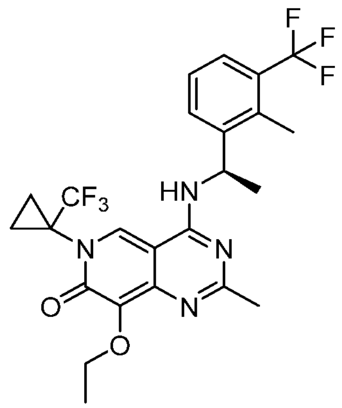

**SZ-022340** ,

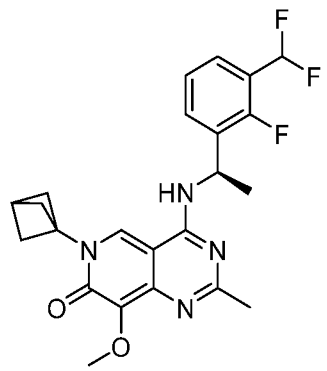

**SZ-022345** ,

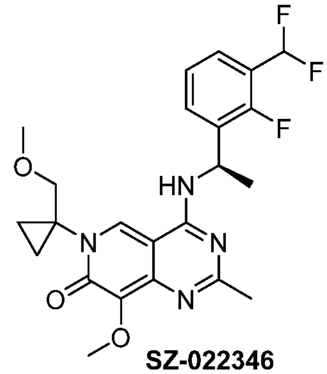
SZ-022346 ,

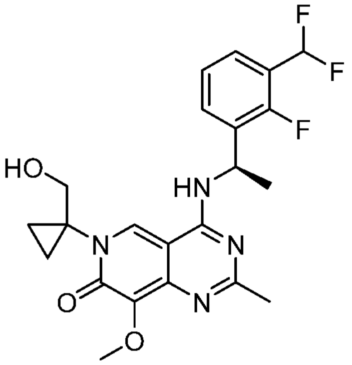
SZ-022347 ,

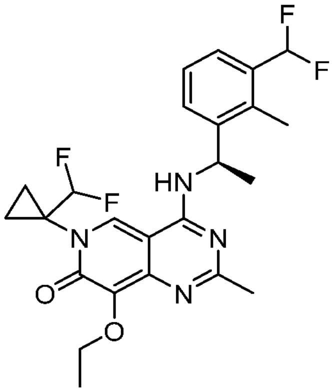
SZ-022337 ,

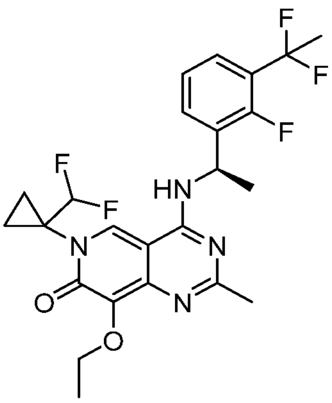
SZ-022338 ,

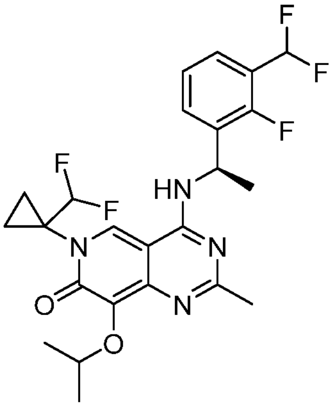
SZ-022313 ,

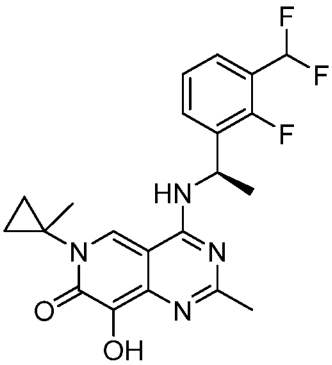
SZ-022323 ,

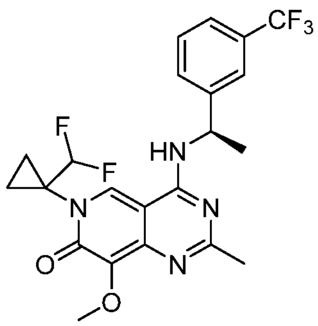
SZ-022348 ,

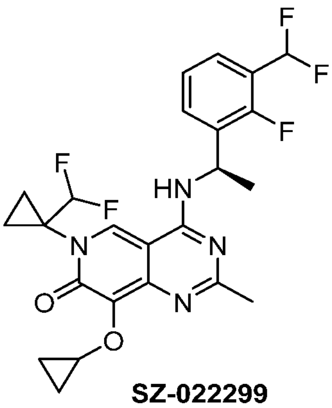
SZ-022299 ,

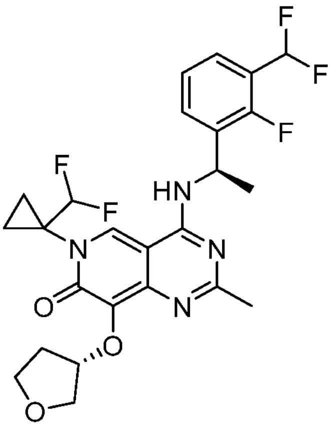
SZ-022351

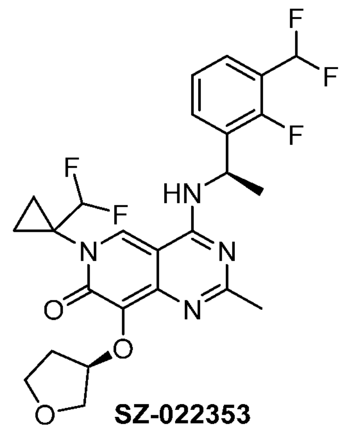

**SZ-022353** ,

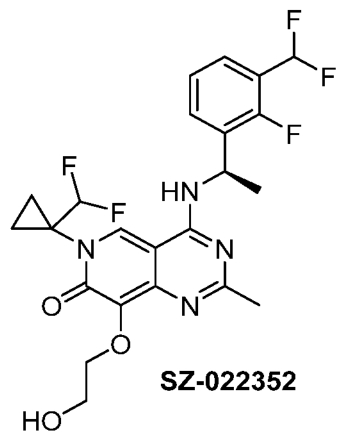

**SZ-022352** ,

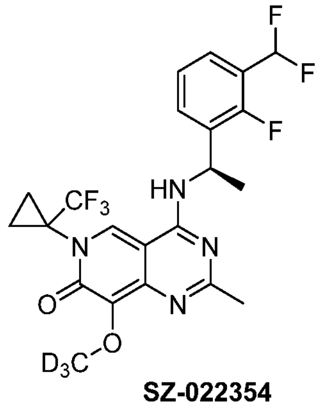

**SZ-022354** ,

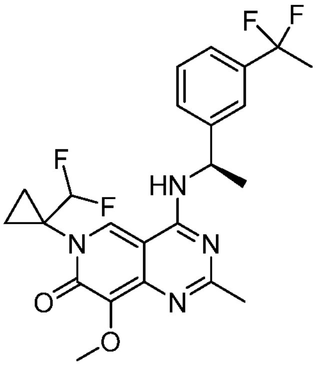

**SZ-022350** ,

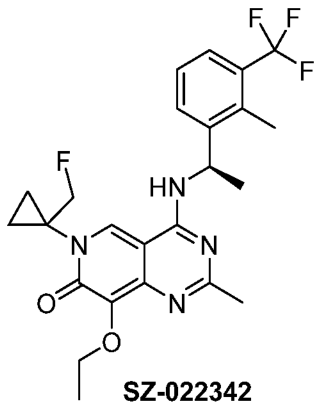

**SZ-022342** ,

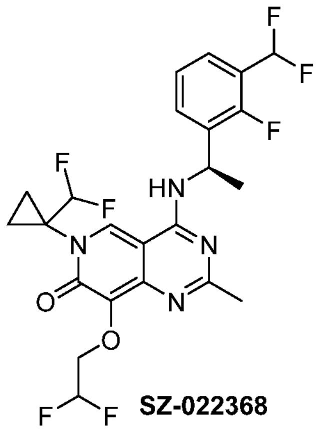

**SZ-022368** ,

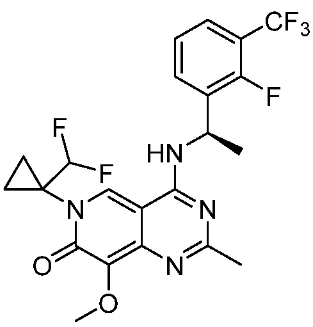

**SZ-022349** ,

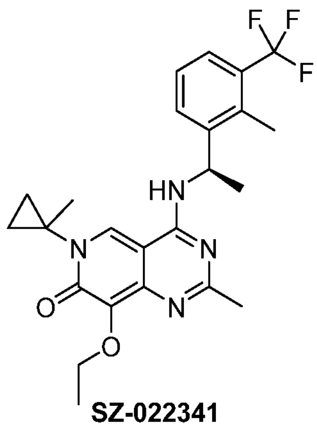

**SZ-022341** ,

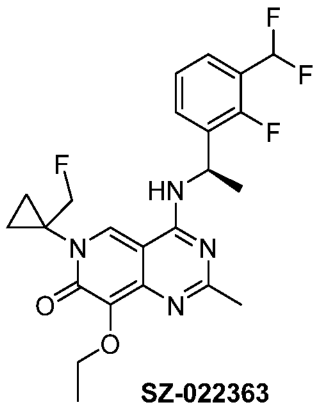

**SZ-022363** ,

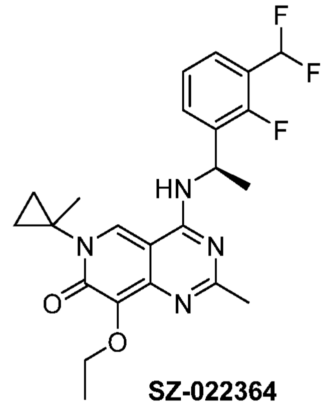

SZ-022364 ,

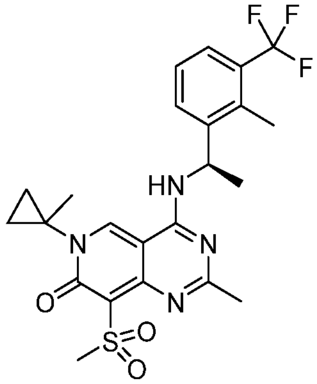

SZ-022371 ,

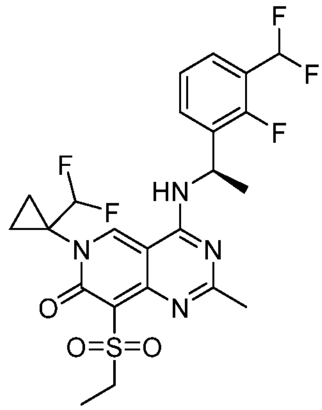

SZ-022372 ,

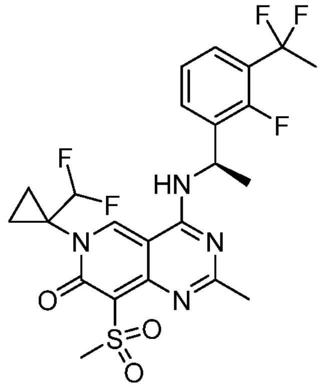

SZ-022370 ,

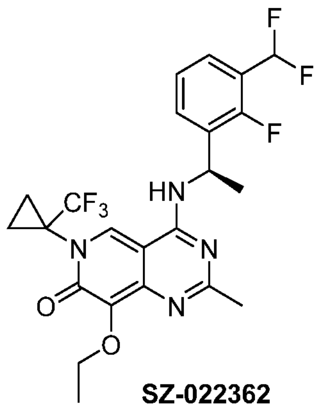

SZ-022362 ,

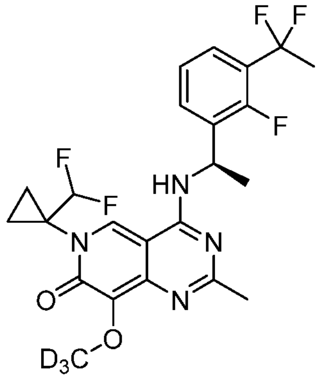

SZ-022355 ,

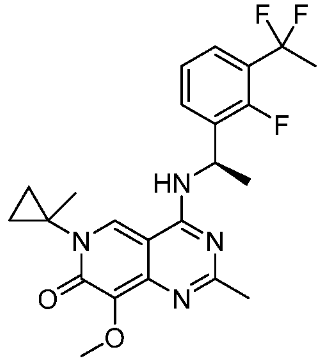

SZ-022356 ,

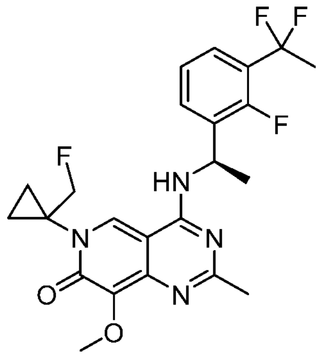

SZ-022357 ,

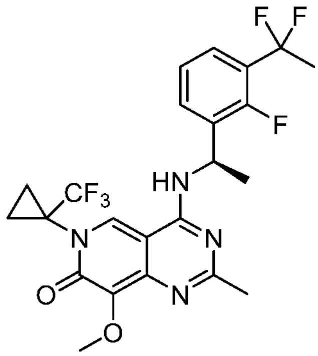

SZ-022358 ,

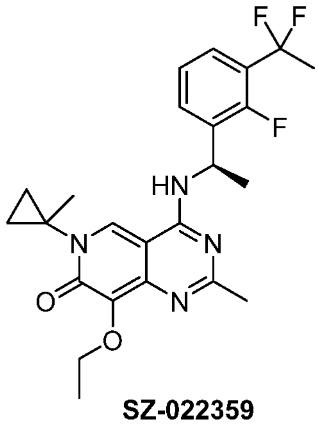

**SZ-022359**

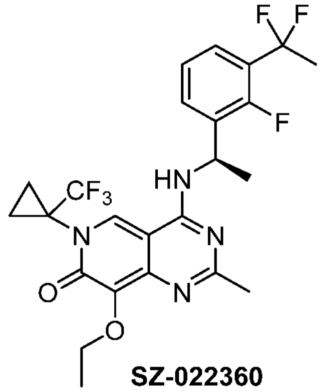

**SZ-022360**

,

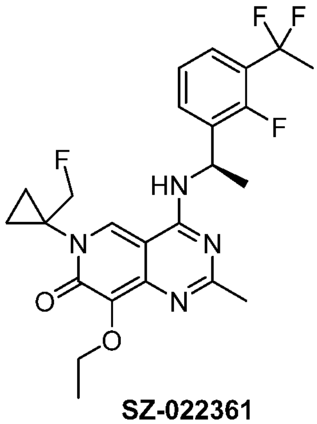

**SZ-022361**

,

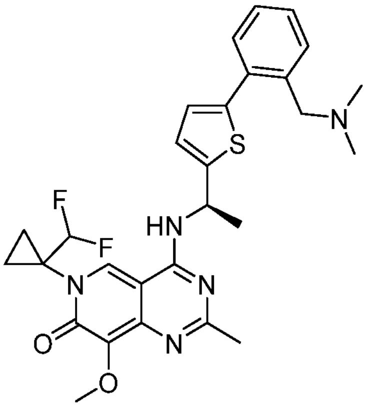

**SZ-022373**

,

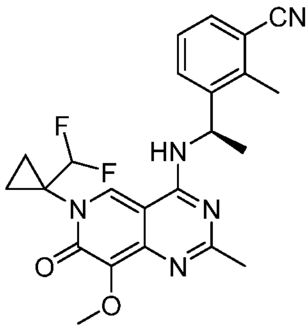

**SZ-022381**

,

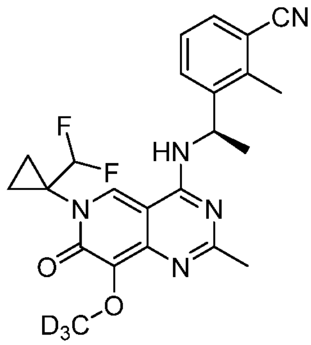

**SZ-022382**

,

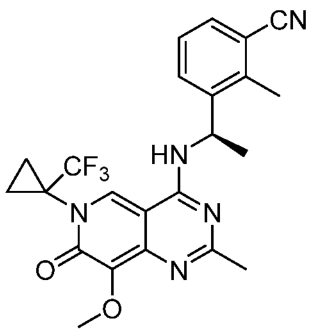

**SZ-022383**

, and

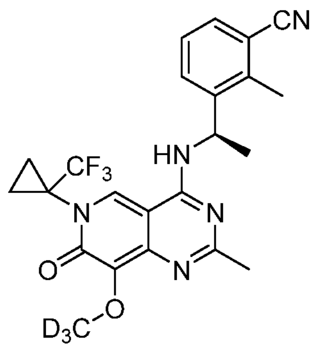

**SZ-022384**

.

**15.** A pharmaceutical composition, comprising:

(1) the fused ring compound represented by formula I or the pharmaceutically acceptable salt thereof according to any one of claims 1 to 14, and
(2) a pharmaceutically acceptable auxiliary material.

**16.** Use of the fused ring compound represented by formula I or the pharmaceutically acceptable salt thereof according to any one of claims 1 to 14 or the pharmaceutical composition according to claim 15 in the preparation of an SOS1 inhibitor.

**17.** The use according to claim 16, wherein the use satisfies one or more of the following conditions:

(1) a disease mediated by the SOS1 is lung cancer, pancreatic cancer, pancreatic ductal carcinoma, colon cancer, rectal cancer, appendiceal cancer, esophageal squamous carcinoma, head and neck squamous carcinoma, breast cancer, or other solid tumors; and
(2) the SOS 1 inhibitor is used in a mammalian organism; and/or the SOS 1 inhibitor is used *in vitro,* mainly for experimental purposes.

**18.** Use of the fused ring compound represented by formula I or the pharmaceutically acceptable salt thereof according to any one of claims 1 to 14 or the pharmaceutical composition according to claim 15 in the preparation of a medicament, wherein the medicament is used for preventing and/or treating one or more of the following diseases: lung cancer, pancreatic cancer, pancreatic ductal carcinoma, colon cancer, rectal cancer, appendiceal cancer, esophageal squamous carcinoma, head and neck squamous carcinoma, breast cancer, and other solid tumors.

**INTERNATIONAL SEARCH REPORT**

International application No.
**PCT/CN2022/110019**

**A. CLASSIFICATION OF SUBJECT MATTER**

C07D 471/04(2006.01)i; C07D 453/02(2006.01)i; C07D 519/00(2006.01)i; A61P 35/00(2006.01)i; A61K 31/519(2006.01)i

According to International Patent Classification (IPC) or to both national classification and IPC

**B. FIELDS SEARCHED**

Minimum documentation searched (classification system followed by classification symbols)

C07D; A61P; A61K

Documentation searched other than minimum documentation to the extent that such documents are included in the fields searched

Electronic data base consulted during the international search (name of data base and, where practicable, search terms used)

DWPI, SIPOABS, WOTXT, EPTXT, USTXT, CNTXT, CATXT, GBTXT, JPTXT, KRABS, CNABS, CNKI, STNext, ISI Web of Science: SOS蛋白, 吡啶并嘧啶酮, 肿瘤, 抑制剂, SOS protein, pyrimidinone, tumor, inhibitor, 信诺维医药, EVOPOINT BIOSCIENCES

**C. DOCUMENTS CONSIDERED TO BE RELEVANT**

| Category* | Citation of document, with indication, where appropriate, of the relevant passages | Relevant to claim No. |
|---|---|---|
| E | CN 114907341 A (WUHAN RENFU INNOVATIVE DRUG R&D CENTER CO., LTD. et al.) 16 August 2022 (2022-08-16)<br>abstract, and claims 1-33 | 1-18 |
| PX | CN 113200981 A (HANGZHOU INNOGATE PHARMA CO., LTD.) 03 August 2021 (2021-08-03)<br>abstract, and claims 1-17 | 1-18 |
| PX | CN 114456165 A (SHANGHAI HANSOH BIOMEDICAL CO., LTD. et al.) 10 May 2022 (2022-05-10)<br>abstract, and claims 1-10 | 1-18 |
| PX | CN 114524810 A (SHANGHAI RINGENE BIOPHARMA CO., LTD.) 24 May 2022 (2022-05-24)<br>abstract, and claims 1-10 | 1-18 |
| PX | CN 114539245 A (SHANGHAI HANSOH BIOMEDICAL CO., LTD. et al.) 27 May 2022 (2022-05-27)<br>abstract, and claims 1-13 | 1-18 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 October 2022** | **01 November 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/110019**

**C. DOCUMENTS CONSIDERED TO BE RELEVANT**

| Category* | Citation of document, with indication, where appropriate, of the relevant passages | Relevant to claim No. |
|---|---|---|
| PX | CN 114835703 A (SUZHOU ZELGEN BIOPHARMACEUTICALS CO., LTD. et al.) 02 August 2022 (2022-08-02)<br>abstract, and claims 1-10 | 1-18 |
| PX | WO 2021259972 A1 (BOEHRINGER INGELHEIM INTERNATIONAL GMBH) 30 December 2021 (2021-12-30)<br>abstract, and table A | 1-18 |
| PX | WO 2022017519 A1 (MEDSHINE DISCOVERY INC.) 27 January 2022 (2022-01-27)<br>abstract, and claims 1-13 | 1-18 |
| PX | WO 2022140427 A1 (QILU REGOR THERAPEUTICS INC. et al.) 30 June 2022 (2022-06-30)<br>abstract, and claims 1-25 | 1-18 |
| X | CN 111372932 A (BOEHRINGER INGELHEIM INTERNATIONAL GMBH) 03 July 2020 (2020-07-03)<br>abstract, and table 20, and claims 1 and 20-27 | 1-18 |
| X | WO 2020254451 A1 (BOEHRINGER INGELHEIM INTERNATIONAL GMBH) 24 December 2020 (2020-12-24)<br>abstract, and table 20 | 1-18 |

Form PCT/ISA/210 (second sheet) (January 2015)

**INTERNATIONAL SEARCH REPORT**
**Information on patent family members**

International application No.
**PCT/CN2022/110019**

| Patent document cited in search report | Publication date (day/month/year) | Patent family member(s) | Publication date (day/month/year) |
|---|---|---|---|
| CN 114907341 A | 16 August 2022 | None | |
| CN 113200981 A | 03 August 2021 | None | |
| CN 114456165 A | 10 May 2022 | None | |
| CN 114524810 A | 24 May 2022 | WO 2022105921 A1 | 27 May 2022 |
| CN 114539245 A | 27 May 2022 | None | |
| CN 114835703 A | 02 August 2022 | None | |
| WO 2021259972 A1 | 30 December 2021 | None | |
| WO 2022017519 A1 | 27 January 2022 | None | |
| WO 2022140427 A1 | 30 June 2022 | None | |
| CN 111372932 A | 03 July 2020 | AU 2018390927 A1 | 28 May 2020 |
| | | IL 275379 A | 30 July 2020 |
| | | US 2021009588 A1 | 14 January 2021 |
| | | WO 2019122129 A1 | 27 June 2019 |
| | | US 2019194192 A1 | 27 June 2019 |
| | | CR 20210307 A | 27 July 2021 |
| | | PE 20210163 A1 | 26 January 2021 |
| | | EA 202091491 A1 | 13 November 2020 |
| | | CO 2020007218 A2 | 19 June 2020 |
| | | KR 20200111163 A | 28 September 2020 |
| | | TW 201938557 A | 01 October 2019 |
| | | CL 2021000907 A1 | 29 October 2021 |
| | | CA 3085835 A1 | 27 June 2019 |
| | | CL 2020001501 A1 | 13 November 2020 |
| | | JP 2021506864 A | 22 February 2021 |
| | | EC SP20040257 A | 31 August 2020 |
| | | CR 20200312 A | 11 September 2020 |
| | | SG 11202005881 Y A | 29 July 2020 |
| | | MA 51290 A | 31 March 2021 |
| | | JO P20200154 A1 | 21 June 2019 |
| | | EP 3728254 A1 | 28 October 2020 |
| | | AR 114164 A1 | 29 July 2020 |
| | | UA 126173 C2 | 25 August 2022 |
| | | BR 112020010123 A2 | 10 November 2020 |
| WO 2020254451 A1 | 24 December 2020 | CN 114375202 A | 19 April 2022 |
| | | JP 2022537044 A | 23 August 2022 |
| | | CA 3142239 A1 | 24 December 2020 |
| | | US 2022249492 A1 | 11 August 2022 |
| | | BR 112021024532 A2 | 24 May 2022 |
| | | TW 202114683 A | 16 April 2021 |
| | | KR 20220024191 A | 03 March 2022 |
| | | AU 2020296914 A1 | 23 December 2021 |
| | | EP 3986408 A1 | 27 April 2022 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202110884595
- CN 202111063419
- CN 202111300099
- CN 202111415010
- CN 2022100684941
- WO 2019122129 A

### Non-patent literature cited in the description

- Pharmacopoeia of The People's Republic of China. 2015, vol. 4
- **RAYMOND C ROWE.** Handbook of Pharmaceutical Excipients. 2009